# EUROPÄISCHE PATENTSCHRIFT

(11) **EP 2 310 013 B1**
(45) Veröffentlichungstag und Bekanntmachung des Hinweises auf die Patenterteilung: **23.08.2017**
(21) Anmeldenummer: 09777234.7
(22) Anmeldetag: 16.07.2009
(51) Int. Cl.: A61K 31/4985, A61K 31/506, C07D 403/10, C07D 487/04, C07D 403/14, A61P 35/00

(54) **BICYCLISCHE TRIAZOLDERIVATE ZUR BEHANDLUNG VON TUMOREN**
BICYCLIC TRIAZOLE DERIVATIVES FOR TREATING TUMORS
DÉRIVÉS BICYCLIQUES DE TRIAZOLE POUR LE TRAITEMENT DE TUMEURS

(30) Priorität: 14.08.2008 DE 102008037790
(43) Veröffentlichungstag der Anmeldung: 20.04.2011
(73) Patentinhaber: Merck Patent GmbH, 64293 Darmstadt (DE)
(72) Erfinder: STIEBER, Frank, 69121 Heidelberg (DE); SCHADT, Oliver, 63517 Rodenbach (DE); DORSCH, Dieter, 64372 Ober-Ramstadt (DE); BLAUKAT, Andree, 69198 Schriesheim (DE)
(86) Internationale Anmeldenummer: PCT/EP2009/005172
(87) Internationale Veröffentlichungsnummer: WO 2010/017870

(56) Entgegenhaltungen:
- WO-A1-2005/004607
- WO-A1-2007/075567
- WO-A1-2007/132308
- WO-A2-2006/015263
- US-A1- 2007 015 771
- US-A1- 2007 043 057

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten. Die vorliegende Erfindung betrifft insbesondere Verbindungen und die Verwendung von Verbindungen, bei denen die Hemmung, Regulierung und/oder Modulation der Signaltransduktion von Met-Kinase eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet.
Ein anderer Met-Kinase-Inhibitor zur Krebstherapie ist von J.G. Christensen et al. in Cancer Res. 2003, 63(21), 7345-55 beschrieben.
Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Die Synthese von kleinen Verbindungen, die die Signaltransduktion der Tyrosinkinasen und/oder Serin/Threonin-Kinasen, insbesondere der Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung Verbindungen der Formel I, die die Signaltransduktion der Met-Kinase hemmen, regulieren und/oder modulieren, Zusammensetzungen, die diese Verbindungen enthalten, sowie Verfahren zu ihrer Verwendung zur Behandlung von Met-Kinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Die vorliegende Erfindung richtet sich auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Andere Triazolo-pyrazine sind als cMet-Kinase- Inhibitoren in WO 2005/004607, WO 2007/132308 und US 2007/0265272 beschrieben. Triazolo-pyridazinderivate sind als Met-Kinase-Inhibitoren beschrieben in WO 2007/064797, WO 2007/075567, WO 2007/138472, WO 2008/008539, WO 2008/051805. Andere Triazolderivate sind in US 2007/015771 A1, US 2007/043057 A1 und WO 2006/015263 beschrieben.

### ZUSAMMENFASSUNG DER ERFINDUNG

Die Erfindung betrifft Verbindungen der Formel I 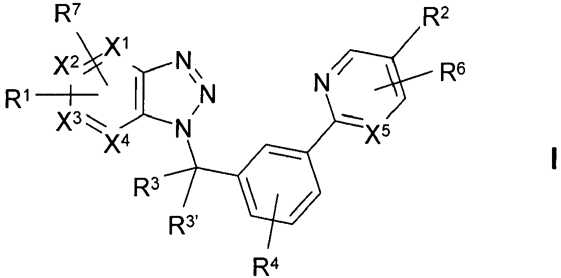 worin
- X¹, X², X³, X⁴, X⁵: jeweils unabhängig voneinander CH oder N,
- R¹: H, Hal, A, S(O)ₘA, Ar, Het, O[C(R⁵)₂]ₙAr, O[C(R⁵)₂]ₙHet oder OR⁵,
- R⁷: H oder Hal,
- R²: A, Hal, [C(R⁵)₂]ₙN(R⁵)₂, [C(R⁵)₂]ₙHet, O[C(R⁵)₂]ₚN(R⁵)₂, O[C(R⁵)₂]ₙHet, [C(R⁵)₂]ₙOR⁵, O[C(R⁵)₂]ₚOR⁵, O-[C(R⁵)₂]ₙ-cycloalkylen-[C(R⁵)₂]ₙ-N(R⁵)₂, [C(R⁵)₂]ₙNR⁵COOA oder CH=CH-COOR⁵,
- R³, R^{3'}: jeweils unabhängig voneinander H oder R⁸,
- R⁴, R⁶: H,
- R⁵: H oder R⁸,
- R⁸: unverzweigtes oder verzweigtes Alkyl mit 1-6 C-Atomen,
- A: unverzweigtes oder verzweigtes Alkyl mit 1-10 C-Atomen, worin 1-7 H-Atome durch OH, F, Cl und/oder Br ersetzt sein können,
oder cyclisches Alkyl mit 3-7 C-Atomen, das einfach durch OH substituiert sein kann,
- Ar: unsubstituiertes oder ein-, zwei- oder dreifach durch Hal, A und/oder CN substituiertes Phenyl,
- Het: Piperidinyl, Pyrrolidinyl, Morpholinyl, Piperazinyl, Oxazolidinyl, Pyrazolyl, Pyridinyl, Pyrimidinyl, Furyl, Thienyl, Oxazolyl, Oxadiazolyl, Imidazolyl, Pyrrolyl, Isoxazolyl oder
Imidazolidinyl, wobei die Reste auch ein- oder zweifach durch Hal, A, COOR⁵, O[C(R⁵)₂]ₚOR⁵, [C(R⁵)₂]ₙHet¹, O[C(R)₂]ₙHet¹ und/oder =O substituiert sein können,
- Het¹: Piperidinyl, Pyrrolidinyl, Morpholinyl, Piperazinyl, Oxazolidinyl oder Imidazolidinyl, wobei die Reste auch ein- oder zweifach durch COOA, =O und/oder A substituiert sein können,
- Hal: F, Cl, Br oder I,
- m: 0, 1 oder 2,
- n: 0, 1, 2, 3 oder 4,
- p: 1, 2, 3 oder 4
bedeuten,
sowie ihre pharmazeutisch verwendbaren Salze, Tautomere und Stereoisomere, einschließlich deren Mischungen in allen Verhältnissen,
Unter Verbindungen der Formel I versteht man auch die Hydrate und Solvate dieser Verbindungen, ferner pharmazeutisch verwendbare Derivate. Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter pharmazeutisch verwendbaren Derivaten versteht man z.B. die Salze der erfindungsgemäßen Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1-2 sowie ihrer pharmazeutisch verwendbaren Salze, Tautomeren und Stereoisomeren, dadurch gekennzeichnet, daß man
a) eine Verbindung der Formel II 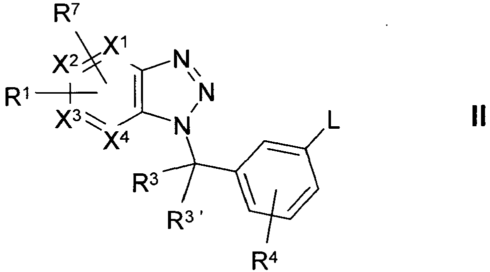
   worin X¹, X², X³, X⁴, R¹, R³, R^{3'}, R⁴ und R⁷ die in Anspruch 1 angegebenen Bedeutungen haben und
   L einen Boronsäure- oder Boronsäureesterrest bedeutet,
   mit einer Verbindung der Formel III 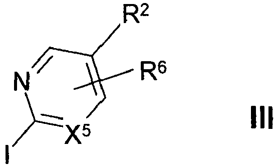 worin X⁵, R² und R⁶ die in Anspruch 1 angegebenen Bedeutungen haben,
   umsetzt,
   oder
b) einen Rest R¹, R² und/oder R⁷ durch einen anderen Rest R¹, R² und/oder R⁷ austauscht, indem ein Halogenatom durch einen Rest Het und/oder Ar, die die in Anspruch 1 angegebenen Bedeutungen haben, ersetzt,
   oder
c) eine Verbindung der Formel IV 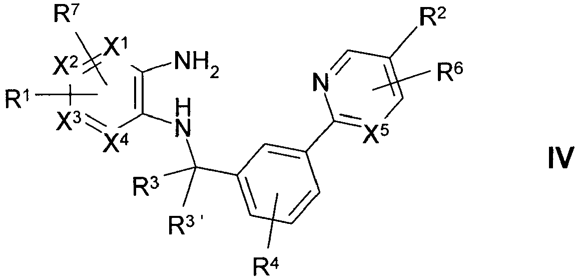 worin X¹, X², X³, X⁴, X⁵, R¹, R², R³, R^{3'}, R⁴, R⁶ und R⁷ die in Anspruch 1 angegebenen Bedeutungen haben, mit NaNO₂ umsetzt,
   und/oder
eine Base oder Säure der Formel I in eines ihrer Salze umwandelt.

Vor- und nachstehend haben die Reste X¹, X², X³, X⁴, X⁵, R¹, R², R³, R^{3'}, R⁴, R⁶ und R⁷ die bei der Formel I angegebenen Bedeutungen, sofern nicht ausdrücklich etwas anderes angegeben ist.
Für alle Reste, die mehrfach auftreten, wie z. B. R⁵, gilt, daß deren Bedeutungen unabhängig voneinander sind.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1- , 2- , 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet besonders bevorzugt unverzweigtes oder verzweigtes Alkyl mit 1-10 C-Atomen, worin 1-7 H-Atome durch OH, F, Cl und/oder Br ersetzt sein können,
oder
cyclisches Alkyl mit 3-7 C-Atomen, das einfach durch OH substituiert sein kann.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. Cyclisches Alkyl (Cycloalkyl) bedeutet vorzugsweise Cyclopropyl, Cyclobutyl, Cylopentyl, Cyclohexyl oder Cycloheptyl.

Cycloalkylen bedeutet vorzugsweise Cyclopropylen, Cyclobutylen, Cylopentylen, Cyclohexylen oder Cycloheptylen.

X¹, X⁴ bedeuten vorzugsweise CH oder N.
X², X³ bedeuten vorzugsweise CH.
X⁵ bedeutet vorzugsweise N, ferner CH.
R¹ bedeutet vorzugsweise H, Hal, A, S(O)ₘA, Ar, Het, O[C(R⁵)₂]ₙAr, O[C(R⁵)₂]ₙHet oder OR5.
R¹ bedeutet besonders bevorzugt H, Hal, A, OR⁵, S(O)ₘA oder
Thiazolyl, Thiophenyl, Furanyl, Pyrrolyl, Oxazolyl, Isoxazolyl, Oxadiazolyl, Pyrazolyl, Imidazolyl, Thiadiazolyl, Pyridazinyl, Pyrazinyl, Pyridinyl, Pyrimidinyl, Pyrazolyloxy,
wobei die Heterocyclen auch ein-, zwei- oder dreifach durch Hal, A und/oder O[C(R⁵)₂]ₚOR⁵ substituiert sein können,
oder
ein-, zwei- oder dreifach durch Hal und/oder CN substituiertes Phenyl oder Phenoxy.
R² bedeutet vorzugsweise A, Hai, [C(R⁵)₂]ₙN(R⁵)₂, [C(R⁵)₂]ₙHet, O[C(R⁵)₂]ₚN(R⁵)₂, O[C(R⁵)₂]ₙHet, [C(R⁵)₂]ₙOR⁵, O[C(R⁵)₂]pOR⁵, O-[C(R⁵)₂]ₙ-cycloalkylen-[C(R⁵)₂]ₙ-N(R⁵)2, [C(R⁵)₂]ₙNR⁵COOA oder CH=CH-COOR⁵.
R³, R^{3'} bedeuten vorzugsweise, jeweils unabhängig voneinander, H oder R⁸, besonders bevorzugt H, Methyl, Ethyl oder Propyl, ganz besonders bevorzugt H oder Methyl.
R⁴, R⁶ bedeuten vorzugsweise H.
R⁷ bedeutet vorzugsweise H oder Hal.
R⁵ bedeutet vorzugsweise H, Methyl, Ethyl oder Propyl, ganz besonders bevorzugt H oder Methyl.
R⁸ bedeutet vorzugsweise Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl oder Hexyl.

Ar bedeutet z.B. Phenyl, o-, m- oder p-Tolyl, o-, m- oder p-Ethylphenyl, o-, m-oder p-Propylphenyl, o-, m- oder p-Isopropylphenyl, o-, m- oder p-tert.-Butylphenyl, o-, m- oder p-Fluorphenyl, o-, m- oder p-Bromphenyl, o-, m- oder p- Chlorphenyl, o-, m- oder p-Cyanphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.

Ar bedeutet besonders bevorzugt unsubstituiertes oder ein-, zwei- oder dreifach durch Hal, A und/oder CN substituiertes Phenyl.

Het bedeutet ganz besonders bevorzugt Piperidinyl, Pyrrolidinyl, Morpholinyl, Piperazinyl, Oxazolidinyl, Pyrazolyl, Pyridinyl, Pyrimidinyl, Furyl, Thienyl, Oxazolyl, Oxadiazolyl, Imidazolyl, Pyrrolyl, Isoxazolyl oder Imidazolidinyl, wobei die Reste auch ein- oder zweifach durch Hal, A, COOR⁵, O[C(R⁵)₂]ₚ-OR⁵, [C(R⁵)₂]ₙHet¹, O[C(R⁵)₂]ₙHet¹ und/oder =O substituiert sein können.

Het¹ bedeutet vorzugsweise Piperidinyl, Pyrrolidinyl, Morpholinyl, Piperazinyl, Oxazolidinyl oder Imidazolidinyl, wobei die Reste auch ein- oder zweifach durch COOA, =O und/oder A substituiert sein können.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind. Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man eine Verbindung der Formel II mit einer Verbindung der Formel III umsetzt. Die Umsetzung erfolgt unter Bedingungen wie sie dem Fachmann für eine Suzuki-Reaktion bekannt sind.

Die Ausgangsverbindungen der Formeln II und III sind in der Regel bekannt. Sind sie neu, so können sie aber nach an sich bekannten Methoden hergestellt werden.
In den Verbindungen der Formel II bedeutet L vorzugsweise

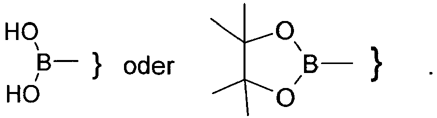

Die Umsetzung erfolgt unter Standardbedingungen einer Suzuki-Kopplung. Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 60° und etwa 90°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Ethanol Toluol, Dimethoxyethan.

Verbindungen der Formel I können weiterhin vorzugsweise erhalten werden, indem man einen Rest R¹ und/oder R⁷ durch einen anderen Rest R¹ und/oder R⁷ austauscht. Vorzugsweise wird ein Halogenatom gegen einen Rest Het und/oder Ar, die die in Anspruch 1 angegebenen Bedeutungen haben, ausgetauscht. Die Umsetzung erfolgt vorzugsweise unter den Bedingungen einer Suzuki-Kopplung.

Verbindungen der Formel I können weiterhin vorzugsweise erhalten werden, indem man eine Verbindung der Formel IV vorzugsweise mit NaNO₂ umsetzt. Die Umsetzung erfolgt unter Standardbedingungen.
Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 60° und etwa 90°.
Als inerte Lösungsmittel eignen sich die oben genannten.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen.

Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 % igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt.

Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird.

Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie deren Salze davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung der erfindungsgemäßen Verbindungen nach Anspruch 1 und/oder ihre physiologisch unbedenklichen Salze zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I und/oder ihre physiologisch unbedenklichen Salze zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.

Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I können an Patienten zur Behandlung von Krebs, insbesondere schnell wachsenden Tumoren, verabreicht werden.

Besonders bevorzugt ist die Verwendung zur Herstellung eines Arzneimittels zur Behandlung von Krankheiten, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Herstellung eines Arzneimittels zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανß3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und Invivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffmann-La Roche |
| | Ormiplatin | SM-11355 (Sumitomo) |
| | Iproplatin | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothec | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang) |
| | BBR-3576 (Novuspharrna) | KW-2170 (Kyowa Hakko) |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubicin | GPX-100 (Gem Pharmaceutical |
| | Mitoxantron (Novantron) | |
| | | |
| Antimitotische Mittel | Paclitaxel | SB 408075 (GlaxoSmithKline) |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B |
| | RPR 109881A (Aventis) | (PharmaMar) |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | DN-5109 (Indena) |
| | Cryptophycin 52 (Eli Lilly) | AVLB (Prescient NeuroPharma) |
| | Vinflunin (Fabre) | Azaepothilon B (BMS) |
| | Auristatin PE (Teikoku Hormor | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | Thymectacin (NewBiotics) | O6-Benzylguanin (Paligent) |
| | Edotreotid (Novartis) | |
| | | |
| Farnesyltransferase-Inhibitoren | Arglabin (NuOncology Labs) | Tipifarnib (Johnson & Johnson) |
| | lonafarnib (Schering-Plough) | Perillylalkohol (DOR BioPharma |
| | BAY-43-9006 (Bayer) | |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransferase-Inhibitoren | Tacedinalin (Pfizer) | Pivaloyloxymethylbutyrat (Titan; |
| | SAHA (Aton Pharma) | Depsipeptid (Fujisawa) |
| | MS-275 (Schering AG) | |
| | | |
| Metalloproteinase-Inhibitoren Ribonucleosidreduktase-Inhibitoren | Neovastat (Aeterna Laboratori | CMT -3 (CollaGenex) |
| | Marimastat (British Biotech) | BMS-275291 (Celltech) |
| | Galliummaltolat (Titan) | Tezacitabin (Aventis) |
| | Triapin (Vion) | Didox (Molecules for Health) |
| TNF-alpha-Agonisten / Antagonisten | Virulizin (Lorus Therapeutics) | Revimid (Celgene) |
| | CDC-394 (Celgene) | |
| | | |
| Endothelin-A-Rezept Antagonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys) |
| | Oncophage (Antigenics) | Pentrix (Australian Cancer Technology) |
| | GMK (Progenics) | JSF-154 (Tragen) |
| | Adenokarzinom-Impfstoff (Biomira) | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | 3-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | Arzoxifen (Eli Lilly) |
| | Dexamethason | |
| | | |
| Photodynamische Mittel | Talaporfin (Light Sciences) | Pd-Bacteriopheophorbid (Yeda) |
| | Theralux (Theratechnologies) | Lutetium-Texaphyrin (Pharmacyclics) |
| | Motexafin-Gadolinium (Pharmacyclics) | |
| | | Hypericin |
| | | |
| Tyrosinkinase-Inhibitoren | Imatinib (Novartis) | Kahalid F (PharmaMar) |
| | Leflunomid (Sugen/Pharmacia | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | 2C4 (Genentech) |
| | PKI166 (Novartis) | MDX-447 (Medarex) |
| | GW2016 (GlaxoSmithKline) | ABX-EGF (Abgenix) |
| | EKB-509 (Wyeth) | IMC-1C11 (ImClone) |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | Tocladesin (cyclisches-AMP-Agonist, Ribapharm) | Ranpirnase (Ribonuclease-Stimulans, Alfacell) |
| | Alvocidib (CDK-Inhibitor, Avent | Galarubicin (RNA-Synthese-Inhibitor, Dong-A) |
| | CV-247 (COX-2-Inhibitor, Ivy Medical) | Tirapazamin (Reduktionsmittel, SRI International) |
| | P54 (COX-2-Inhibitor, Phytopharm) | |
| | | N-Acetylcystein (Reduktionsmitt Zambon) |
| | CapCell™ (CYP450-Stimulans Bavarian Nordic) | |
| | | R-Flurbiprofen (NF-kappaB-Inhibitor, Encore) |
| | GCS-IOO (gal3-Antagonist, GlycoGenesys) | |
| | | 3CPA (NF-kappaB-Inhibitor, Act Biotech) |
| | G17DT-Immunogen (Gastrin-Inhibitor, Aphton) | |
| | | Seocalcitol (Vitamin-D-Rezeptor Agonist, Leo) |
| | Efaproxiral (Oxygenator, Allos Therapeutics) | |
| | | 131-I-TM-601 (DNA-Antagonist, TransMolecular) |
| | PI-88 (Heparanase-Inhibitor, Progen) | |
| | | Eflornithin (ODC-Inhibitor, ILEX Oncology) |
| | Tesmilifen (Histamin-Antagonis YM BioSciences) | |
| | | Minodronsäure (Osteoclasten-Inhibitor, Yamanouchi) |
| | Histamin (Histamin-H2-Rezept Agonist, Maxim) | |
| | | Indisulam (p53-Stimulans, Eisai |
| | Tiazofurin (IMPDH-Inhibitor, Ribapharm) | Aplidin (PPT-Inhibitor, PharmaMar) |
| | Cilengitid (Integrin-Antagonist, Merck KGaA) | Rituximab (CD20-Antikörper, Genentech) |
| | SR-31747 (IL-1-Antagonist, Sanofi-Synthelabo) | Gemtuzumab (CD33-Antikörper Wyeth Ayerst) |
| | CCI-779 (mTOR-Kinase-Inhibitor, Wyeth) | PG2 (Hämatopoese-Verstärker, Pharmagenesis) |
| | Exisulind (PDE-V-Inhibitor, Cell Pathways) | Immunol™ (Triclosan-Oralspülung, Endo) |
| | CP-461 (PDE-V-Inhibitor, Cell Pathways) | Triacetyluridin (Uridin-Prodrug, Wellstat) |
| | AG-2037 (GART-Inhibitor, Pfizer) | SN-4071 (Sarkom-Mittel, Signature BioScience) |
| | WX-UK1 (Plasminogenaktivator- Inhibitor, Wilex) | TransMID-107™ (Immunotoxin, KS Biomedix) |
| | PBI-1402 (PMN-Stimulans, ProMetic LifeSciences) | PCK-3145 (Apoptose-Förderer, Procyon) |
| | Bortezomib (Proteasom-Inhibitor, Millennium) | Doranidazol (Apoptose-Förderer, Pola) |
| | SRL-172 (T-Zell-Stimulans, SR Pharma) | CHS-828 (cytotoxisches Mittel, Leo) |
| | TLK-286 (Glutathion-S-Transferase-Inhibitor, Telik) | trans-Retinsäure (Differentiator, NIH) |
| | PT-100 (Wachstumsfaktor-Agonist, Point Therapeutics) | MX6 (Apoptose-Förderer, MAXIA) |
| | Midostaurin (PKC-Inhibitor, Novartis) | Apomin (Apoptose-Förderer, ILEX Oncology) |
| | Bryostatin-1 (PKC-Stimulans, GPC Biotech) | Urocidin (Apoptose-Förderer, Bioniche) |
| | CDA-II (Apoptose-Förderer, Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer ChemGenex) | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity-(Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidasekonjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert.

Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer). Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

Kinase-Reaktionsbedingungen pro well:
30 µl Assaypuffer
10 µl zu testende Substanz in Assaypuffer mit 10 % DMSO
10 µl ATP (Endkonzentration 1 µM kalt, 0,35 µCi ³³P-ATP)
50 µl Gemisch Met Kinase/Substrat in Assaypuffer; (10 ng Enzym/well, 50 ng pAGLT/well)

Verwendete Lösungen:
- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

### In vivo-Tests

Experimenteller Ablauf: Weibliche Balb/C Mäuse (Züchter: Charles River Wiga) waren bei der Ankunft im Alter von 5 Wochen. Sie wurden 7 Tage lang an unsere Haltungsbedingungen akklimatisiert. Anschließend wurden jeder Maus 4 Millionen TPR-Met / NIH3T3 - Zellen in 100 µl PBS (ohne Ca++ und Mg++) subkutan im Beckenbereich injiziert. Nach 5 Tagen wurden die Tiere in 3 Gruppen randomisiert, so dass jede Gruppe von 9 Mäusen ein mittleres Tumorvolumen von 110 µl (Spanne: 55 - 165) hatte. Der Kontrollgruppe wurden 100 µl Vehikel (0,25 % Methylzellulose / 100 mM Acetatpuffer, pH 5.5), den Behandlungsgruppen wurden 200 mg/kg "A56" bzw. "A91" gelöst im Vehikel (Volumen ebenfalls 100 µl / Tier) per Schlundsonde täglich verabreicht. Nach 9 Tagen hatten die Kontrollen ein mittleres Volumen von 1530 µl und der Versuch wurde beendet.

Messung des Tumorvolumens: Die Länge (L) und Breite (B) wurde mit einer Schubleere gemessen und das Tumorvolumen nach der Formel LxBxB/2 berechnet.

Haltungsbedingungen: je 4 bzw. 5 Tiere pro Käfig, Fütterung mit kommerziellem Mäusefutter (Fa. Sniff).

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-Ionisation) M⁺
   FAB (Fast Atom Bombardment) (M+H)⁺
   ESI (Electrospray lonization) (M+H)⁺
APCI-MS (atmospheric pressure chemical ionization - mass spectrometry) (M+H)⁺.

### HPLC-Methoden:

### Methode A:

Flußrate: 2 ml/min
99:01 - 0:100 Wasser + 0.1 %(Vol.) TFA : Acetonitril + 0.1 %(Vol.) TFA 0.0 bis 0.2 min: 99:01
0.2 bis 3.8 min: 99:01---> 0:100
3.8 bis 4.2 min: 0:100
Säule: Chromolith Performance RP18e; 100 mm lang, Innendurchmesser 3 mm, Wellenlänge: 220nm
Retentionszeit Rt. in Minuten [min].

### Methode B:

Gradient: 4.2 min/ Fluss: 2 ml/min
99% (A) : 1 % (B) - 0:100 Wasser + 0.01 %(Vol.) AS (A) : Acetonitril + 0.01%(Vol.) AS (B)
0.0 bis 0.2 min: 99:01
0.2 bis 3.8 min 99:01 - 0:100
3.8 bis 4.2 min 0:100

### Methode C:

Gradient: 4.2 min/ Fluss: 2 ml/min
99% (A) : 1 % (B) - 0:100
   Wasser + 0.05 %(Vol.) AS (A): Acetonitril + 0.04%(Vol.) AS (B)
   0.0 bis 0.2 min: 99:01
   0.2 bis 3.8 min 99:01 - 0:100
3.8 bis 4.2 min 0:100

### BEISPIELE

### Herstellung der Benzylalkohole

### Herstellung von {3-[5-(3-Dimethylamino-propoxy)-pyrimidin-2-yl]-phenyl}-methanol

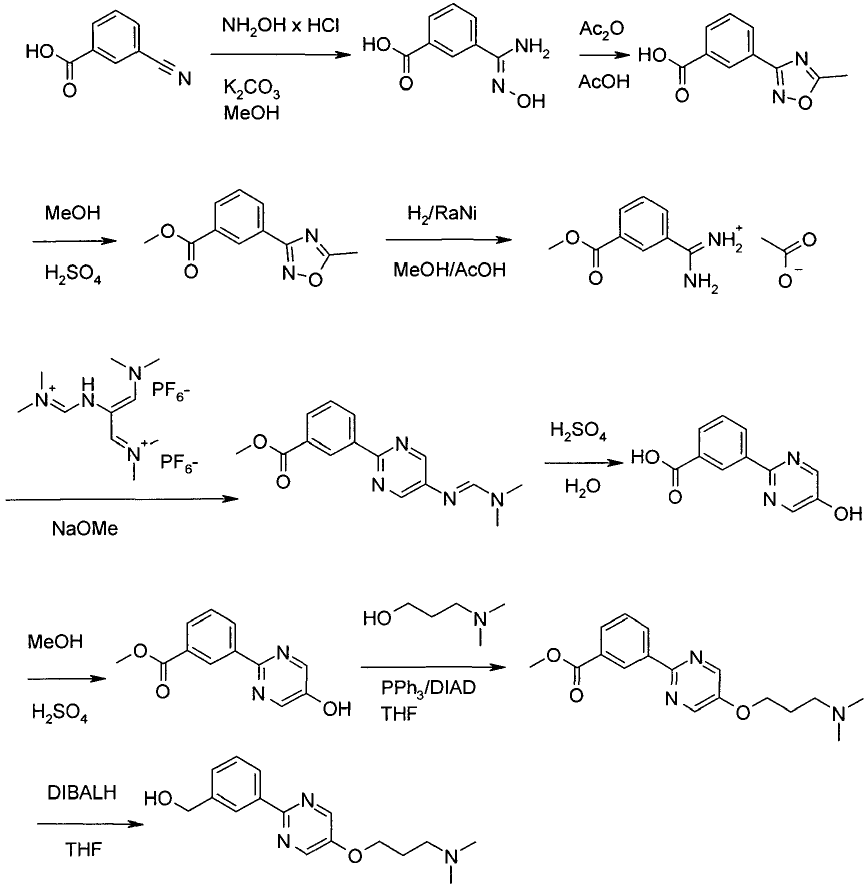

### Stufe 1:

Zu einer auf 30°C gehaltenen Suspension von 500 g (3.40 mol) 3-Cyanbenzoesäure in 8 l Methanol werden unter Rühren portionsweise 1382 g (10.0 mol) Kaliumcarbonat gegeben. Anschließend werden bei einer Innentemperatur von 40 - 45°C 695 g (10.0 mol) Hydroxylammoniumchlorid in kleinen Portionen zugegeben. Dann wird das Reaktionsgemisch 15 Stunden zum Sieden erhitzt. Das Reaktionsgemisch wird im Vakuum eingeengt, der Rückstand in Wasser gelöst und mit 37%iger wässriger Salzsäure angesäuert. Der entstandene Niederschlag wird abgesaugt, mit Wasser gewaschen und im Vakuum getrocknet: 3-(N-Hydroxycarbamimidoyl)-benzoesäure als farblose Kristalle; LCMS 181.

### Stufe 2:

Ein Gemisch von 614 g (3.41 mol) 3-(N-Hydroxycarbamimidoyl)-benzoesäure, 756 ml (8.0 mol) Essigsäureanhydrid und 2 l Essigsäure wird 14 Stunden auf eine Temperatur von 118°C erhitzt. Das Reaktionsgemisch wird auf 6°C gekühlt und abgesaugt. Der Rückstand wird in 2 l Wasser aufgenommen, abgesaugt und gut mit Wasser gewaschen. Der Rückstand wird aus Ethanol / Wasser umkristallisiert: 3-(5-Methyl-[1,2,4]oxadiazol-3-yl)-benzoesäure als farblose Kristalle; F. 225°C; LCMS 205.

### Stufe 3:

Eine Suspension von 30.0 g (147 mmol) 3-(5-Methyl-[1,2,4]oxadiazol-3-yl)-benzoesäure in 150 ml Methanol wird mit 7.83 ml (147 mmol) konzentrierter Schwefelsäure versetzt und 18 Stunden zum Sieden erhitzt. Das Reaktionsgemisch wird im Eisbad gekühlt, mit Wasser versetzt, abgesaugt und gut mit Wasser gewaschen: 3-(5-Methyl-[1,2,4]oxadiazol-3-yl)-benzoesäuremethyl-ester als farblose Kristalle; LCMS 219.

### Stufe 4:

Eine Lösung von 327 g (1.47 mol) 3-(5-Methyl-[1,2,4]oxadiazol-3-yl)-benzoesäuremethylester in 3 l Methanol wird mit 150 ml Essigsäure, 150 ml Wasser und 50 g wasserfeuchtem Raney-Nickel versetzt und 18 Stunden bei Raumtemperatur und Normaldruck hydriert. Der Katalysator wird abfiltriert und das Filtrat eingedampft. Der Rückstand wird in tert.-Butylmethylether aufgenommen, zum Sieden erhitzt und abgesaugt. Der Rückstand wird im Vakuum getrocknet: 3-Methoxycarbonylbenzamidinium-Acetat als farblose Kristalle; LCMS 179.

### Stufe 5:

Zu einer Suspension von 259 g (1.09 mol) 3-Methoxycarbonylbenzamidinium-Acetat und 528 g (1.08 mol) ({2-Dimethylamino-1-[dimethylimmoniomethyl]-vinylamino}-methylen)-dimethyl-ammonium-di-hexafluorophosphat (hergestellt gemäß C. B. Dousson et al., Synthesis 2005, 1817) in 1 l Methanol werden unter Rühren 2.2 l einer frisch bereiteten 1.5 M Natriummethanolat-Lösung zugetropft. Dann wird das Reaktionsgemisch innerhalb von 40 min auf 60°C erwärmt und 30 min bei dieser Temperatur gehalten. Dann wird das Reaktionsgemisch auf Raumtemperatur abgekühlt, mit 10 l Dichlormethan verdünnt und dreimal mit je 5 l Wasser gewaschen. Die organische Phase wird über Natriumsulfat getrocknet und eingedampft. Der Rückstand wird aus Ethylacetat umkristallisiert: 3-[5-(Dimethylamino-methylenamino)-pyrimidin-2-yl]-benzoesäuremethylester als beige Kristalle; F. 140°C; LCMS 285.

### Stufe 6:

Eine Suspension von 103.5 g (364 mmol) 3-[5-(Dimethylamino-methylen-amino)-pyrimidin-2-yl]-benzoesäuremethylester in 1.3 l Wasser wird mit 160 ml (2.88 mol) konzentrierter Schwefelsäure versetzt und 4 Stunden zum Sieden erhitzt. Das Reaktionsgemisch wird auf Raumtemperatur abgekühlt, mit Wasser verdünnt und abgesaugt. Der Rückstand wird mit Wasser gewaschen und im Vakuum getrocknet: 3-(5-Hydroxypyrimidin-2-yl)-benzoesäure als bräunliche Kristalle; LCMS 217.

### Stufe 7:

Eine Suspension von 88.0 g (366 mmol) 3-(5-Hydroxypyrimidin-2-yl)-benzoesäure in 1.4 I Methanol wird mit 32.7 ml (445 mmol) Thionylchlorid versetzt und 2 Stunden auf 80°C erhitzt. Dann werden 20 ml (276 mmol) Thionylchlorid und nach 2 Stunden noch mal 10 ml (138 mmol) Thionylchlorid zugegeben. Nach jeder Zugabe wird das Reaktionsgemisch 2 Stunden bei 80°C gerührt. Das Reaktionsgemisch wird im Vakuum auf ein Volumen von ca. 300 ml eingeengt. Der entstandene Niederschlag wird abfiltriert und im Vakuum getrocknet: 3-(5-Hydroxypyrimidin-2-yl)-benzoesäuremethylester als bräunliche Kristalle; LCMS 231.

### Stufe 8:

Eine unter Stickstoff gehaltene Lösung von 6.1 g (26.5 mmol) 3-(5-Hydroxy-pyrimidin-2-yl)-benzoesäuremethylester, 10.5 g (39.8 mmol) Triphenylphosphin und 4.76 ml (39.8 mmol) 3-(Dimethylamino)-1-propanol in 200 ml THF wird im Eisbad gekühlt und langsam unter Rühren 8.21 ml (39.8 mmol) Diisopropylazodicarboxylat zugetropft. Nach 2 Stunden Rühren bei Raumtemperatur wird das Reaktionsgemisch im Vakuum eingedampft. Der Rückstand wird zwischen Dichlormethan und gesättigter wässriger Kaliumhydrogensulfat-Lösung verteilt. Die wässrige Phase wird abgetrennt, mit gesättigter wässriger Natronlauge auf einen pH-Wert von 12 gebracht und zweimal mit Dichlormethan extrahiert. Die organische Phase wird über Natriumsulfat getrocknet und eingedampft. Der Rückstand wird an einer Kieselgelsäule mit Dichlormethan/Methanol als Laufmittel chromatographiert: 3-[5-(3-Dimethylamino-propoxy)-pyrimidin-2-yl]-benzoesäuremethylester als farblose Kristalle; LCMS 316.

### Stufe 9:

Zu einer unter Stickstoff gehaltenen Lösung von 12.6 g (40.0 mmol) 3-[5-(3-Dimethylamino-propoxy)-pyrimidin-2-yl]-benzoesäuremethylester in 200 ml THF werden unter Rühren 200 ml einer 1 M Lösung von Diisobutylaluminiumhydrid in THF zugetropft. Nach 1 Stunde Rühren bei Raumtemperatur werden 10 ml einer gesättigten wässrigen Natriumsulfat-Lösung zugetropft. Der entstandene Niederschlag wird abgesaugt und mit Dichlormethan gewaschen. Das Filtrat wird über Natriumsulfat getrocknet und eingedampft. Der Rückstand wird in einem Gemisch von Diethylether und Petrolether aufgenommen. Der entstandene Niederschlag wird abgesaugt, mit Petrolether gewaschen und im Vakuum getrocknet: {3-[5-(3-Dimethylamino-propoxy)-pyrimidin-2-yl]-phenyl}-methanol als weiße Kristalle; F. 103-104 °C; LCMS 288; Rt. = 1.76 min (Methode A).

**Analog kann hergestellt werden:**

| Verbindung Nr. | Name und/oder Struktur | LCMS [M+H] | Rt. in min |
|---|---|---|---|
| | 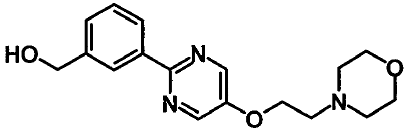 | 316 | 1.73 (Methode A) |
| | 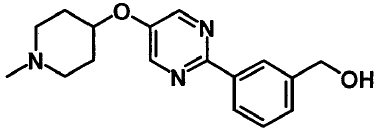 | 300 | |
| | 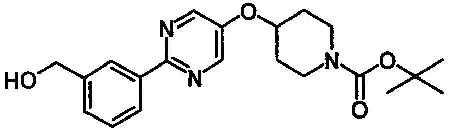 | 386 | |
| | 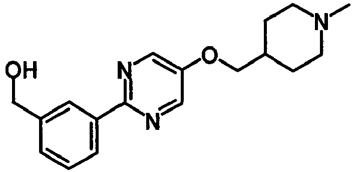 | 314 | |
| | 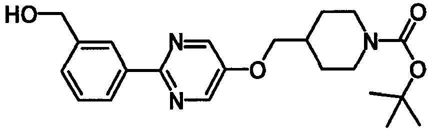 | 400 | |

### Alternative Syntheseroute zur Herstellung des 3-(5-Hydroxy-pyrimidin-2-yl)-benzoesäuremethylester

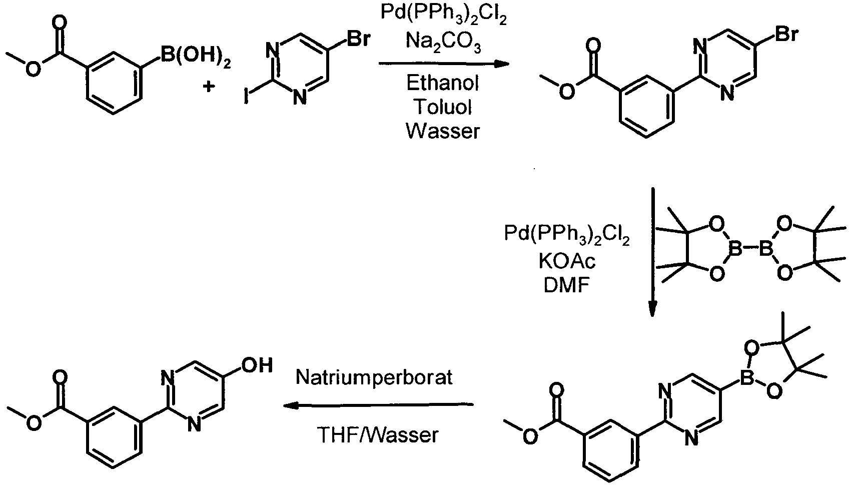

### Stufe 1:

Eine Lösung von 14.5 g (50.5 mmol) 5-Brom-2-iodpyrimidin in 50 ml Toluol wird mit einer Lösung von 10.6 g (100 mmol) Natriumcarbonat in 50 ml Wasser versetzt und unter Stickstoff auf 80°C erwärmt. Dann werden 351 mg (0.50 mmol) Bis(triphenylphosphin)-palladium(II)-chlorid und eine Lösung von 9.18 g (50.0 mmol) (3-Methoxycarbonylphenyl)-boronsäure in 75 ml Ethanol zugegeben und die entstandene Suspension 24 Stunden bei 80°C gerührt. Das Reaktionsgemisch wird im Vakuum eingedampft und der Rückstand wird zwischen Wasser und Ethylacetat verteilt. Die organische Phase wird über Natriumsulfat getrocknet und eingedampft. Der Rückstand wird in 100 ml Methanol aufgenommen und mit 5.30 g (50 mmol) Natriumcarbonat versetzt. Die entstandene Suspension wird 32 Stunden zum Rückfluss erhitzt. Nach Abkühlen auf Raumtemperatur wird abgesaugt. Der Rückstand wird mit Methanol und Wasser gewaschen und im Vakuum getrocknet: 3-(5-Brompyrimidin-2-yl)-benzoesäuremethylester als sandfarbene Kristalle; LCMS 293/295;
¹H-NMR (d₆-DMSO): ö [ppm] = 3.91 (s, 3H), 7.71 (t, J = 7.8 Hz, 1 H), 8.14 (dt, J = 7.5 Hz, J = 1.5 Hz, 1 H), 8.61 (dt, J = 7.9 Hz, J = 1.4 Hz, 1 H), 8.96 (t, J = 1.7 Hz, 1H), 9.13 (s, 2H).

### Stufe 2:

Eine Lösung von 7.44 g (25.4 mmol) 3-(5-Brompyrimidin-2-yl)-benzoesäure-methylester und 7.26 g (27.9 mmol) Bis-(pinacolato)-dibor in 50 ml DMF wird mit 7.47 g (76.2 mmol) Kaliumacetat versetzt und unter Stickstoff auf 80°C erhitzt. Dann werden 535 mg (0.76 mmol) Bis(triphenylphosphin)-palladium(II)-chlorid zugegeben und 18 Stunden bei 80°C gerührt. Das Reaktionsgemisch wird zwischen Wasser und Dichlormethan verteilt. Die organische Phase wird über Natriumsulfat getrocknet und eingedampft. Der Rückstand wird mit tert.Butylmethylether erhitzt, abkühlen lassen und abgesaugt und mit tert.Butylmethylether gewaschen und im Vakuum getrocknet: 3-[5-(4,4,5,5-Tetramethyl-[1,3,2]dioxaborolan-2-yl)-pyrimidin-2-yl]-benzoesäuremethylester als beige Kristalle;
¹H-NMR (d₆-DMSO): ö [ppm] = 1.35 (s, 12H), 3.92 (s, 3H), 7.72 (t, J = 7.8 Hz, 1 H), 8.15 (dt, J = 7.5 Hz, J = 1.5 Hz, 1 H), 8.69 (dt, J = 7.9 Hz, 1.4 Hz, 1H), 9.04 (t, J = 1.7 Hz, 1H), 9.07 (s, 2H).

### Stufe 3:

Eine Lösung von 1.93 g (5.39 mmol) 3-[5-(4,4,5,5-Tetramethyl-[1,3,2]dioxa-borolan-2-yl)-pyrimidin-2-yl]-benzoesäuremethylester in 13 ml THF wird mit einer Suspension von 1.24 g (8.09 mmol) Natriumperborat-Tetrahydrat in 13 ml Wasser versetzt und die entstandene zweiphasige Mischung 18 Stunden bei Raumtemperatur gerührt. Das Reaktionsgemisch wird filtriert und das Filtrat im Vakuum auf ca. die Hälfte des ursprünglichen Volumens eingedampft. Es wird erneut filtriert und das Filtrat mit 10 ml 1 N Salzsäure angesäuert. Der entstandene Niederschlag wird abgesaugt, mit Wasser gewaschen und im Vakuum getrocknet: 3-(5-Hydroxypyrimidin-2-yl)-benzoe-säuremethylester als hellgelbe Kristalle; LCMS 231;
1 H-NMR (d₆-DMSO): δ [ppm] = 3.91 (s, 3H), 7.64 (t, J = 7.8 Hz, 1 H), 8.02 (dt, J = 7.5 Hz, 1.5 Hz, 1H), 8.49 (s, 2H) 8.52 (dt, J = 7.9 Hz, 1.4 Hz, 1H), 8.89 (t, J = 1.7 Hz, 1H), 10.7 (bs, 1H).

### Herstellung von [3-(5-Methyl-pyridin-2-yl)-phenyl]-methanol

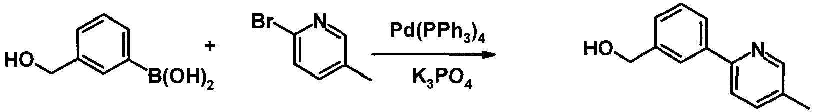

Zu einer unter Stickstoff gehaltenen Suspension von 849 mg (4.0 mmol) Trikaliumphosphat, 344 mg (2.0 mmol) 2-Brom-5-methylpyridin und 304 mg (2.0 mmol) 3-Hydroxymethylbenzolboronsäure in 12 ml Dioxan und 1 ml Wasser werden 92 mg (0.08 mmol) Tetrakis(triphenylphosphin)-palladium gegeben und das Gemisch 18 Stunden unter Rühren zum Sieden erhitzt. Das Reaktionsgemisch wird auf Raumtemperatur abgekühlt und zwischen Wasser und Ethylacetat verteilt. Die organische Phase wird über Natriumsulfat getrocknet, eingedampft und der Rückstand an einer Kieselgelsäule mit Dichlormethan/Methanol als Laufmittel chromatographiert: [3-(5-Methyl-pyridin-2-yl)-phenyl]-methanol als gelbliches Öl; LCMS 200.

### Herstellung von [3-5-Methyl-pyrimidin-2-yl)-phenyl]-methanol

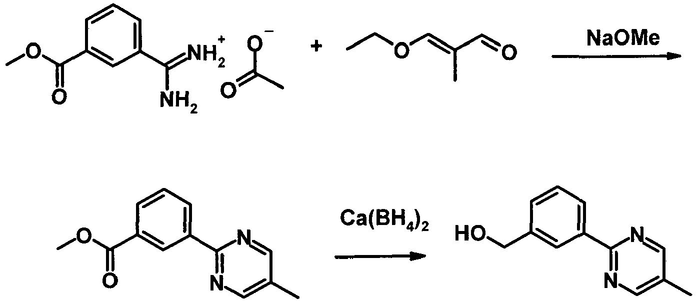

### Stufe 1:

Eine Suspension von 2.41 g (10.0 mmol) 3-Carbamimidoyl-benzoesäuremethylester-Acetat in 40 ml Methanol wird mit 1.31 ml (11.0 mmol) 3-Ethoxymethacrolein und 2.04 ml (11.0 mmol) einer 30%igen Lösung von Natriumethanolat in Methanol versetzt und die resultierende Lösung 18 Stunden bei 50°C gerührt. Das Reaktionsgemisch wird im Vakuum eingeengt und mit Wasser versetzt. Der entstandene Niederschlag wird abgesaugt, mit Wasser gewaschen und im Vakuum getrocknet: 3-(5-Methylpyrimidin-2-yl)-benzoesäuremethylester als farblose Kristalle; LCMS 229.

### Stufe 2:

Zu einer Suspension von 400 mg (10.6 mmol) Natriumborhydrid in 20 ml THF werden 600 mg (5.41 mmol) gepulvertes Calciumchlorid gegeben und das Gemisch 1.5 Stunden bei Raumtemperatur gerührt. Zu dieser Suspension wird unter Rühren eine Lösung von 751 mg (3.29 mmol) 3-(5-Methylpyrimidin-2-yl)-benzoesäuremethylester in 10 ml THF zugetropft und 18 Stunden bei Raumtemperatur gerührt. Das Reaktionsgemisch wird 10 ml 1 N NaOH, Wasser und Dichlormethan versetzt und filtriert. Die organische Phase des Filtrats wird abgetrennt, über Natriumsulfat getrocknet und eingedampft. Der Rückstand wird an einer Kieselgelsäule mit Dichlormethan/Methanol als Laufmittel chromatographiert: [3-(5-Methylpyrimidin-2-yl)-phenyl]-methanol als farbloser Feststoff; LCMS 201.

### Herstellung von [3-(5-Brom-pyrimidin-2-yl)-phenyl]-methanol

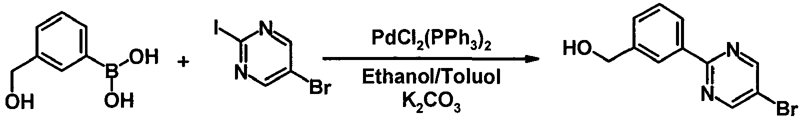

Eine unter Stickstoff gehaltene Lösung von 95.0 g (332 mmol) 5-Brom-2-iodpyrimidin in 325 ml Toluol wird mit einer Lösung von 70.0 g (660 mmol) Natriumcarbonat in 325 ml Wasser versetzt und das Gemisch auf 80° C erhitzt. Dazu werden 2.3 g (3.3 mmol) Bis(triphenylphosphin)palladium(II)-chlorid gegeben und anschließend eine Lösung von 50.0 g (329 mmol) 3-(Hydroxymethyl)-benzolboronsäure in 650 ml Ethanol zugetropft. Das Reaktionsgemisch wird 18 Stunden bei 80° C gerührt. Das Reaktionsgemisch wird auf Raumtemperatur abgekühlt und filtriert. Das Filtrat wird mit 1 I Ethylacetat und 1 I Wasser versetzt. Die organische Phase wird abgetrennt, über Natriumsulfat getrocknet und eingedampft. Der Rückstand wird aus 2-Propanol umkristallisiert: [3-(5-Brompyrimidin-2-yl)-phenyl]-methanol als hellgelbe Kristalle; F. 115-116°C; LCMS 265,267.

### Herstellung von (E)-3-[2-(3-Hydroxymethyl-phenyl)-pyrimidin-5-yl]-acrylsäuremethylester

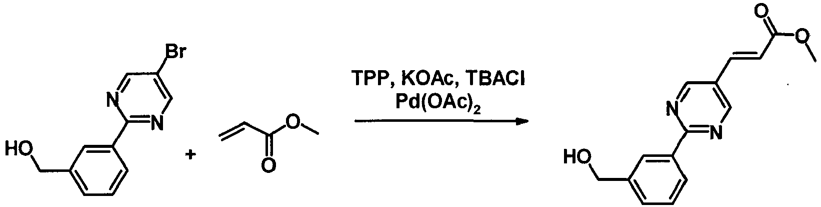

100 mg (0.38 mmol) [3-(5-Brom-pyrimidin-2-yl)-phenyl]-methanol und 51 µl (0.56 mmol) Methylacrylat werden in 2 ml DMF suspendiert und mit 20 mg (0.075 mmol) Triphenylphosphin, 222 mg (2.26 mmol) Kaliumacetat und 157 mg (0.57 mmol) Tetra-*n*-butylammoniumchlorid versetzt. Das Reaktionsgemisch wird entgast, mit Argon gespült und unter Argonatmosphäre mit 17 mg (0.075 mmol) Palladium(II)-acetat versetzt. Es wird 2 h auf 80°C erhitzt. Nach dem Abkühlen wird mit Wasser versetzt, wobei sich ein hellgrauer Niederschlag bildet. Dieser wird abgesaugt, mit Wasser gewaschen und im Vakuum getrocknet. Das Produkt wird ohne weitere Aufreinigung weiter umgesetzt; Ausbeute: 111 mg; HPLC: Rt. = 2.42 min (Methode A); LC-MS: 271 (M+H).

### Herstellung von {(E)-3-[2-(3-Hydroxymethyl-phenyl)-pyrimidin-5-yl]-allyl}-carbaminsäure-tert.-butylester

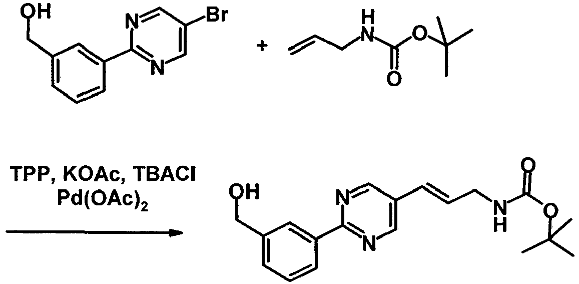

812 mg (3.06 mmol) [3-(5-Brom-pyrimidin-2-yl)-phenyl]-methanol und 722 mg (4.59 mmol) tert.-Butyl-N-allylcarbamat werden in 16 ml DMF suspendiert und mit 160 mg (0.61 mmol) Triphenylphosphin, 1.8 g (4.6 mmol) Kaliumacetat und 1.28 g (4.59 mmol) Tetra-n-butylammoniumchlorid versetzt. Das Reaktionsgemisch wird entgast und mit Argon gespült und unter Argonatmosphäre mit 137 mg (0.0.61 mmol) Palladium(II)-acetat versetzt. Es wird 2 h auf 80°C erhitzt. Nach dem Abkühlen wird über Kieselgur abgesaugt und das Filtrat in Wasser gegeben und mit 2 x 100 ml Ethylacetat extrahiert, über Natriumsulfat getrocknet und eingedampft. Das Produkt wurde ohne weitere Aufreinigung weiter umgesetzt.
Ausbeute: 380 mg; HPLC: Rt. = 2.66 min (Methode A); LC-MS: 342 (M+H).

### Herstellung von {3-[2-(3-Hydroxymethyl-phenyl-pyrimidin-5-yl]-propyl}-carbaminsäure-tert.-butylester

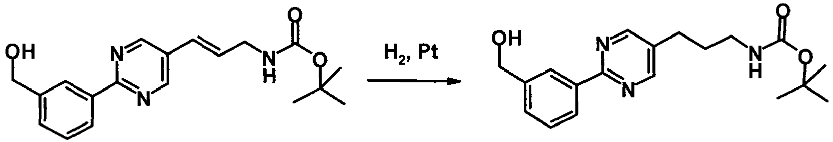

280 mg (0.82 mmol) {(E)-3-[2-(3-Hydroxymethyl-phenyl)-pyrimidin-5-yl]-allyl}-carbamsäure-tert.-butylester werden in 10 ml THF gelöst, mit 300 mg Platin auf Aktivkohle (5%, enthält 56 % Wasser) unter Wasserstoffatmosphäre 17 h bei Raumtemperatur geschüttelt. Der Katalysator wird abgesaugt und das Filtrat zum Rückstand eingedampft.
Ausbeute: 289 mg; HPLC: Rt. = 2.60 min (Methode A), LC-MS: 344 (M+H).

### Herstellung von {3-[5-(4-Methyl-piperazin-1-yl)pyrimidin-2-yl]-phenyl]-methanol

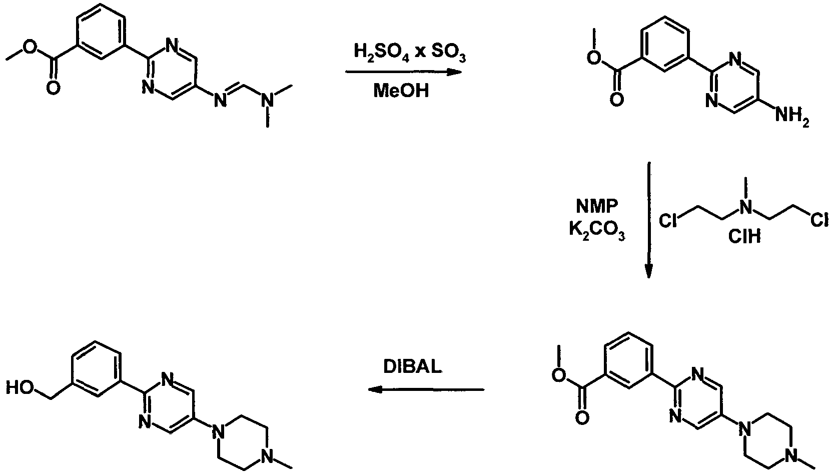

### Stufe 1:

10.2 g (35.9 mmol) 3-[5-(Dimethylamino-methylenamino)-pyrimidin-2-yl]-benzoesäuremethylester werden in 1 I Methanol suspendiert. Unter leichter Kühlung (ca. 5-10°C) werden 5.3 ml (107.3 mmol) rauchende Schwefelsäure tropfenweise zugetropft (Achtung, stark exotherme Reaktion). Nach beendeter Zugabe wird zunächst 30 min bei RT und anschließend bei 88°C Ölbadtemperatur gerührt. Die Reaktion wird mittels HPLC verfolgt. Nach 20 h wird die klare, dunkelgelbe Lösung zum Rückstand abgezogen. Der Rückstand wird in 600 ml Ethylacetat gelöst und mit 2 x 150 ml 1 N NaOH und 2 x 1 N HCl gewaschen, über Natriumsulfat getrocknet und eingedampft.
Ausbeute: 3 g; HPLC: Rt. = 2.17 min (Methode A); LC-MS: 300 (M+H).

### Stufe 2:

2.5 g (10.9 mmol) 3-(5-Amino-pyrimidin-2-yl)-benzoesäuremethylester werden in 10 ml NMP gelöst, mit 2.59 g (18.5 mmol) Kaliumcarbonat und 3.6 g (18.5 mmol) Bis-(2-chlor-ethyl)-ethyl-amin Hydrochlorid versetzt. Die Suspension wird unter Argonatmosphäre 15 h bei 120°C gerührt. Anschließend wird weitere 12 h bei 140°C gerührt. Nach dem Abkühlen auf Raumtemperatur wird das Reaktionsgemisch in 150 ml Wasser eingerührt. Der entstandene Niederschlag wird über Kieselgur abgesaugt und verworfen. Das Filtrat wird mit 32%-iger NaOH auf pH=14 eingestellt. Die leicht getrübte Lösung wird mit 2 x 200 ml Ethylacetat extrahiert. Die vereinigten organischen Phasen werden mit gesättigter Natriumchloridlösung gewaschen, über Natriumsulfat getrocknet und zum Rückstand eingeengt und im Vakuum getrocknet. Das Produkt wird ohne weitere Aufreinigung weiter umgesetzt.
Ausbeute: 860 mg; HPLC: Rt. = 2.11 min (Methode A); LC-MS: 313 (M+H).

### Stufe 3:

860 mg (2.75 mmol) 3-[5-(4-Methyl-piperazin-1-yl)-pyrimidin-2-yl]-benzoe-säuremethylester werden in 16 ml THF gelöst und bei Raumtemperatur werden 13.8 ml (13.8 mmol) 1 M Diisobutylaluminiumhydrid in THF zugetropft und das Reaktionsgemisch wird 1 h bei Raumtemperatur gerührt. Es werden weitere 13.8 ml (13.8 mmol) 1 M Diisobutylaluminiumhydrid in THF zugetropft und das Reaktionsgemisch wird 1 h bei Raumtemperatur gerührt. Das Reaktionsgemisch wird unter Eiskühlung mit 3 ml gesättigter Natriumsulfatlösung versetzt. Das gelartige Gemisch wird mit Dichlormethan versetzt, 30 min gerührt und filtriert. Das Filtrat wird über Natriumsulfat getrocknet und eingedampft.
Ausbeute: 300 mg, gelber Feststoff. Das Produkt wird ohne weitere Aufreinigung weiter umgesetzt; HPLC: 1.68 min (Methode A); LC-MS: 285 (M+H).

### Herstellung von 4-[2-(3-Hydroxymethyl-phenyl)-pyrimidin-5-yl]-piperazin-1-carbonsäure-tert.-butylester

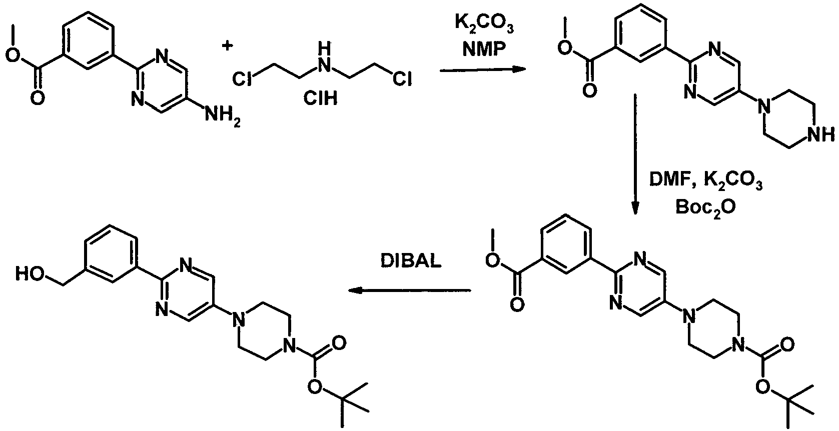

### Stufe 1:

3.2 g (13.95 mmol) 3-(5-Amino-pyrimidin-2-yl)-benzoesäuremethylester werden in 80 ml NMP gelöst, mit 4.73 g (25.96 mmol) Bis(2-chlorethyl)-ammoniumchlorid und 3.13 g (23.73 mmol) Kaliumcarbonat versetzt. Die Suspension wird unter Argonatmosphäre 7 Tage bei 130°C gerührt. Das Reaktionsgemisch wird filtriert, das Filtrat wird in 1 I Diethylether eingerührt. Dabei ölt ein Rückstand aus. Die organische Phase wird abgetrennt und verworfen. Der Rückstand wird mit 500 ml Ethylacetat und 200 ml gesättigter Natriumhydrogencarbonatlösung versetzt, die organische Phase abgetrennt und die wässrige Phase nochmals mit 500 ml Ethylacetat extrahiert. Die organischen Phasen werden vereinigt, über Natriumsulfat getrocknet und eingedampft. Der Rückstand wird ohne weitere Aufarbeitung weiter umgesetzt.
Ausbeute: 2.4 g; HPLC: Rt. = 2.07 min (Methode A); LC-MS: 299 (M+H).

### Stufe 2:

2.4 g (5.4 mmol) 3-(5-Piperazin-1-yl-pyrimidin-2-yl)-benzoesäuremethylester wird in 15 ml DMF gelöst, mit 2.98 g (21.6 mmol) Kaliumcarbonat und 1.5 ml (7.0 mmol) Di-tert.-butyldicarbonat versetzt und 30 min bei Raumtemperatur gerührt. Das Reaktionsgemisch wird filtriert und das Filtrat eingeengt. Der Rückstand wird in 200 ml Ethylacetat und 50 ml gesättigter Natriumhydrogencarbonatlösung aufgenommen. Die organische Phase wird abgetrennt und mit 50 ml 1 N HCl gewaschen, über Natriumsulfat getrocknet und eingeengt. Das Produkt wird ohne weitere Aufreinigung weiter umgesetzt.
Ausbeute: 1.1 g; HPLC: 3.18 min (Methode A); LC-MS: 399 (M+H).

### Stufe 3:

862 mg (2.16 mmol) 4-[2-(3-Methoxycarbonyl-phenyl)-pyrimidin-5-yl]-piperazin-1-carbonsäure-tert.-butylester werden in 15 ml THF gelöst und bei Raumtemperatur mit 10.8 ml (10.8 mmol) 1 M Diisobutylaluminiumhydrid in THF versetzt. Das Reaktionsgemisch wird 1 h bei Raumtemperatur gerührt. Das Reaktionsgemisch wird unter Eiskühlung mit 3 ml gesättigter Natriumsulfatlösung versetzt. Das gelartige Gemisch wird mit 30 ml Dichlormethan und 5 ml Methanol versetzt, 10 min gerührt und über Kieselgur abgesaugt. Das Filtrat wird über Natriumsulfat getrocknet und eingedampft. Der Rückstand wird in Dichlormethan gelöst, filtriert und das Filtrat eingedampft. Das Produkt wird ohne weitere Aufreinigung weiter umgesetzt; Ausbeute: 677 mg; HPLC: 2.66 min (Methode A); LC-MS: 371 (M+H).

### Herstellung von (3-{5-[1-(2-Morpholin-4-yl-ethyl)-1H-pyrazol-4-yl]-pyrimidin-2-yl}-phenyl)-methanol

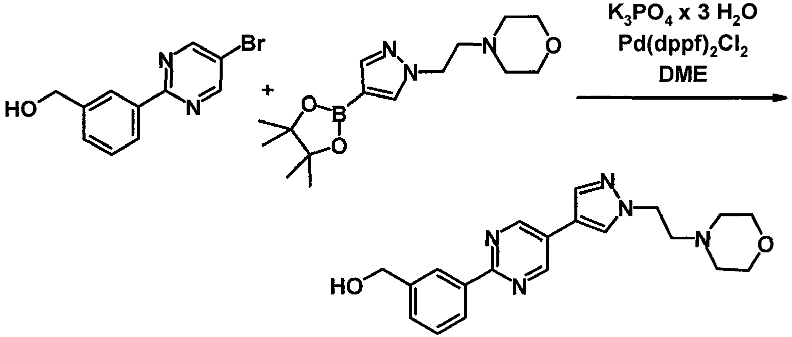

Unter Argonatmosphäre werden 2.82 g (10 mmol) [3-(5-Brom-pyrimidin-2-yl)-phenyl]-methanol in 100 ml Ethylenglycoldimethylether gelöst, mit 3.38 g (10 mmol) 4-{2-[4-(4,4,5,5-Tetramethyl-[1,3,2]dioxaborolan-2-yl)-pyrazol-1-yl]-ethyl}-morpholin und 4.25 g (20 mmol) Trikaliumphosphat Trihydrat versetzt. Das Reaktionsgemisch wird zweimal evakuiert und mit Argon gespült. 840 mg (1.2 mmol) Bis(triphenylphosphin)-palladium(II)-chlorid werden zugegeben, nochmals evakuiert und Argon gespült. Das Reaktionsgemisch wird 16 Stunden bei 80°C gerührt. Das Reaktionsgemsich wird mit Dichlormethan und Wasser verdünnt und über Celite filtriert. Die organische Phase wird abgetrennt, erneut mit Wasser gewaschen, die organische Phase über Natriumsulfat getrocknet und zum Rückstand eingeengt. Der Rückstand wird aus Isopropanol umkristallisiert; Ausbeute: 2.74 g, LCMS: 366 (M+H).

Analog können folgende Verbindungen hergestellt werden. In einigen Fällen werden die Rohprodukte mittels Säulenchromatographie an Kieselgel aufgereinigt.

| Verbindung Nr. | Name und/oder Struktur | LCMS [M+H] | Rt. in min |
|---|---|---|---|
| | 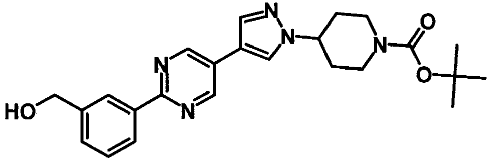 | 436 | |
| | 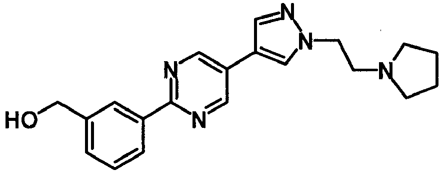 | 350 | |

### Herstellung der Benzylamine aus den Benzylalkoholen

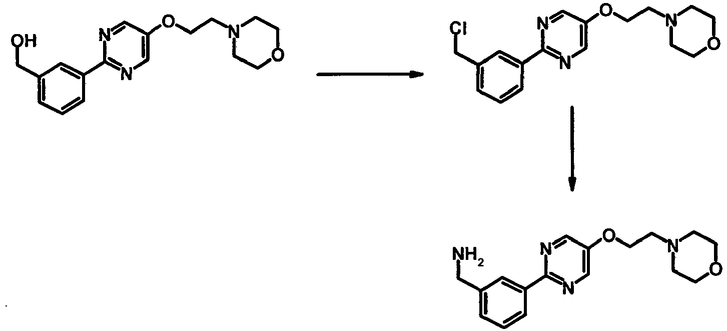

### Stufe 1:

3.66 g (11.6 mmol) {3-[5-(2-Morpholin-4-yl-ethoxy)-pyrimidin-2-yl]-phenyl}-methanol werden mit 16.5 ml (227 mmol) Thionylchlorid versetzt und 30 min bei Raumtemperatur gerührt. Das Reaktionsgemisch wird mit Diethylether versetzt, wobei sich ein Niederschlag bildet. Der Überstand wird abdekantiert, der Rückstand mit 50 ml Acetonitril verrührt und die gebildeten Kristalle werden abgesaugt, mit Acetonitril und Diethylether gewaschen und getrocknet.
Ausbeute: 4.0 g; hellbeigefarbene Kristalle; Rt. 2.24 min (Methode A); LCMS: 334 (M+H).

### Stufe 2:

587 mg (2.70 mmol) Di-*tert*.-butyliminodicarboxylat werden in 10 ml Ethylmethylketon gelöst, mit 2.64 g (8.10 mmol) Cäsiumcarbonat versetzt und 90 min gerührt. Anschließend werden 1.0 g (2.70 mmol) 4-{2-[2-(3-Chlormethyl-phenyl)-pyrimidin-5-yloxy]-ethyl}-morpholin und 29 mg (0.22 mmol) Lithiumiodid zugegeben. Das Reaktionsgemsich wird 16 h bei Raumtemperatur und 6 h bei 70°C gerührt. Das Reaktionsgemisch wird filtriert und der Rückstand mit Ethylacetat gewaschen, das Filtrat wird eingedampft und in Ethylacetat gelöst. Die organische Phase wird mit gesättigter Natriumhydrogencarbonatlösung und Wasser gewaschen, über Natriumsulfat getrocknet und eingeengt. Das Rohprodukt wird in 5 ml Dioxan und 5 ml 4N HCl in Dioxan versetzt und bei Raumtemperatur gerührt. Es bildet sich ein Niederschlag, die organsiche Phase wird abdekantiert und der Rückstand in Wasser gelöst. Die wässrige Phase wird mit Ethylacetat gewaschen, mit 32%iger Natronlauge auf pH 12 gebracht und zweimal mit Ethylacetat extrahiert. Die vereinigten organischen Phasen werden über Natriumsulfat getrocknet und eingeengt.
Ausbeute: 510 mg; Rt. = 1.52 min; LCMS 315 (M+H).

### Alternative Syntheseroute:

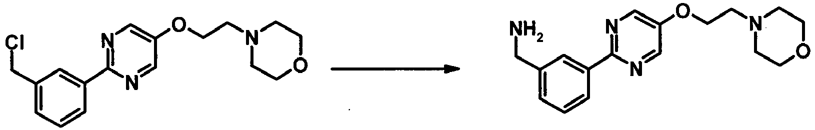

5.20 g (14.0 mmol) 4-{2-[2-(3-Chlormethyl-phenyl)-pyrimidin-5-yloxy]-ethyl}-morpholin werden in 36 ml 25%iger Ammoniaklösung und 36 ml *n-*Butanol gelöst. Das Reaktionsgemisch wird bei 120°C für 20 min in der Mikrowelle bestrahlt. Die organische Phase wird abgetrennt und die wässrige Phase nochmals mit Butanol extrahiert. Die vereinigten organischen Phasen werden über Natriumsulfat getrocknet und unter vermindertem Druck wird das Butanol abdestilliert und anschließend im Hochvakuum getrocknet. Das Produkt wurde ohne weitere Reinigung weiter umgesetzt; Ausbeute: 2.26 g.

### Alternative Synthese zur Herstellung von Benzylaminen aus Benzylalkoholen

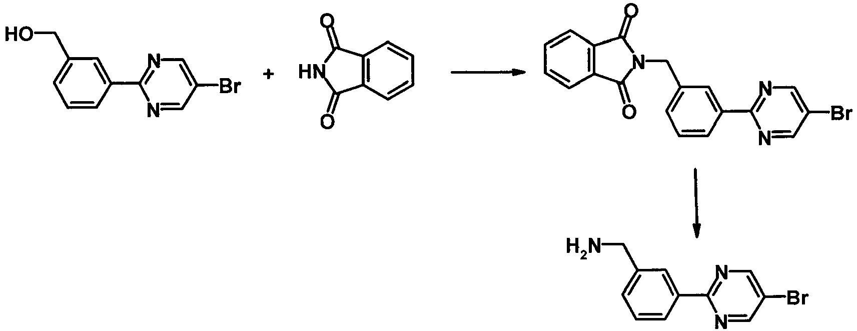

### Stufe 1:

5.0 g (18.9 mmol) [3-(5-Brom-pyrimidin-2-yl)-phenyl]-methanol und 3.05 g (20.7 mmol) Phtalimid werden in 150 ml THF gelöst, mit 6.9 g (20.7 mmol) polymergebundenem Triphenylphosphin (3 mol/g) versetzt und 15 min bei Raumtemperatur geschüttelt. Anschließend wird 4.78 g (20.7 mmol) Di-*tert*.-butylazodicarboxylat zugegeben und unter Stickstoffatmosphäre 18 h bei Raumtemperatur geschüttelt. Das Reaktionsgemsisch wird filtriert, der Rückstand intensiv mit DMF und DMF/Methanol gewaschen und das Filtrat eingeengt. Der Rückstand wird in Ethylacetat aufgenommen und mit Wasser gewaschen, getrocknet und eingeengt. Der Rückstand wird mittels Säulenchromatographie an Kieselgel aufgereinigt; HPLC: Rt. = 3.41 min (Methode A), LCMS: 394/396 (M+H).

### Stufe 2:

Das Produkt von Stufe 1 wird in 60 ml Ethanol versetzt und mit 5 equiv. Hydrazin Hydrat versetzt. Das Reaktionsgemisch wird 18 h bei 70°C gerührt, eingeengt und in Ethylacetat und gesättigter Natriumhydrogencarbonatlösung aufgenommen. Die organische Phase wird abgetrennt, getrocknet und mittels Säulenchromatographie an Kieselgel aufgereinigt; HPLC: Rt. = 2.11 min (Methode A), LCMS: 264/266 (M+H).

**Analog den beschrieben Vorschriften werden folgende Benzylamine hergestellt:**

| Verbindung Nr. | Name und/oder Struktur | LCMS [M+H] | Rt. in min |
|---|---|---|---|
| | 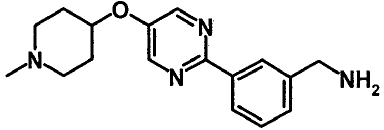 | 299 | |
| | 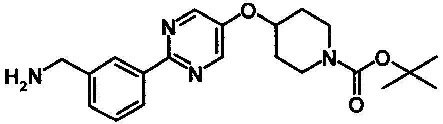 | 385 | |
| | 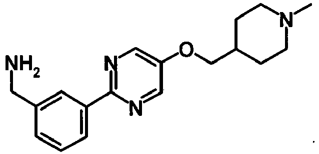 | 313 | |
| | 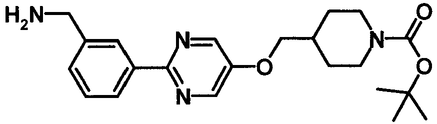 | 399 | |
| | 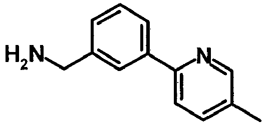 | 199 | |
| | 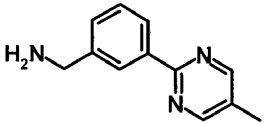 | 200 | |
| | 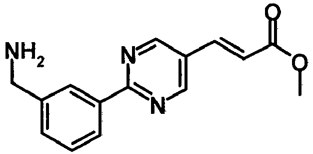 | 270 | |
| | 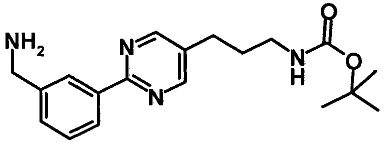 | 343 | |
| | 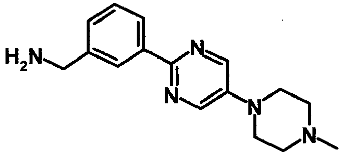 | 284 | |
| | 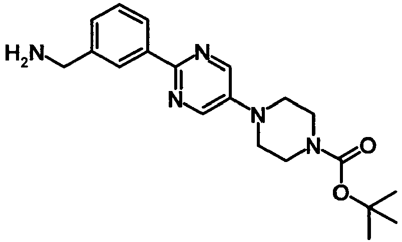 | 370 | |
| | 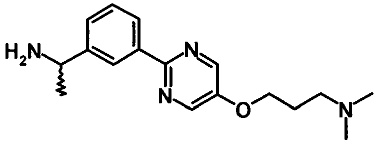 | 301 | |
| | 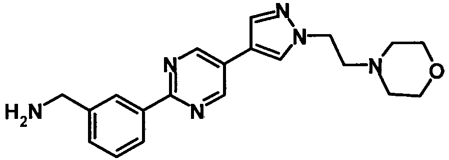 | 365 | |
| | 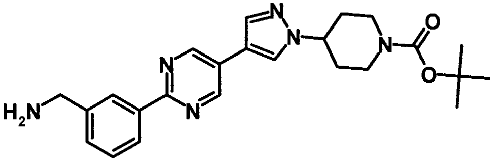 | 435 | |
| | 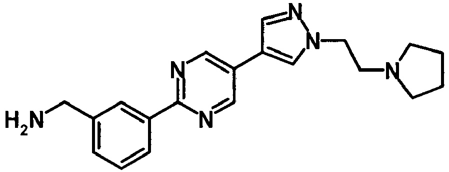 | 349 | |

### Herstellung von 6-(1-Methyl-1H-pyrazol-4-yl)-1-{3-[5-(2-morpholin-4-yl-ethoxy)-pyrimidin-2-yl}-benzyl}-1H-[1,2,3]triazolo[4,5-b]pyrazin ("A2")

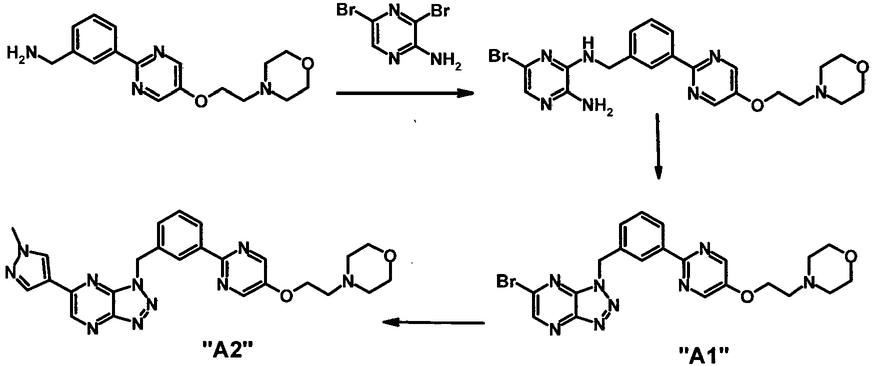

### Stufe 1:

400 mg (1.27 mmol) 3-(5-(2-Morpholin-4-yl-ethoxy)-pyrimidin-2-yl]-benzylamin werden mit 800 µl Diisopropylethylamin versetzt. Zu dieser Suspension werden 319 mg (1.26 mmol) 2-Amino-3,5-dibrompyrazin hinzugefügt und das Reaktionsgemisch 5 Stunden bei 130°C gerührt. Die braune Reaktionslösung wird mit Dichlormethan und Wasser versetzt, die organische Phase wird über Natriumsulfat getrocknet und eingeengt. Das Rohprodukt wird mittels Säulenchromatographie an Kieselgel gereinigt.
Ausbeute: 396 mg braunes Öl; Rt. = 2,20 min (Methode A), LC-MS: 487 (M+H).

### Stufe 2:

396 mg (0.81 mmol) 5-Bromo-N*3*-{3-[5-(2-morpholin-4-yl-ethoxy)-pyrimidin-2-yl]-benzyl}-pyrazin-2,3-diamin werden in 7 ml Wasser/Essigsäure 1:1 gelöst. Zu dieser orangen Lösung wird eine Lösung aus 562 mg (8.14 mmol) Natriumnitrit in 3.5 ml Wasser langsam zugetropft. Die Temperatur steigt dabei von 22°C auf 26°C an. Es wird 1 Stunde bei Raumtemperatur gerührt. Anschließend wird 4 Stunden bei 65°C Innentemperatur gerührt.

Das Reaktionsgemisch wird eingeengt, der Rückstand in Wasser gelöst und mit festem Natriumhydrogencarbonat neutralisiert. Dabei fällt ein braunes Öl aus. Es wird mit einer Mischung aus Ethylacetat und wenig Methanol extrahiert. Die organische Phase wird getrocknet und eingeengt.

Ein Teil des Rohproduktes wird mittels präparativer HPLC aufgereinigt, der Rest ohne weitere Aufreinigung weiter umgesetzt. Man erhält 6-Brom-1-{3-[5-(2-morpholin-4-yl-ethoxy)-pyrimidin-2-yl]-benzyl}-1H-[1,2,3]triazolo[4,5-b]pyrazin ("A1"); Produkt liegt als TFA-Salz vor;
¹H-NMR (500 MHz, DMSO-d₆) δ [ppm] 10.05 (b, 1H), 9.00 (s, 1H), 8.70 (s, 2H), 8.38 (s, 1 H), 8.28 (m, 1 H), 7.48-7.54 (m, 2H), 6.08 (s, 2H), 4.58 (b, 2H), 3.98 (b, 2H), 3.1-3.8 (b, 8H).

### Stufe 3:

Unter Argonatmosphäre werden 225 mg (0.24 mmol) 6-Brom-1-{3-[5-(2-morpholin-4-yl-ethoxy)-pyrimidin-2-yl]-benzyl)-1H-[1,2,3]triazolo[4,5-b]pyrazin in 5 ml Ethylenglycoldimethylether gelöst und mit 102 mg (0.48 mmol) Trikaliumphosphat Trihydrat und 55 mg (0.26 mmol) 1-Methyl-4-(4,4,5,5-tetramethyl-1,3,2-dioxaborolan-2-yl)-1 H-pyrazol versetzt. Das Reaktionsgemisch wird zweimal evakuiert und mit Argon gespült. 14 mg (0.02 mmol) Bis(triphenylphosphin)-palladium(II)-chlorid werden zugegeben, nochmals evakuiert und Argon gespült. Das Reaktionsgemisch wird 16 Stunden bei 80°C gerührt. Das Reaktionsgemsich wird mit 10 ml Wasser versetzt, wobei ein Öl ausfällt. Es wird mit Dichlormethan und mit Dichlormethan mit ca. 10% MeOH extrahiert, die wässrige Phase mit 32%iger NaOH auf pH 14 gebracht und erneut mit Dichlormethan extrahiert. Die vereinigten organischen Phasen werden über Natriumsulfat getrocknet und mittels präparativer HPLC aufgereingt. Man erhält 42 mg 6-(1-Methyl-1H-pyrazol-4-yl)-1-{3-[5-(2-morpholin-4-yl-ethoxy)-pyrimidin-2-yl]-benzyl}-1H-[1,2,3]triazolo[4,5-b]pyrazin ("A2"); Produkt liegt als TFA-Salz vor; Rt. = 2.26 (Methode A), LCMS: 499 (M+H);
¹H-NMR (500 MHz, DMSO-d₆) δ [ppm] 10.01 (b, 1H), 9.20 (s, 1H), 8.69 (s, 2H), 8.64 (s, 1 H), 8.44 (s, 1 H), 8.31 (s, 1H), 8.27 (d, 1 H), 7.59 (d, 1 H), 7.52 (t, 1H), 6.04 (s, 2H), 4.57 (b, 2H), 3.98 (b, 2H), 3.95 (s, 3H), 3.1-3.8 (b, 8H).

### Herstellung von 6-Brom-1-[3-(5-brom-pyrimidin-2-yl)-benzyl]-1H-[1,2,3]triazolo[4,5-b]pyrazin "B1")

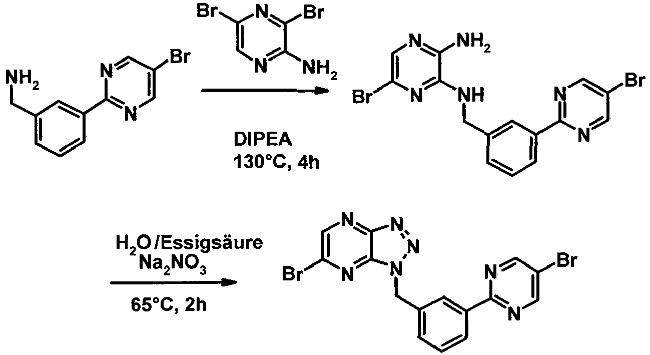

### Stufe 1:

17.2 g (55.3 mmol) 3-(5-Brom-pyrimidin-2-yl)-benzylamin und 14.3 g (55.3 mmol) 2-Amino-3,5-dibrompyrazin werden mit 50 ml (294 mmol) N-Ethyl-*N,N*-diisopropylamin versetzt.. Das Reaktionsgemisch wird 4 h bei 130 °C gerührt. Die Lösung wird filtriert, der Rückstand wird in Ethylacetat gelöst und mit Wasser zweimal gewaschen. Die organische Phase wird über Natriumsulfat getrocknet und eingedampft.
Ausbeute: 24.85 g, HPLC: Rt = 3.14 min (Methode B), LC-MS: [M+H]⁺ = 437.

### Stufe 2:

23.9 g (43.7 mmol) 5-Brom-N'3'-[3-(5-brom-pyrimidin-2-yl)-benzyl]-pyrazin-2,3-diamin werden in 240 ml Wasser und 240 ml Essigsäure (96%) gelöst und mit 30.1g (437 mmol) Natriumnitrit gelöst in 240 ml Wasser versetzt. Es wird 1 h bei Raumtemperatur und 4 h bei 65°C gerührt. Das Reaktionsgemisch wird abgekühlt und der Rückstand abgesaugt. Der Rückstand mit Ether verrührt und ohne Aufreinigung weiter umgesetzt. Ausbeute: 15.5 g, HPLC: Rt = 3.28 min (Methode C), LC-MS: [M+H]⁺ = 448.

### Herstellung von 1-[3-(5-Brom-pyrimidin-2-yl)-benzyl]-6-(1-methyl-1H-pyrazol-4-yl)-1H-[1,2,3]triazolo[4,5-b]pyrazin ("B2")

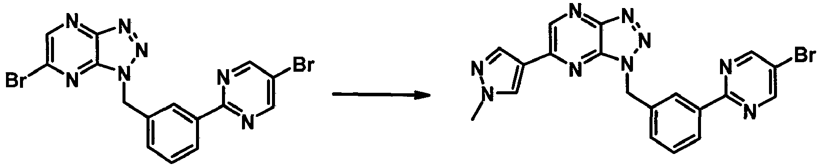

2.00 g (3.67 mmol) 6-Brom-1-[3-(5-brom-pyrimidin-2-yl)-benzyl]-1 H-[1,2,3]triazolo[4,5-b]pyrazin, 840 mg (4.04 mmol)1-Methyl-4-(4,4,5,5-tetramethyl-[1,3,2]dioxaborolan-2-yl)-1H-pyrazol und 778 mg (7.34 mmol) Natriumcarbonat werden in 3.7 ml (204 mmol) Wasser und 15 ml *N,N-*Dimethylformamid suspendiert, mehrfach entgast, evakuiert und mit Stickstoff gespült. 257 mg (0.367 mmol) Bis(triphenylphosphin)-palladium(II)-chlorid werden hinzugegeben und nochmals evakuiert und mit Stickstoff gespült. Das Reaktionsgemisch wird 24 h bei 80°C gerührt. Das Reaktionsgemisch wird filtriert und mit Ethylacetat gewaschen und eingedampft. Der Rückstand wird mit Isopropanol verrührt und ohne zusätzliche Aufreinigung weiter umgesetzt.
HPLC: Rt = 2.96 min (Methode A), LC-MS: [M+H]⁺ = 448/450, Rₜ = 2.36 min (Methode C);
¹H-NMR (500 MHz, DMSO-d₆) δ [ppm] 9.19 (s, 1 H), 9.07 (s, 2H), 8.62 (s, 1 H), 8.52 (s, 1 H), 8.33 (s, 1 H), 8.32 (d, J=5.9, 1 H), 7.64 (d, J=7.7, 1 H), 7.55 (t, J=7.7, 1 H), 6.06 (s, 2H), 3.95 (s, 3H).

### Analog werden folgende Verbindungen hergestellt

### 3-{3-[3-(5-Brom-pyrimidin-2-yl)-benzyl]-3H-[1,2,3]triazolo[4,5-b]pyrazin-5-yl}-benzonitril ("B3")

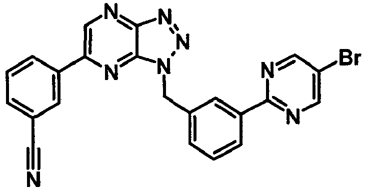

HPLC: Rt = 3.34 min (Methode A), LC-MS: [M+H]⁺ = 469/471; ¹H-NMR (500 MHz, DMSO-d₆) δ [ppm] 9.07 (s, 2H), 8.73 (s, 1 H), 8.37 (s, 1 H), 8.33 (m, 1 H), 7.89 (s, 1 H), 7.76 (m, 1 H), 7.72-7.64 (m, 2H), 7.51 (m, 1 H), 7.47 (m, 1 H), 2.54 (s, 2H).

### 1-[3-(5-Brom-pyrimidin-2-yl)-benzyl]-6-(3,5-difluor-phenyl)-1H-[1,2,3]triazolo[4,5-b]pyrazin ("B4")

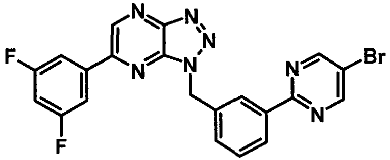

HPLC: Rt = 3.56 min (Methode A), LC-MS: [M+H]⁺ =480/482.

### Herstellung von 1-[3-(5-Brom-pyrimidin-2-yl)-benzyl]-6-(1-methyl-1H-pyrazol-4-yloxy)-1H-[1,2,3]triazolo[4,5-b]pyrazin ("B5")

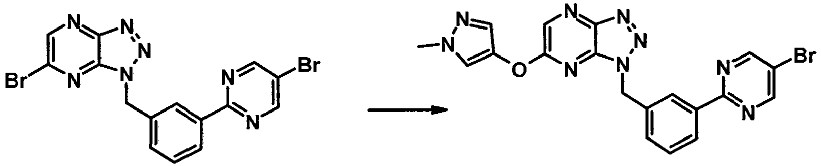

500 mg (0.932 mmol) 6-Brom-1-[3-(5-brom-pyrimidin-2-yl)-benzyl]-1 H-[1,2,3]triazolo[4,5-b]pyrazin, 213 mg (1.03 mmol) 1-Methyl-4-(4,4,5,5-tetramethyl-[1,3,2]dioxaborolan-2-yl)-1H-pyrazol und 396 mg (1.86 mmol) tri-Kaliumphosphat Trihydrat werden in 20 ml Ethylenglycoldimethylether suspendiert, mehrfach entgast, evakuiert und mit Stickstoff gespült. 65.4 mg (0.093 mmol) Bis(triphenylphosphin)-palladium(II)-chlorid (15.2% Pd) werden hinzugegeben und nochmals evakuiert und mit Stickstoff gespült. Das Reaktionsgemisch wird 24 h bei 80°C gerührt. Das Reaktionsgemisch wird filtriert, das Filtrat wird mit Wasser versetzt und mit Ethylacetat extrahiert. Die organische Phase wird über Natriumsulfat getrocknet, unter vermindertem Druck am Rotationsverdampfer eingeengt und säulenchromatisch an Kieselgel aufgereinigt.
Ausbeute: 38 mg, HPLC: Rt = 2.96 min (Methode C), LC-MS: [M+H]⁺ = 464/466;
¹H-NMR (500 MHz, DMSO-d₆) δ [ppm] 9.06 (s, 2H), 8.65 (s, 1H), 8.43 (s, 1 H), 8.32 (d, J=7.4, 1 H), 8.11 (s, 1 H), 7.67 (s, 1 H), 7.58 (d, J=7.6, 1 H), 7.54 (t, J=7.6, 1 H), 6.01 (s, 2H), 3.85 (s, 3H).

### Analog werden folgende Verbindungen hergestellt:

### 3-{3-[3-(5-Brom-pyrimidin-2-yl)-benzyl]-3H-[1,2,3]triazolo[4,5-b]pyrazin-5-yloxy}-benzonitril ("B6")

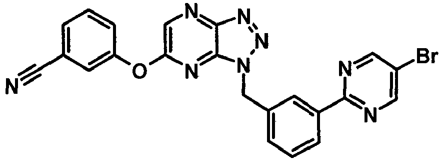

Das Rohprodukt wird mittels präparativer HPLC aufgereinigt. HPLC: Rₜ = 3.25 min (Methode A), LC-MS: [M+H]⁺ = 485/487;
¹H-NMR (500 MHz, DMSO-d₆) δ [ppm] 9.06 (s, 2H), 8.73 (s, 1H), 8.33 (s, 1H), 8.29 (d, J=7.8, 1H), 7.89 (s, 1H), 7.75 (dd, J=1.5, 7.2, 1H), 7.68 (m, 1H), 7.51- 7.41 (m, 3H), 5.82 (s, 2H).

### 1-[3-(5-Brom-pyrimidin-2-yl-benzyl]-6-(3,5-difluor-phenoxy)-1H-[1,2,31triazolo[4,5-b]pyrazin ("B7")

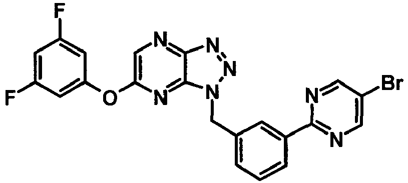

HPLC: Rt = 3.52 min (Methode A), LC-MS: [M+H]⁺ = 495/497;
¹H-NMR (500 MHz, DMSO-d₆) δ [ppm] 9.06 (s, 2H), 8.72 (s, 1H), 8.36 (s, 1 H), 8.32-8.28 (m, 1 H), 7.50-7.44 (m, 2H), 7.20 (m, 2H), 7.18 (m, 1H), 5.87 (s, 2H).

### Herstellung von 6-(1-Methyl-1H-pvrazol-4-yl)-1-(3-{5-[1-(2-pyrrolidin-1-yl-ethyl)-1H-pyrazol-4-yl-pyrimidin-2-yl)-benzyl)-1H-[1,2,3]triazolo[4,5-b]pyrazin Hydrochlorid ("A24")

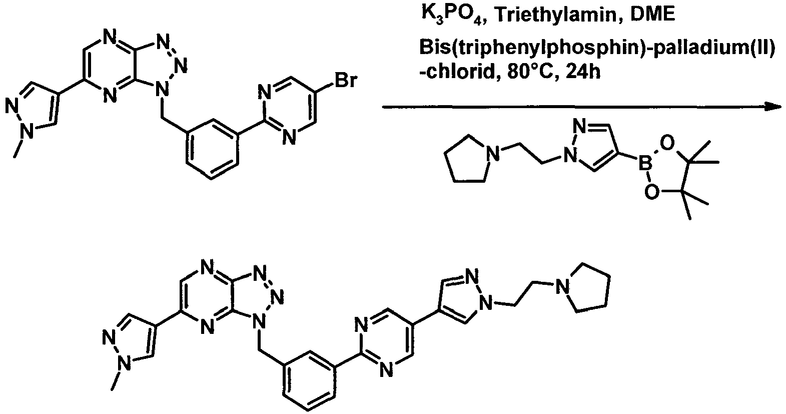

50 mg (0.104 mmol) 1-[3-(5-Brom-pyrimidin-2-yl)-benzyl]-6-(1-methyl-1H-pyrazol-4-yl)-1H-[1,2,3]triazolo[4,5-b]pyrazin, 51.5 mg 1-(2-Pyrrolidin-1-yl-ethyl)-4-(4,4,5,5-tetramethyl-[1,2,3]dioxaborolan-2-yl)-1H-pyrazol und 44.0 mg (0.207 mmol) tri-Kaliumphosphat Trihydrat werden in 2 ml Ethylenglycoldimethylether suspendiert, mehrfach entgast, evakuiert und mit Stickstoff gespült. 7.3 mg (0.010 mmol) Bis(triphenylphosphin)-palladium(II)-chlorid (15.2% Pd) un 1.5 µl Triethylamin werden hinzugegeben und nochmals evakuiert und mit Stickstoff gespült. Das Reaktionsgemisch wird 24 h bei 80°C gerührt. Das Reaktionsgemisch wird abgekühlt und mit Ethylacetat und Wasser versetzt. Die organische Phase wird abgetrennt, die wässrige Phase wird mit Ethylacetat extrahiert. Anschließend werden die vereinigten organischen Phasen über Natriumsulfat getrocknet und das Lösungsmittel wird unter verminderten Druck abdestilliert. Die wässrige Phase wird nochmals mit Dichlormethan extrahiert, über Natriumsulfat getrocknet und unter vermindertem Druck eingedampft. Beide Rückstände werden zusammen mittels präparativer HPLC aufgereinigt. Der Rückstand wird in Methanol gelöst, mit methanolischer HCl versetzt und an der Genevac eingedampft. Das Produkt liegt als Hydrochlorid vor.
HPLC: Rt = 2.36 min (Methode A), LC-MS: [M+H]⁺ = 533;
¹H-NMR (500 MHz, DMSO-d₆) δ [ppm] 10.13 (s, 1H), 9.20 (s, 1H), 9.16 (s, 2H), 8.64 (s, 1H), 8.52 (s, 1H), 8.50 (s, 1H), 8.35 (d, J=7.8, 1H), 8.31 (s, 1 H), 8.23 (s, 1H), 7.61 (d, J=7.8, 1H), 7.55 (t, J=7.7, 1H), 6.06 (s, 2H), 4.60 (t, J=6.2, 2H), 3.95 (s, 3H), 3.69 (t, J=6.1, 2H), 3.52- 1.19 (m, 8H).

### Herstellung von 2-{3-[6-(1-Methyl-1H-pyrazol-4-yl)-[1,2,3]triazolo[4,5-b]pyrazin-1-ylmethyll-phenyl}-pyrimidin-5-ol ("B8")

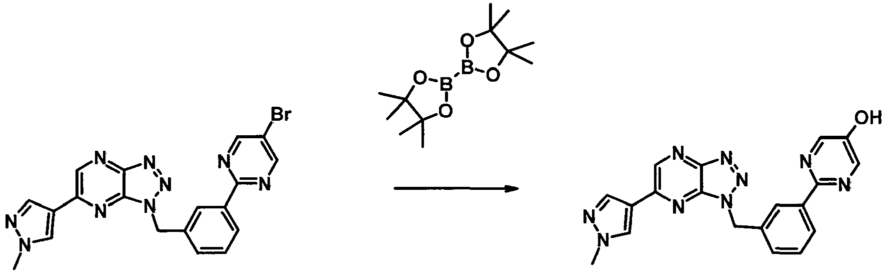

800 mg (1.66 mmol) 1-[3-(5-Brom-pyrimidin-2-yl)-benzyl]-6-(1-methyl-1H-pyrazol-4-yl)-1 H-[1,2,3]triazolo[4,5-b]pyrazin wird in 10ml THF und 1 ml DMF suspendiert, und mit 515 mg (1.99 mmol) Bis(pinacolato)diboron und 488 mg (4.97 mmol) Kaliumacetat versetzt. Das Reaktionsgemisch wird mehrfach evakuiert und mit Argon gespült. 16.3 mg (0.023 mmol) Bis(triphenylphosphin)-palladium(II)-chlorid wird zugegeben und nochmals evakuiert und mit Argon gespült. Das Reaktionsgemisch wird 24 h bei 80°C gerührt. Nach vollständiger Umsetzung des Edukts wird das Reaktionsgemisch mit 255 mg (1.656 mmol) Natriumperborat Trihydrat und 2 ml Wasser versetzt und bei Raumtemperatur 24 h gerührt. Das Reaktionsgemisch wird abgesaugt und mit Ethylacetat gewaschen. Anschließend wird das Filtrat mit NaOH-Lösung auf pH 12 gebracht und mit Ethylacetat extrahiert. Die wässrige Phase wird mit Salzsäure neutralisiert und mit Ethylacetat zweimal extrahiert. Danach werden die vereinigten organischen Phasen über Natriumsulfat getrocknet und unter vermindertem Druck eingedampft.
Ausbeute: 320 mg, HPLC: Rt = 2.42 min, LC-MS: [M+H]⁺ = 385.

### Analog werden hergestellt:

### 2-{3-[3-(3-Hydroxyl-pyrimidin-2-yl)-benzyl]-3H-[1,2,3]triazolo[4,5-b]pyrazin-5-yl}-benzonitril ("B9")

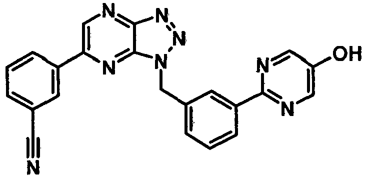

HPLC: Rₜ = 2.81 min (Methode A), LC-MS: [M+H]⁺ = 407;
¹H-NMR (500 MHz, DMSO-d₆) δ [ppm] 10.95 s, 1H), 9.59 (s, 1H), 8.81 (s, 1 H), 8.68 (d, J=8.1, 1 H), 8.54 (s, 1 H), 8.44 (s, 2H), 8.24 (d, J=7.8, 1H), 8.09 (d, J=7.7, 1H), 7.84 (t, J=7.9, 1H), 7.58 (d, J=7.7, 1H), 7.49 (t, J=7.7, 1 H), 6.17 (s, 2H).

### 2-{3-[6-(3,5-Difluor-phenyl)-[1,2,3]triazolo[4,5-b]pyrazin-1-ylmethyl]-phenyl}-pyrimidin-5-ol ("B10")

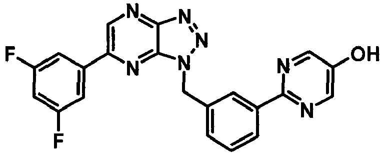

HPLC: Rt = 2.97 min (Methode A), LC-MS: [M+H]⁺ = 418;
¹HNMR (500 MHz, DMSO-d₆) δ [ppm] 10.59 (s, 1H), 9.55 (s, 1H), 8.60 (s, 1 H), 8.43 (s, 2H), 8.23 (d, J=7.8, 1 H), 8.11 (d, J=6.7, 2H), 7.58 (d, J=7.7, 1 H), 7.53 (d, J=9.1, 1 H), 7.49 (m, 1 H), 6.16 (s, 2H).

### 2-{3-[6-(1-Methyl-1H-pvrazol-4-vloxy)-[1,2,3]triazolo[4,5-b]pyrazin-1-ylmethyl]-phenyl}-pyrimidin-5-ol ("B11")

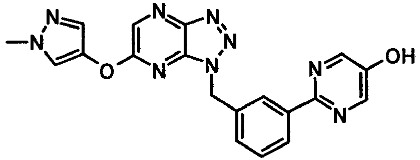

LC-MS: [M+H]⁺ = 402.

### 3-{3-[3-(5-Hydroxy-pyrimidin-2-yl)-benzyl]-3H-[1,2,3]triazolo[4,5-b]pyrazin-5-yloxy}-benzonitril ("B12")

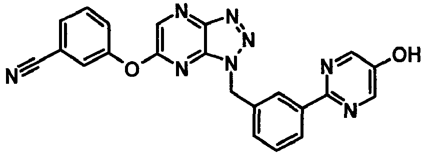

LC-MS: [M+H]⁺ = 423.

### 2-{3-[6-(3,5-Difluor-phenoxy)-[1,2,3]triazolo[4,5-b]pyrazin-1-ylmethyl]-phenyl}-pyrimidin-5-ol ("B13")

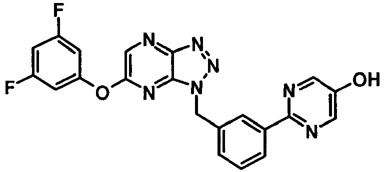

LC-MS: [M+H]⁺ = 434.

### Herstellung von Dimethyl-[2-(2-{3-[6-(1-methyl-1H-pyrazol-4-yl)-[1,2,3]triazolo[4,5-b]pyrazin-1-ylmethyl]-phenyl}-pyrimidin-5-yloxy)-ethyl]-amin Hydrochlorid ("B14")

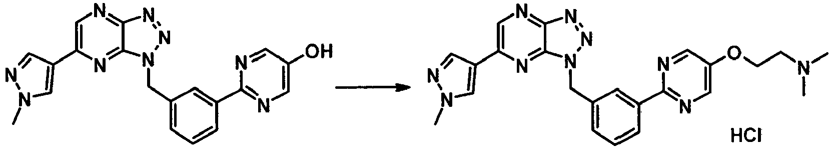

60.00 mg (0.156 mmol) 2-{3-[6-(1-Methyl-1H-pyrazol-4-yl)-[1,2,3]triazolo[4,5-b]pyrazin-1-ylmethyl]-phenyl}-pyrimidin-5-ol und 15.6 µl (0.156 mmol) 2-(Dimethylamino)-ethanol in 5 ml Tetrahydrofuran und 1 ml *N,N*-Dimethylformamid werden mit 77.9 mg (0.234 mmol) polymergebundenem Triphenylphosphin versetzt. Anschließend wird dieses Reaktionsgemisch evakuiert, mit Stickstoff gespült und 5 min geschüttelt. Das Reaktionsgemisch wird mit 53.8 mg (0.234 mmol) Di-*tert*.butylazodicarboxylat versetzt und nochmals evakuiert und mit Stickstoff gespült. Der Ansatz wird bei RT 4 h geschüttelt. Anschließend wurden nochmals 15.6 µl (0.156 mmol) 2-(Dimethylamino)-ethanol, 77.9 mg (0.234 mmol) polymergebundenes Triphenylphosphin und 53.8 mg (0.234 mmol) Di-*tert*.butylazodicarboxylat hinzugegeben und 24 h geschüttelt. Das Reaktionsgemisch wird über Celite abgesaugt und mit DMF gewaschen. Anschließend wird das Filtrat unter vermindertem Druck eingedampft und mittels präparative HPLC aufgereinigt. Der Rückstand wird in Methanol gelöst, mit methanolischer HCl versetzt und an der Genevac eingedampft. Das Produkt liegt als Hydrochlorid vor.
Ausbeute: 20 mg, HPLC: Rₜ = 2.20 min (Methode A), LC-MS: [M+H]⁺ = 457; ¹H-NMR (500 MHz, DMSO-d₆) δ [ppm] 10.31 (s, 1 H), 9.19 (s, 1 H), 8.69 (s, 2H), 8.64 (s, 1 H), 8.45 (s, 1 H), 8.31 (s, 1 H), 8.27 (d, J=7.8, 1 H), 7.57 (d, J=7.7, 1 H), 7.52 (t, J=7.7, 1 H), 6.04 (s, 2H), 4.60-4.56 (m, 2H), 3.95 (s, 3H), 3.56 (t, J=4.7, 2H), 2.86 (d, J=4.8, 6H).

**Analog werden folgende Verbindungen hergestellt:**

| Verbindung Nr. | Name und/oder Struktur | LCMS [M+H] | HPLC Rt. in min |
|---|---|---|---|
| "A4" | 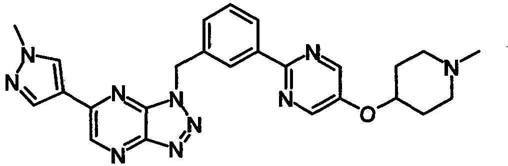 | 483 | |
| "A5" | 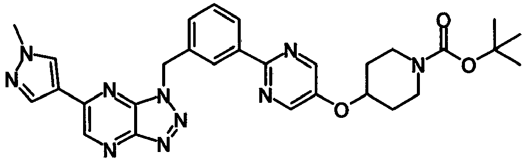 | 569 | |
| "A6" | 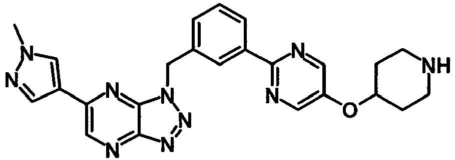 | 469 | 2.24 (Methode A) |
| | Hydrochlorid | | |
| ¹H-NMR (500 MHz, DMSO-d₆) δ [ppm] 9.19 (s, 1H), 8.74 (s, 1H), 8.69 (s, 2H), 8.63 (s, 1H), 8.42 (s, 1H), 8.31 (s, 1H), 8.26 (d, J=7.8, 1H), 7.57 (d, J=7.6, 1H), 7.51 (t, J=7.7, 1H), 6.04 (s, 2H), 4.86 (m, 1H), 3.95 (s, 3H), 3.25 (d, J=13.5, 2H), 3.08 (m, 2H), 2.15 (m, 2H), 1.92 (d, J=12.4, 2H) | | | |
| "A7" | 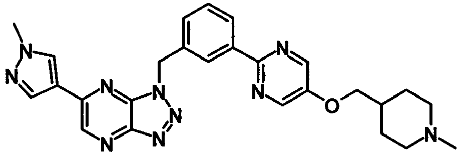 | 497 | |
| "A8" | 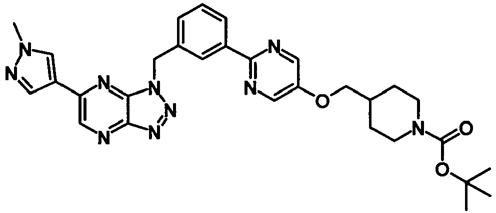 | 583 | |
| "A9" | 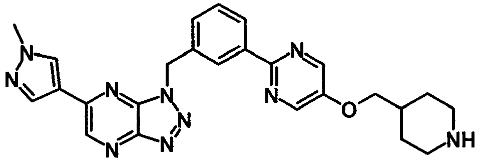 | 483 | 2.31 (Methode A) |
| | Hydrochlorid | | |
| ¹H-NMR (500 MHz, DMSO-d₆) δ [ppm] 9.19 (s, 1H), 8.86 (s, 1H), 8.64 (s, 2H), 8.55 (s, 1H), 8.44 (s, 1H), 8.31 (s, 1H), 8.25 (d, J=7.7, 1H), 7.56 (d, J=7.8, 1H), 7.51 (t, J=7.7, 1H), 6.04 (s, 2H), 5.74 (s, 1H), 4.09 (d, J=6.3, 2H), 3.95 (s, 3H), 3.27 (dd, J=11.1, 26.7, 2H), 2.89 (t, J=11.8, 2H), 2.12 (m, 1H), | | | |
| 1.92 (m, 2H), 1.51 (dd, J=12.0, 22.2, 2H) | | | |
| "A10" | 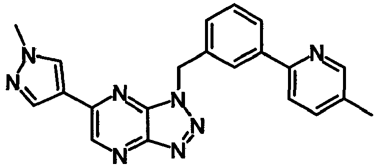 | 383 | |
| "A11" | 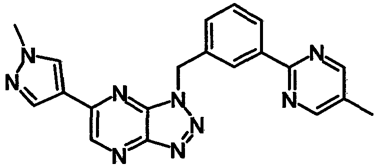 | 384 | |
| "A12" | 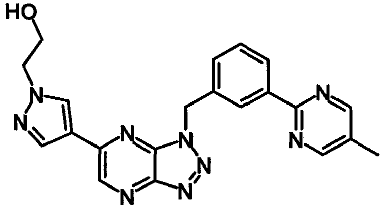 | 414 | |
| "A13" | 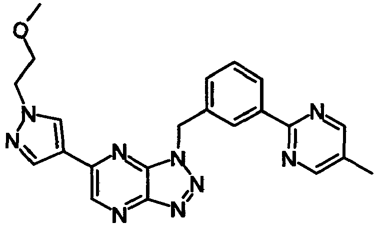 | 428 | |
| "A14" | 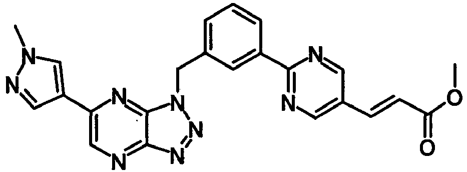 | 454 | |
| "A15" | 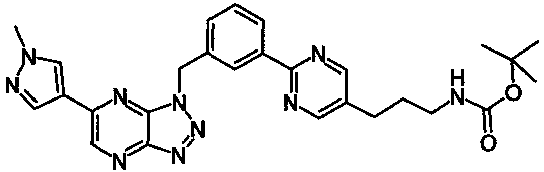 | 527 | |
| "A16" | 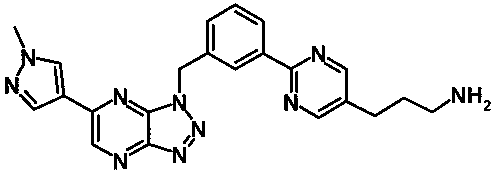 | 427 | |
| "A17" | 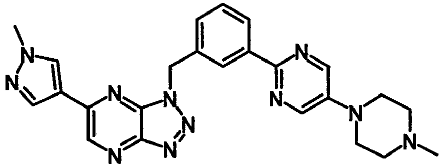 | 468 | |
| "A18" | 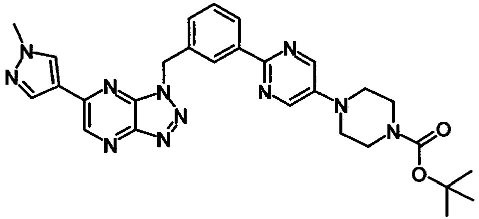 | 554 | |
| "A19" | 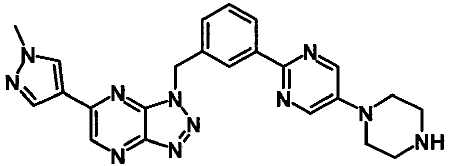 | 454 | |
| "A20" | 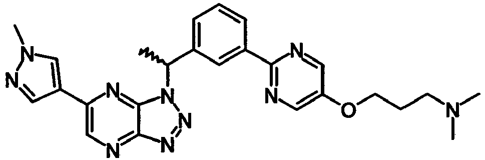 | 485 | |
| "A21" | 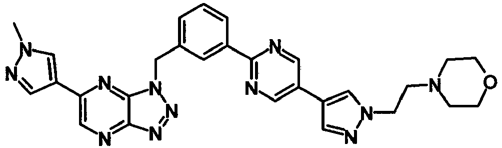 | 549 | |
| "A22" | 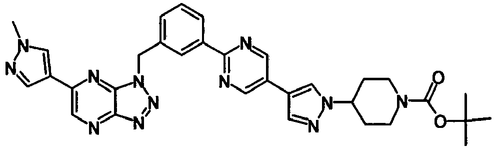 | 619 | |
| "A23" | 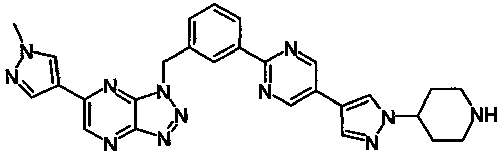 | 519 | |
| "A24" | 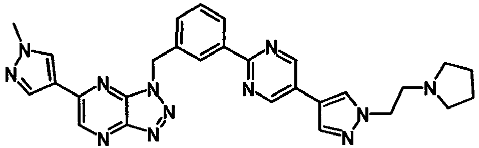 | 533 | |
| "A25" | 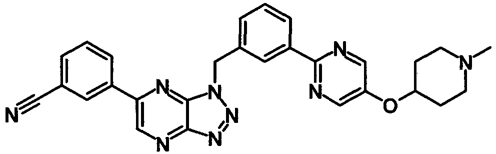 | 504 | |
| "A26" | 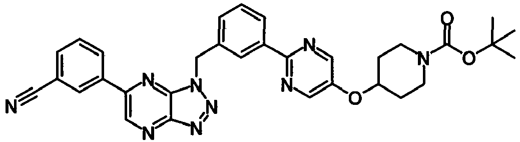 | 590 | |
| "A27" | 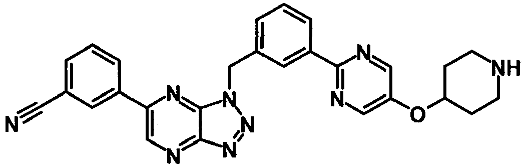 | 490 | 2.51 (Methode A) |
| | Hydrochlorid | | |
| ¹H-NMR (500 MHz, DMSO-d₆) δ [ppm] 9.58 (s, 1H), 8.78 (s, 1H), 8.76-8.72 (m, 1H), 8.70 (s, 2H), 8.66 (d, J=8.1, 1H), 8.53 (s, 1H), 8,27 (d, J=7.8, 1H), 8.06 (d, J=7.7, 1H), 7.84 (t, J=7.9, 1H), 7.62 (d, J=7.6, 1H), 7.53 (t, J=7.7, 1H), 6.17 (s, 2H), 4.86 (m, 1H), 3.98 (m, 1H), 3.26 (m, 2H), 3.07 (m, 2H), 2.15 (m, 2H). 1.91 (m, 2H) | | | |
| "A28" | 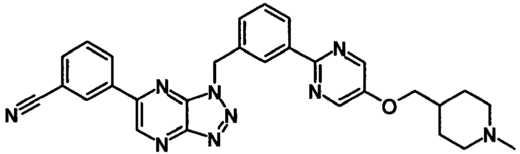 | 518 | |
| "A29" | 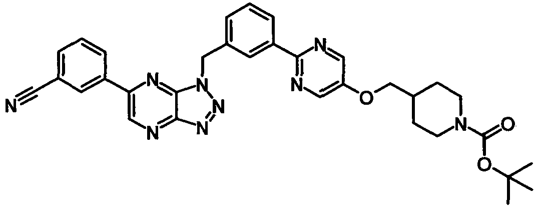 | 604 | |
| "A30" | 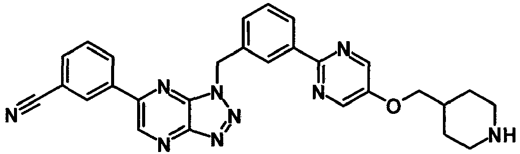 | 504 | 2.57 (Methode A) |
| | Hydrochlorid | | |
| ¹H-NMR (500 MHz, DMSO-d₆) δ [ppm] 9.58 (s, 1H), 8.78 (m, 2H), 8.66 (d, J=8.1, 1H), 8.65 (s, 2H), 8.54 (m, 1H), 8.48 (s, 1H), 8.26 (d, J=7.8, 1H), 8.07 (d, J=7.7, 1H), 7.84 (t, J=7.9, 1H), 7.60 (d, J=7.7, 1H), 7.51 (t, J=7.7, 1H), 6.17 (s, 2H), 4.12 (d, J=6.3, 2H), 3.26 (m, 2H), 2.68 (m, 2H), 1.89 (m, 2H), 1.57-1.18 (m, 2H) | | | |
| "A31" | 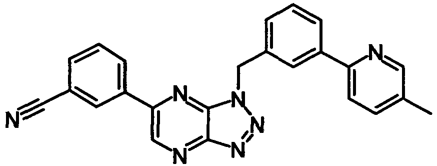 | 404 | |
| "A32" | 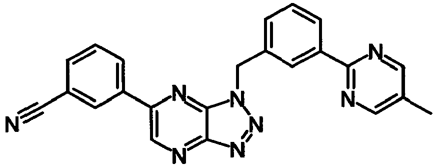 | 405 | |
| "A33" | 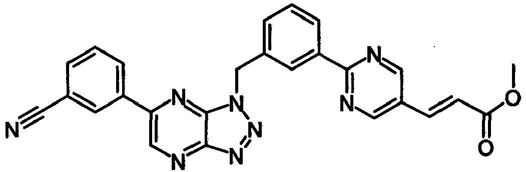 | 475 | |
| "A34" | 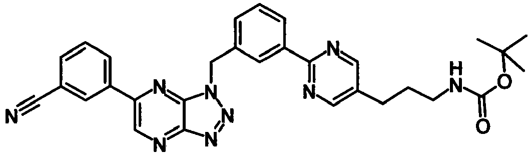 | 548 | |
| "A35" | 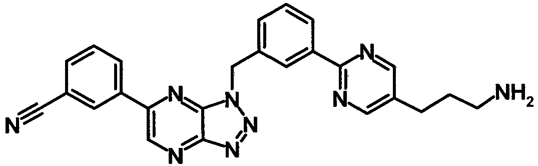 | 448 | |
| "A36" | 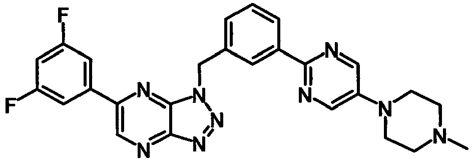 | 500 | |
| "A37" | 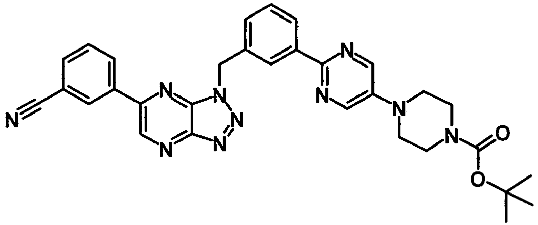 | 575 | |
| "A38" | 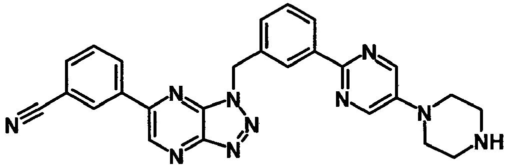 | 475 | |
| "A39" | 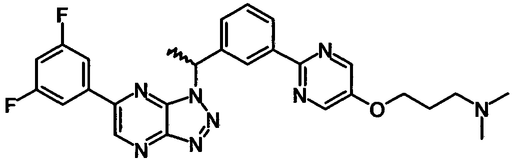 | 517 | |
| "A40" | 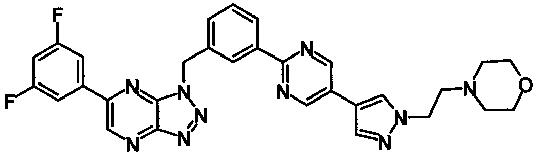 | 549 | |
| "A41" | 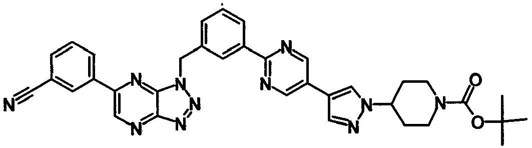 | 640 | |
| "A42" | 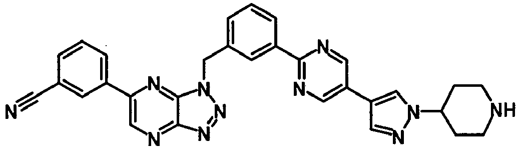 | 540 | |
| "A43" | 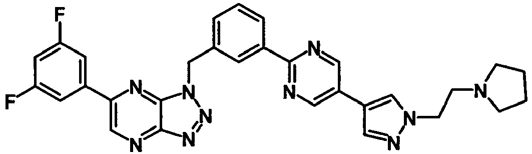 | 565 | |
| "B15" | 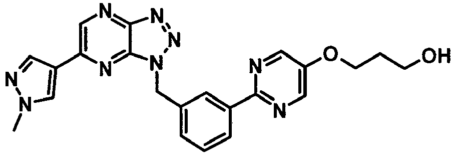 | 444 | 2.50 (Methode A) |
| | Hydrochlorid | | |
| ¹H-NMR (500 MHz, DMSO-d₆) δ [ppm] 9.19 (s, 1H), 8.63 (s, 4H), 8.46 (s, 1H), 8.32 (s, 1H), 8.25 (d, J=7.7, 1H), 7.54 (d, J=7.7, 1H), 7.50 (t, J=7.6, 1H), 6.03 (s, 2H), 4.25 (t, J=6.3, 2H), 3.95 (s, 3H), 3.58 (t, J=6.2, 2H), 1.91 (p, J=6.3, 2H) | | | |
| "B16" | 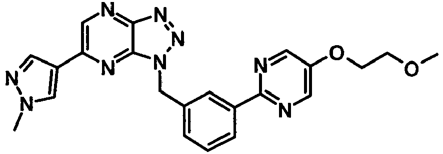 | 444 | 2.66 (Methode A) |
| | Hydrochlorid | | |
| ¹H-NMR (500 MHz, DMSO-d₆) δ [ppm] 9.19 (s, 1H), 8.65 (s, 2H), 8.63 (s, 1H), 8.45 (s, 1H), 8.32 (s, 1H), 8.26 (d, J=7.7, 1H), 7.55 (d, J=7.7, 1H), 7.51 (t, J=7.6, 1H), 6.04 (s, 2H), 4.32 (dd, J=3.6, 5.3, 2H), 3.95 (s, 3H), 3.74-3.66 (m, 2H), 3.47 (s, 3H) | | | |
| "B17" | 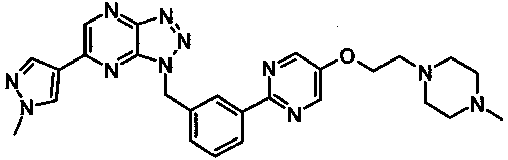 | 512 | 2.12 (Methode A) |
| | Hydrochlorid | | |
| ¹H-NMR (500 MHz, DMSO-d₆) δ [ppm] 9.19 (s, 1H), 8.64 (s, 3H), 8.43 (s, 1H), 8.31 (s, 1H), 8.26 (d, J=7.8, 1H), 7.56 (m, 1H), 7.51 (t, J=7.7, 1H), 6.03 (s, 2H), 4.32 (m, 2H), 3.95 (s, 3H), 3.66-3.37 (m, 2H), 2.77 (s, 3H), 1.42 (m, 8H) | | | |
| "B18" | 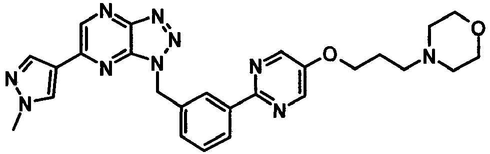 | 513 | 2.26 (Methode A) |
| ¹H-NMR (500 MHz, DMSO-d₆) δ [ppm] 9.19 (s, 1H), 8.63 (s, 3H), 8.46 (s, 1 H), 8.32 (s, 1H), 8.25 (d, J=7.7, 1H), 7.54 (d, J=7.6, 1H), 7.50 (t, J=7.6, 1H), 6.03 (s, 2H), 4.23 (t, J=6.3, 2H), 3.95 (s, 3H); 3.61-3.47 (m,4H),2.65- 2.29 (m, 6H), 1.92 (m, 2H) | | | |
| "B19" | 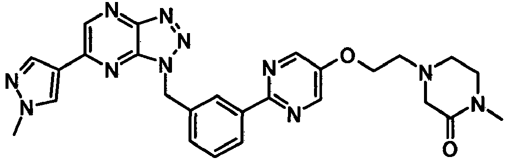 | 526 | 2.16 (Methode A) |
| ¹H-NMR (500 MHz, DMSO-d₆) δ [ppm] 9.19 (s, 1H), 8.64 (s, 2H), 8.63 (s, 1H), 8.45 (s, 1H), 8.31 (s, 1H), 8.25 (d, J=7.6, 1H), 7.54 (d, J=7.7, 1H), 7.50 (t, J=7.6, 1H), 6.04 (s, 2H), 4.32 (t, J=5.4, 2H), 3.95 (s, 3H), 3.56-3,29 (m, 6H), 2.81 (s, 3H), 2.50 (dt, J=1.8, 3.6, 2H) | | | |
| "B20" | 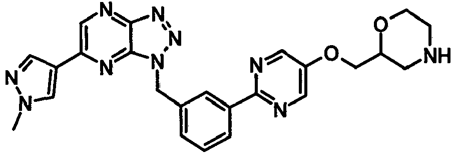 | 485 | 2.23 (Methode A) |
| | Hydrochlorid | | |
| ¹H-NMR (500 MHz, DMSO-d₆) δ [ppm] 9.30 (s, 1H), 9.19, s, 1H), 8.66 (s, 2H), 8.63 (s, 1H), 8.44 (s, 1H), 8.32 (s, 1H), 8.26 (d, J=7.7, 1H), 7.56 (d, J=7.7, 1H), 7.51 (t, J=7.7, 1H), 6.04 (s, 2H), 5.57 (s, 1H), 4.30 (dd, J=4.5, 10.1, 2H), 4.12 (m, 1H), 3.95 (s, 3H), 3.41- 2.38 (m, 6H) | | | |
| "B21" | 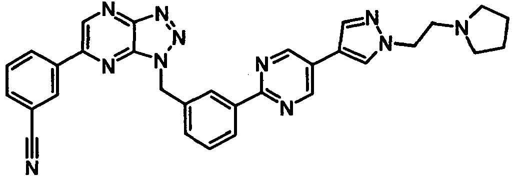 | 554 | 2.64 (Methode A) |
| | Hydrochlorid | | |
| ¹H-NMR (500 MHz, DMSO-d₆) δ [ppm] 9.59 (s, 1H), 9.16 (s, 2H), 8.80 (s, 1H), 8.67(d, J=8.1, 1H), 8.62 (s, 1H), 8.49 (s, 1H), 8.36 (d, J=7.8, 1H), 8.23 (s, 1H), 8.08 (d, J=7.7, 1H); 7.84 (t, J=7.9, 1H), 7.65 (t, J=8.1, 1H), 7.62 (d, J=4.8, 1H), 6.20 (s, 2H), 4.58 (t, J=6.1, 2H), 3.70 (t, 2H), 3.55 (m, 2H), 3.06 (m, 2H), 2.01 (m, 2H), 1.85 (m, 2H) | | | |
| "B22" | 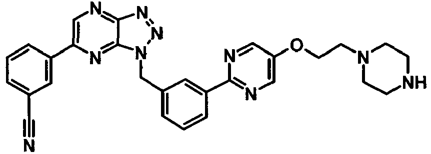 | 519 | 2.30 (Methode A) |
| | Hydrochlorid | | |
| ¹H-NMR (500 MHz, DMSO-d₆) δ [ppm] 9.58(s, 1H), 8.79 (s, 1H), 8.66 (d, J=10.0, 1H), 8.64 (s, 2H), 8.53 (s, 1H), 8.27 (d, J=7.9, 1H), 8.07 (d, J=7.8, 1H), 7.84 (t, J=7.9, 1H), 7.61 (d, J=7.7, 1H), 7.52 (t, J=7.7, 1H), 6.17 (s, 2H), 4.34 (m, 2H), 3.82 (s, 1H), 3.13 (m, 2H), 2.97-2.85 (m, 4H), 2.73- 2.54 (m, 4H) | | | |
| "B23" | 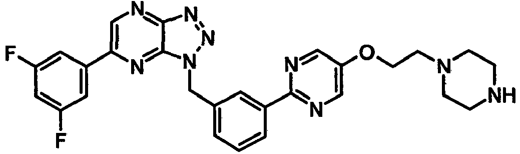 | 530 | 2.45 (Methode A) |
| | Hydrochlorid | | |
| ¹H-NMR (500 MHz, DMSO-d₆) δ [ppm] 9.56 (s, 1H), 9.32 (m, 1H), 8.69 (s, 2H), 8.58 (s, 1H), 8.28 (d, J=7.9, 1H), 8.10 (m, 2H), 7.62 (d, J=7.7, 1H), 7.52 (m, 2H), 6.17 (s, 2H), 4.59 (m, 2H), 3.74 (m, 9H), 3.42 (m, 2H) | | | |
| "B24" | 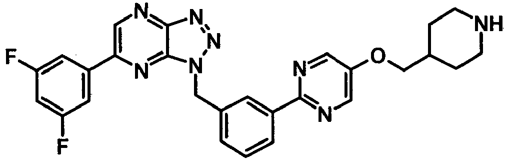 | 515 | 2.73 (Methode A) |
| | Hydrochlorid | | |
| ¹H-NMR (500 MHz, DMSO-d₆) δ [ppm] 9.56 (s, 1H), 8.76 (m, 1H), 8.63 (s, 2H), 8.57 (s, 1H), 8.45 (m, 1H), 8.27 (d, J=7.9, 1H), 8.20 (d, J=6.6, 2H), 7.61 (d, J=7.7, 1H), 7.55-7.46 (m, 2H), 6.16 (s, 2H), 4.10 (d, J=6.3, 2H), 3.30 (m, 2H), 2.91 (m, 2H), 2.12 (m, 1H), 1.93 (m, 2H), 1.50 (m, 2H) | | | |
| "B25" | 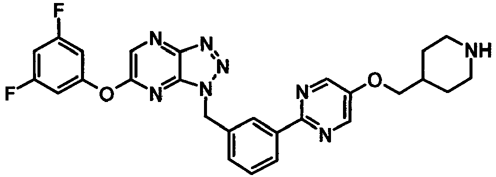 | 531 | |
| | Hydrochlorid | | |
| "B26" | 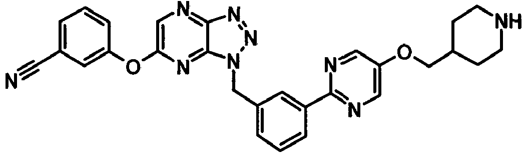 | 520 | |
| | Hydrochlorid | | |
| "B27" | 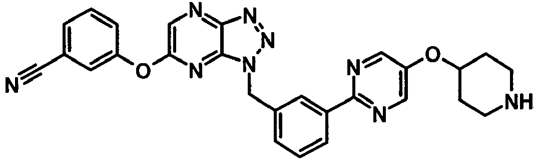 | 506 | |
| | Hydrochlorid | | |
| "B28" | 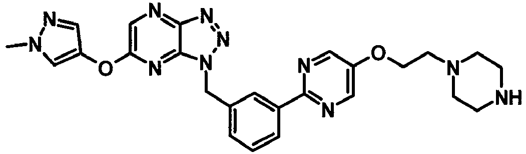 | 514 | |
| | Hydrochlorid | | |
| "B29" | 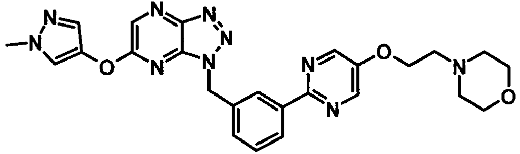 | 515 | |
| | Hydrochlorid | | |
| "B30" | 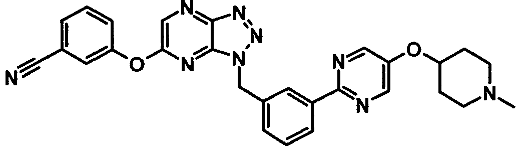 | 520 | |
| | Hydrochlorid | | |
| "B31" | 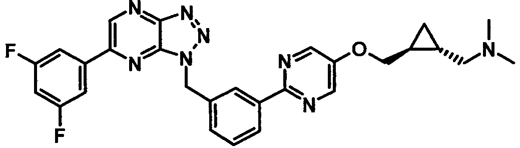 | 529 | |
| | Hydrochlorid | | |
| "B32" | 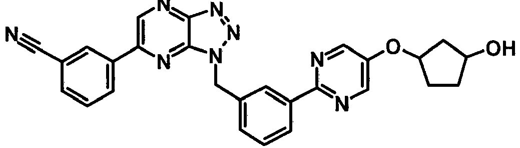 | 491 | |
| "B33" | 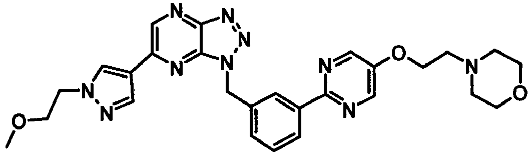 | 543 | 2.08 (Methode C) |
| ¹H-NMR (500 MHz, DMSO-d₆) δ [ppm] 9.21 (s, 1H), 8.67 - 8.61 (m, 3H), 8.46 (b, 1H), 8.35 (s, 1H), 8.25 (d, *J* = 7.6, 1H), 7.47-7.57 (m, 2H), 6.04 (s, 2H), 4.37 (t, *J* = 5.2, 2H), 4.28 (t, 2H), 3.72 (t, 2H), 3.60 - 3.53 (m, 4H), 3.23 (s, 3H), 2.73 (t, *J* = 5.6, 2H), 2.49 - 2.41 (m, 4H) | | | |
| "B34" | 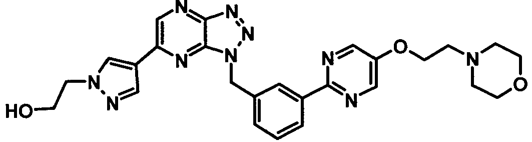 | 529 | 1.94 (Methode C) |
| ¹H-NMR (500 MHz, DMSO-d₆) δ [ppm] 9.22 (s, 1H), 8.69 - 8.60 (m, 3H), 8.46 (s, 1H), 8.35 (s, 1H), 8.29 - 8.22 (m, 1H), 7.47-7.57 (m, 2H), 6.04 (s, 2H), 4.98 (t, *J* = 5.3, 1H), 4.30 (t, *J* = 5.6, 2H), 4.25 (t, *J* = 5.4, 2H), 3.79 (q, *J* = 5.4, 2H), 3.62 - 3.52 (m, 4H), 2.73 (t, *J* = 5.6, 2H), 2.40 - 2.50 (m, 4H) | | | |

### Herstellung von 1-{3-[5-(2-Morpholin-4-yl-ethoxy)-pyrimidin-2-yl]-benzyl]-1H-benzotriazol ("A3"):

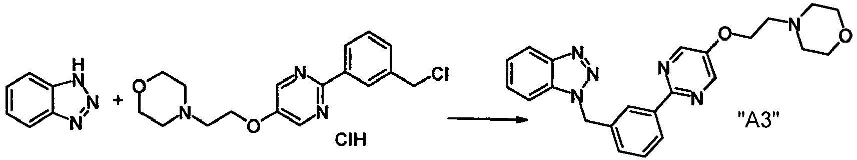

49 mg (0.41 mmol) 1 H-Benzotriazol, 150 mg (0.41 mmol) 4-{2-[2-(3-Chlormethyl-phenyl)-pyrimidin-5-yloxy]-ethyl}-morpholin Hydrochlorid und 136 mg (1.62 mmol) Natriumhydrogencarbonat werden in 4 ml Acetonitril suspendiert und 18 bei 90°C gerührt. Das Reaktionsgemisch wird mit Wasser versetzt und mit Ethylacetat extrahiert. Die organische Phase wird über Natriumsulfat getrocknet, eingeengt und mittels Säulenchromatographie an Kieselgel gereinigt.
Ausbeute: 14 mg; Rt. = 2.27 min; LCMS: 417 (M+H);
¹H-NMR (500 MHz, DMSO-d₆) δ [ppm] 8.63 (s, 2H), 8.27 (b, 1H), 8.23 (td, 1 H), 8.06 (d, 1 H), 7.86 (d, 1 H), 7.38-7.56 (m, 4H), 6.09 (s, 2H), 4.29 (2, 1H), 3.57 (t, 4H), 2.72 (t, 2H), 2.45-2.49 (b, 4 H).

### Analog erhält man 5-Chlor-1-{3-[5-(2-morpholin-4-yl-ethoxy)-pyrimidin-2-yl]-benzyl}-1H-benzotriazol ("B35")

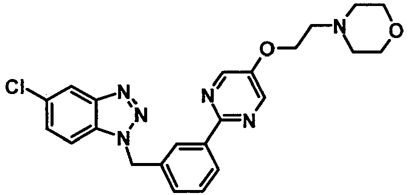

HPLC: Rt = 2.23 min (Methode B), LC-MS: [M+H]⁺ = 451;
¹H NMR (500 MHz, DMSO-d₆) δ [ppm] 8.63 (s, 2H), 8.29 - 8.21 (m, 3H), 7.94 (d, *J* = 8.8, 1 H), 7.59 (dd, J = 8.8, 1.8, 1 H), 7.53 - 7.37 (m, 2H), 6.09 (d, *J* = 12.7, 2H), 4.30 (t, *J* = 5.6, 2H), 3.61 - 3.55 (m, 4H), 2.72 (t, *J* = 5.6, 2H), 2.49 - 2.41 (m, 4H).

### Herstellung von 5-Chlor-3-(3-[5-(2-morpholin-4-yl-ethoxy)-pyrimidin-2-yl]-benzyl}-3H-[1,2,3]triazolo[4,5-b]pyridin "A48")

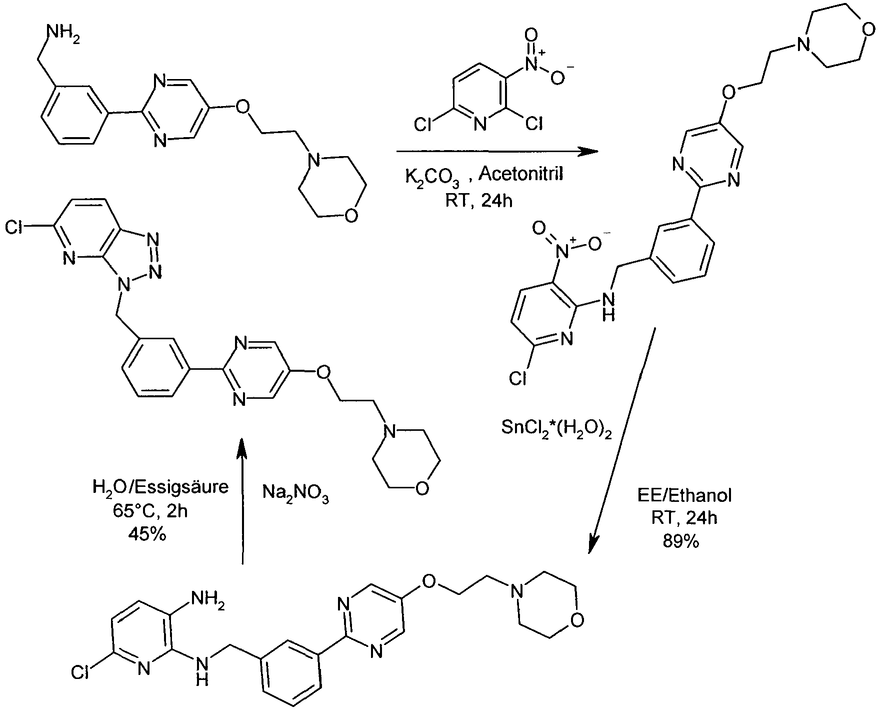

### Stufe 1:

87.8 mg (0.442 mmol) 2,6-Dichlor-3-nitro-pyridin wird in 3 ml Acetonitril gelöst und mit 144 mg (0.442 mmol) Kaliumcarbonat versetzt. Anschließend wird auf 0°C gekühlt und eine Lösung aus 200 mg (0.442 mmol) 3-[5-(2-Morpholin-4-yl-ethoxy)-pyrimidin-2-yl]benzylamin in 3 ml Acetonitril wird zugetropft. Das Reaktionsgemisch wird 3.5 h bei RT gerührt. Anschließend wird das Reaktionsgemisch mit Wasser und Ethylacetat versetzt. Die organische Phase wird über Natriumsulfat getrocknet, unter vermindertem Druck eingedampft und säulenchromatographisch an Kieselgel aufgereinigt. Ausbeute: 115 mg, HPLC: Rt = 2.50 min (Methode B), LC-MS: [M+H]⁺ = 471.

### Stufe 2:

115 mg (0.244 mmol) (6-Chlor-3-nitro-pyridin-2-yl)-{3-[5-(2-morpholin-4-yl-ethoxy)-pyrimidin-2-yl]-benzyl}-amin werden in 7 ml Ethylacetat und 3 ml Ethanol gelöst. 275 mg (1.22 mmol) Zinn(II)-chlorid-Dihydrat werden zugegeben und das Reaktionsgemisch 24 h bei 55°C gerührt. Das Reaktionsgemisch wird mit 32%iger NaOH auf pH 7 eingstellt. Der enstandene Niederschlag wird über Celite abgesaugt und mit EE gewaschen. Das Filtrat wird extrahiert. Anschließend wird die organische Phase mit Wasser gewaschen, über Natriumsulfat getrocknet und unter vermindertem Druck eingedampft.
Ausbeute: 95.5 mg, HPLC: Rt = 2.22 min (Methode C), LC-MS: [M+H]⁺ = 441.

### Stufe 3:

95.5 mg (0.217 mmol) 6-Chloro-N'2'-{3-[5-(2-morpholin-4-yl-ethoxy)-pyrimidin-2-yl]-benzyl}pyridin-2,3-diamin werden in 2.4 ml Wasser und 2.4 ml Essigsäure gelöst. Eine Lösung aus 149.4 mg (2.166 mmol) Natriumnitrit in 2.4 ml Wasser wird zugegeben und 1 h bei RT gerührt. Anschließend wird die Lösung 4 h bei 65°C gerührt. Das Reaktionsgemisch wird mit NaOH neutralisiert und mit Ethylacetat und Wasser versetzt. Die organische Phase wird abgetrennt, die wässrige Phase wird mit Ethylacetat extrahiert und die vereinigten organischen Phasen über Natriumsulfat getrocknet. Das Lösungsmittel wird unter vermindertem Druck eingedampft und säulenchromatographisch an Kieselgel aufgereinigt.
Ausbeute: 45 mg, HPLC: Rₜ = 2.18 min (Methode C), LC-MS: [M+H]⁺ = 452; ¹H-NMR (500 MHz, DMSO-d₆) δ [ppm] 8.67 (d, J=8.6, 1 H), 8.64 (s, 2H), 8.31 (s, 1 H), 8,26 (d, 1 H), 7.61 (d, J=8.6, 1 H), 7.50 (t, J=7.6, 1 H), 7.45 (d, J=7.7, 1H), 6.02 (s, 2H), 4.30 (t, J=5.6, 2H), 3,59-3.54 (m, 4H), 2.73 (t, J=5.6, 2H), 2.49 (ddd, J=3.1, 6.2, 12.6, 4H).

Herstellung von (5-Brom-2-nitro-phenyl)-{3-[5-(2-morpholin-4-yl-ethoxy)-pyrimidin-2-yl]-benzyl}-amin ("B36")

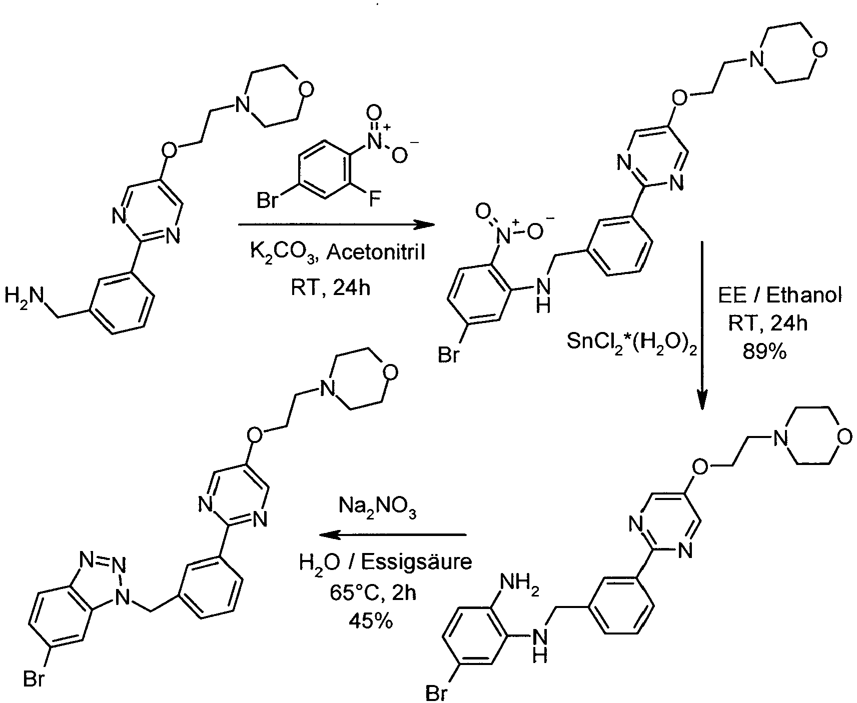

### Stufe 1:

496 mg (0.208 mmol) 4-Brom-2-fluor-1-nitrobenzol wird in 10 ml Acetonitril gelöst und mit 0.305 g (0.208 mmol) Kaliumcarbonat versetzt. Anschließend wird auf 0°C gekühlt und eine Lösung aus 1.00 g (0.208 mmol) 3-[5-Morpholin-4-yl-ethoxy)-pyrimidin-2-yl]-benzylamin in 10 ml Acetonitril wird zugetropft. Das Reaktionsgemisch wird 3.5 h bei RT gerührt.

Anschließend wird das Reaktionsgemisch mit Wasser und Ethylacetat versetzt. Die organische Phase wird über Natriumsulfat getrocknet, unter vermindertem Druck eingedampft und säulenchromatographisch an Kieselgel aufgereinigt; Ausbeute: 1.29 g, HPLC: Rt = 2.61 min (Methode B), LC-MS: [M+H]⁺ = 514/516.

### Stufe 2:

763 mg (1.35 mmol) (5-Brom-2-nitro-phenyl)-{3-[5-(2-morpholin-4-yl-ethoxy)-pyrimidin-2-yl]-benzyl}-amin werden in 14 ml Ethylacetat und 6 ml Ethanol gelöst. 1.53 g (6.75 mmol) Zinn(II)-chlorid-Dihydrat werden zugegeben und das Reaktionsgemisch 24 h bei 55°C gerührt. Das Reaktionsgemisch wird mit 32%iger NaOH auf pH 7 eingstellt. Der enstandene Niederschlag wird über Celite abgesaugt und mit EE gewaschen. Das Filtrat wird extrahiert. Anschließend wird die organische Phase mit Wasser gewaschen, über Natriumsulfat getrocknet und unter vermindertem Druck eingedampft; Ausbeute: 617 mg, HPLC: Rt = 2.28 min (Methode C), LC-MS: [M+H]⁺ = 484/486.

### Stufe 3:

617 mg (1.11 mmol) 4-Bromo-N'-2'-{3-[5-(2-morpholin-4-yl-ethoxy)-pyrimidin-2-yl]-benzyl}-benzen-1,2-diamin werden in 7.2 ml Wasser und 7.2 ml Essigsäure gelöst. Eine Lösung aus 776 mg (11.1 mmol) Natriumnitrit in 7.2 ml Wasser wird zugegeben und 1 h bei RT gerührt. Anschließend wird die Lösung 4 h bei 65°C gerührt. Das Reaktionsgemisch wird mit NaOH neutralisiert und mit Ethylacetat und Wasser versetzt. Die organische Phase wird abgetrennt, die wässrige Phase wird mit Ethylacetat extrahiert und die vereinigten organischen Phasen über Natriumsulfat getrocknet. Das Lösungsmittel wird unter vermindertem Druck eingedampft und säulenchromatographisch an Kieselgel aufgereinigt; Ausbeute: 484 mg, HPLC: Rt = 2.27 min (Methode C), LC-MS: [M+H]⁺ = 495/497.

### Herstellung von 6-(1-Methyl-1H-pyrazol-4-yl)-1-{3-[5-(2-morpholin-4-yl-ethoxy)-pyrimdin-2-yl]-benzyl}-1H-benzotriazol ("A50")

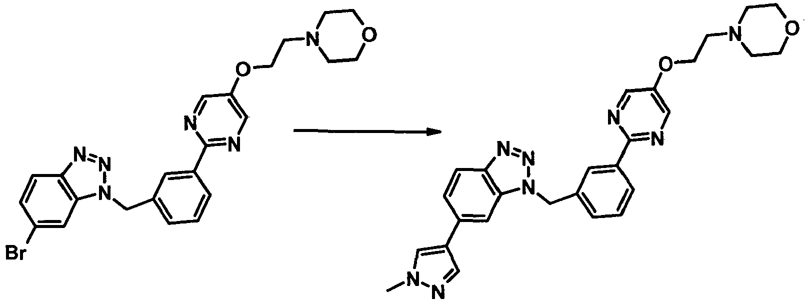

200 mg (0.352 mmol) 6-Brom-1-{3-[5-(2-morpholin-4-yl-ethoxy)-pyrimdin-2-yl]-benzyl}-1H-benzotriazol, 80.6 mg (0.387 mmol) 1-Methyl-4-(4,4,5,5-tetramethyl-[1,3,2]dioxaborolan-2-yl)-1H-pyrazol und 150 mg (0.704 mmol) tri-Kaliumphosphat Trihydrat werden in 6 ml Ethylenglykoldimethylether suspendiert, mehrfach entgast evakuiert und mit Stickstoff gespült. 24.7 mg (0.035 mmol) Bis(triphenylphosphin)-palladium(II)-chlorid werden hinzugegeben und nochmals evakuiert und mit Stickstoff gespült. Das Reaktionsgemisch wird 24 h bei 80°C gerührt. Das Reaktionsgemisch wird mit Wasser versetzt, mit 32%iger NaOH basisch gestellt und mit DCM extrahiert. Die organische Phase wird über Natriumsulfat getrocknet, unter vermindertem Druck am Rotationsverdampfer eingeengt und säulenchromatographisch an Kieselgel aufgereinigt; Ausbeute: 41 mg, HPLC: Rt = 2.08 min (Methode C), LC-MS: [M+H]⁺ = 497;
¹H-NMR (500 MHz, DMSO-d₆) δ [ppm] 8.63 (s, 2H), 8.29 (s, 1H), 8.26-8.20 (m, 2H), 8.10 (s, 1 H), 8.02 (d, J=8.7, 1 H), 7.96 (s, 1 H), 7.63 (dd, J=1.4, 8.7, 1H), 7.49 (dd, J=1.7, 4.9,2H), 6.05 (s, 2H), 4.29 (t, J=5.6, 2H), 3.88 (s, 3H), 3.61-3.51 (m, 4H), 2.72 (t, J=5.6, 2H), 2.53-2.44 (m, 4H).

### Herstellung von 3-{3-[5-(2-Morpholin-4-yl-ethoxy)-pyrimidin-2-yl]-benzyl}-3H-[1,2,3]triazolo[4,5-b]pyridine ("A44"):

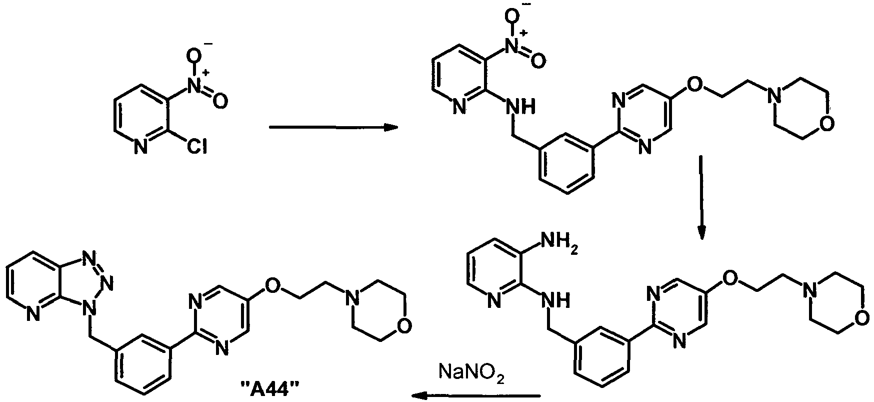

**Analog werden die nachstehenden Verbindungen hergestellt**

| Verbindung Nr. | Name und/oder Struktur | LCMS [M+H] | HPLC Rt. in min |
|---|---|---|---|
| "A45" | 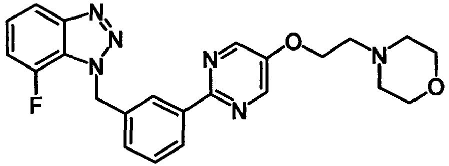 | 435 | |
| "A46" | 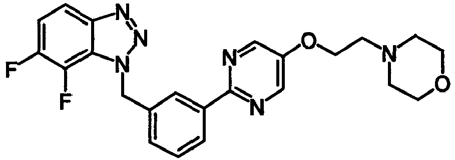 | 453 | 2.21 (Methode C) |
| ¹H-NMR (500 MHz, DMSO-d₆) δ [ppm] 8.63 (s, 2H), 8.25 (d, J=9.2 2H), 7.98 (m, 1H), 7.55-7.47 (m, 2H), 7.38 (d, J=7.5 1H), 6.10 (s, 2H), 3.61-3.54 (m, 2H), 3.28 (m, 4H), 2.72 (t, J=5.6, 2H), 2.50 (m, 4H) | | | |
| "A47" | 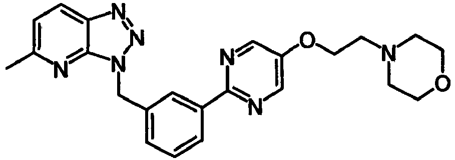 | 432 | |
| "A48" | 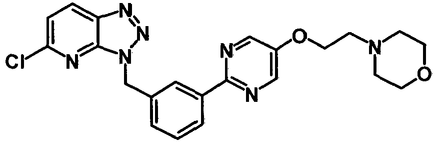 | 452 | |
| "A49" | 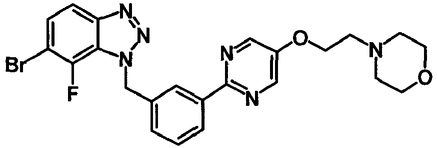 | 413/415 | |
| "A50" | 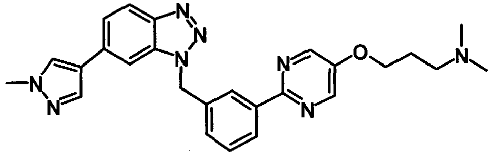 | 469 | |
| "B37" | 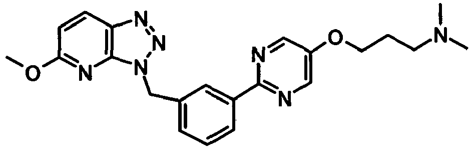 | 420 | 2.43 (Methode A) |
| ¹H-NMR (500 MHz, DMSO-d₆) δ [ppm] 9.45 (s, 1H) 8.64 (s, 2H), 8.44 (s, 1H), 8.39 (d,J=8.9, 1H), 8.25 (d, J=7.7, 1H), 7.55 (d, J=7.8, 1 H), 7.50 (t, J=7.6, 1H), 6.93 (d, J=8.9, 1 H), 5.91 (s, 2H), 4.28 (t, J=5.9, 2H), 4.04 (s, 3H), 3.30 (m, 2H), 2.82 (s, 6H), 2.20-2.15 (m, 2H) | | | |

### Herstellung von 5-(1-Methyl-1H-pyrazol-4-yl)-3-{3-[5-(2-morpholin-4-yl-ethoxy)-pyrimidin-2-yl]-benzyl}-3H-[1,2,3]triazolo[4,5-d]pyrimidin ("A51"):

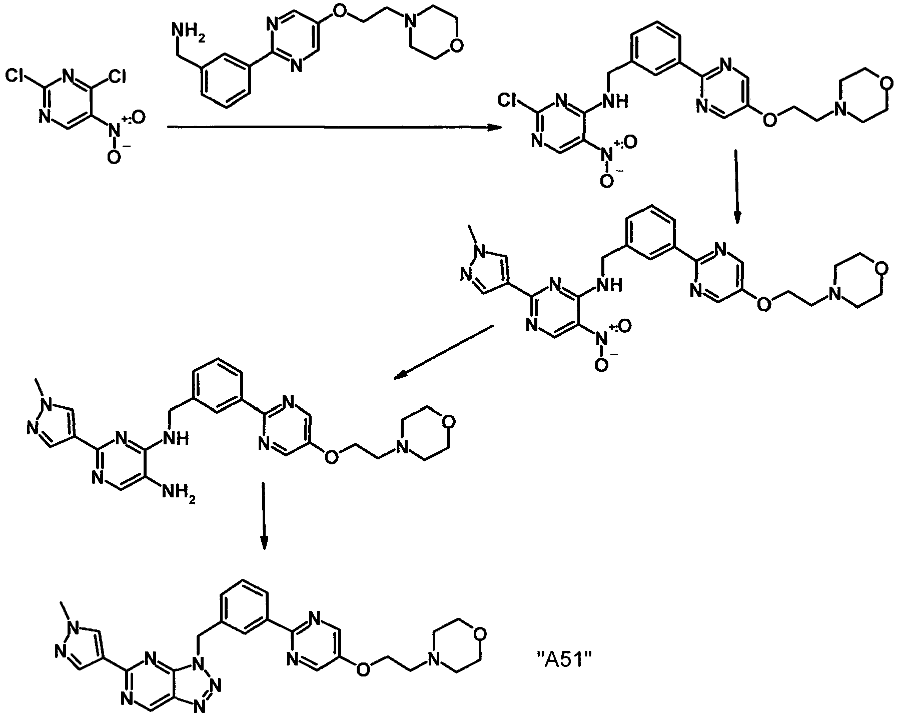

**Analog werden die nachstehenden Verbindungen erhalten:**

| Verbindung Nr. | Name und/oder Struktur | LCMS [M+H] | Rt. in min |
|---|---|---|---|
| "A52" | 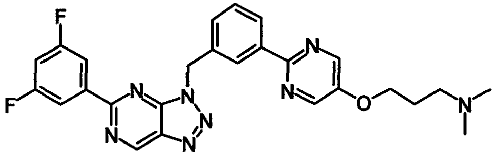 | 503 | |
| "A53" | 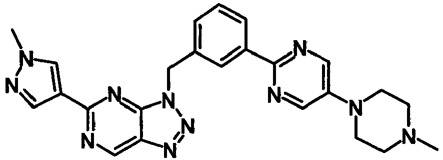 | 468 | |
| "A54" | 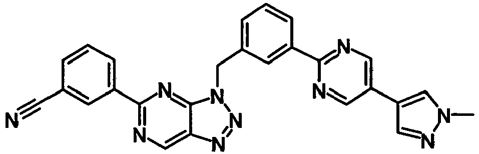 | 471 | |

### Herstellung von 1-{3-[5-(2-Morpholin-4-yl-ethoxy)-pyrimidin-2-yl]-benzyl}-6-(propan-1-sulfonyl)-1H-benzotriazol ("A55"):

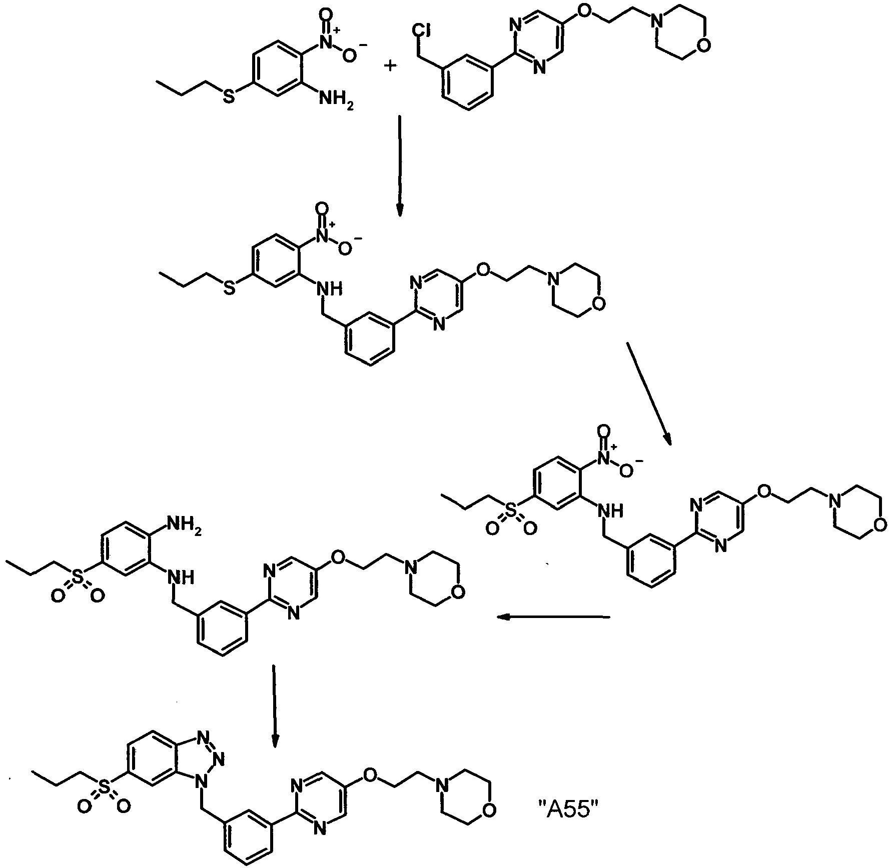

### Pharmakologische Daten

**Tabelle 2 Met-Kinase-Inhibierung**

| Verbindung Nr. | Biochemischer Assay | Zellulärer Assay |
|---|---|---|
| | IC₅₀ (Enzym) | IC₅₀ (Zelle) |
| "A1" | | A |
| "A2" | A | A |
| "A3" | A | B |
| "A6" | A | A |
| "A9" | A | A |
| "A24" | A | A |
| "A27" | A | A |
| "A30" | A | A |
| "A46" | A | A |
| "A48" | A | B |
| "A50" | A | A |
| "B1" | A | B |
| "B5" | A | A |
| "B6" | A | A |
| "B7" | A | A |
| "B9" | A | A |
| "B10" | A | A |
| "B14" | A | A |
| "B15" | A | A |
| "B16" | A | A |
| "B17" | A | A |
| "B18" | A | A |
| "B19" | A | A |
| "B20" | A | A |
| "B21" | A | A |
| "B22" | A | A |
| "B23" | A | A |
| "B24" | A | A |
| "B33" | A | A |
| "B34" | A | A |
| "B35" | B | C |
| "B36" | A | B |
| "B37" | A | A |

| | | |
|---|---|---|
| IC₅₀: 1 nM - 0,1 µM = A 0,1 µM -10 µM = B > 10 µM = C | | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 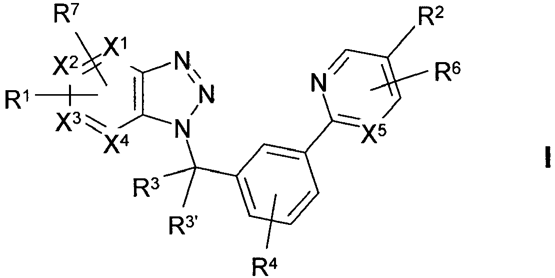 worin
X¹, X², X³, X⁴, X⁵ jeweils unabhängig voneinander CH oder N,
R¹ H, Hal, A, S(O)ₘA, Ar, Het, O[C(R⁵)₂]ₙAr, O[C(R⁵)₂]ₙHet
oder OR⁵,
R⁷ H oder Hal,
R² A, Hal, [C(R⁵)₂]ₙN(R⁵)₂, [C(R⁵)₂]ₙHet, O[C(R⁵)₂]ₚN(R⁵)₂, O[C(R⁵)₂]ₙHet, [C(R⁵)₂]ₙOR⁵, O[C(R⁵)₂]OR⁵, O-[C(R⁵)₂]ₙ-cycloalkylen-[C(R⁵)₂]ₙ-N(R5)₂, [C(R⁵)₂]ₙNR⁵COOA oder CH=CH-COOR⁵,
R³, R³' jeweils unabhängig voneinander H oder R⁸,
R⁴, R⁶ H,
R⁵ H oder R⁸,
R⁸ unverzweigtes oder verzweigtes Alkyl mit 1-6 C-Atomen,
A unverzweigtes oder verzweigtes Alkyl mit 1-10 C-Atomen,
worin 1-7 H-Atome durch OH, F, Cl und/oder Br ersetzt sein können,
oder
cyclisches Alkyl mit 3-7 C-Atomen, das einfach durch OH substituiert sein kann,
Ar unsubstituiertes oder ein-, zwei- oder dreifach durch Hal, A und/oder CN substituiertes Phenyl,
Het Piperidinyl, Pyrrolidinyl, Morpholinyl, Piperazinyl, Oxazolidinyl, Pyrazolyl, Pyridinyl, Pyrimidinyl, Furyl, Thienyl, Oxazolyl, Oxadiazolyl, Imidazolyl, Pyrrolyl, Isoxazolyl oder Imidazolidinyl, wobei die Reste auch ein- oder zweifach durch Hal, A, COOR⁵, O[C(R⁵)₂]ₚOR⁵, [C(R⁵)₂]ₙHet¹, O[C(R⁵)₂]ₙHet¹ und/oder = O substituiert sein können,
Het¹ Piperidinyl, Pyrrolidinyl, Morpholinyl, Piperazinyl, Oxazolidinyl oder Imidazolidinyl, wobei die Reste auch ein- oder zweifach durch COOA, =O und/oder A substituiert sein können,
Hal F, Cl, Br oder I,
m 0, 1 oder 2,
n 0, 1, 2, 3 oder 4,
p 1, 2, 3 oder 4
bedeuten,
sowie ihre pharmazeutisch verwendbaren Salze, Tautomere und Stereoisomere, einschließlich deren Mischungen in allen Verhältnissen.

2. Verbindungen nach Anspruch 1, ausgewählt aus der Gruppe
| Nr. | Struktur und/oder Name |
|---|---|
| "A1" | 6-Brom-1-{3-[5-(2-morpholin-4-yl-ethoxy)-pyrimidin-2-yl]-benzyl}-1H-[1,2,3]triazolo[4,5-b]pyrazin |
| "A2" | 6-(1-Methyl-1H-pyrazol-4-yl)-1-{3-[5-(2-morpholin-4-yl-ethoxy)-pyrimidin-2-yl]-benzyl}-1H-[1,2,3]triazolo[4,5-b]pyrazin |
| "A3" | 1-{3-[5-(2-Morpholin-4-yl-ethoxy)-pyrimidin-2-yl]-benzyl}-1H-benzotriazol |
| "A4" | 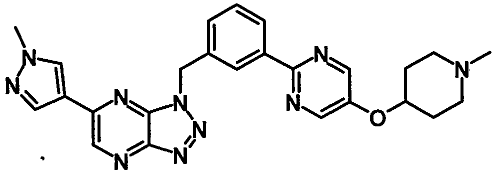 |
| "A5" | 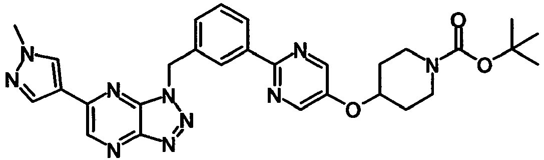 |
| "A6" | 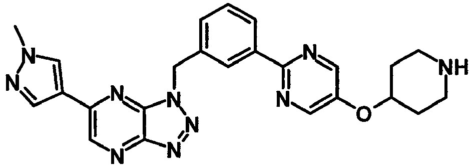 |
| "A7" | 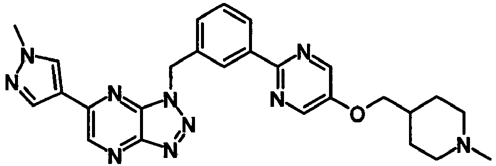 |
| "A8" | 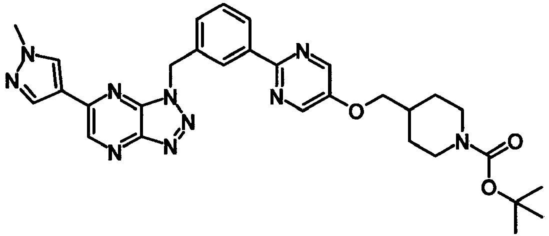 |
| "A9" | 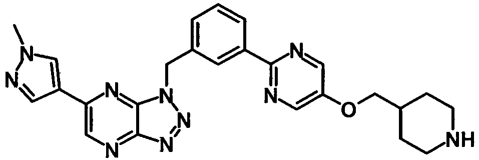 |
| "A10" | 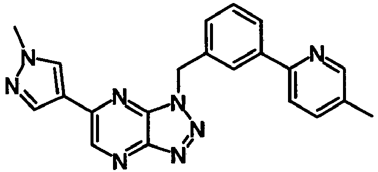 |
| "A11" | 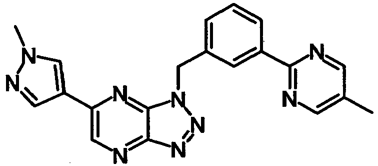 |
| "A12" | 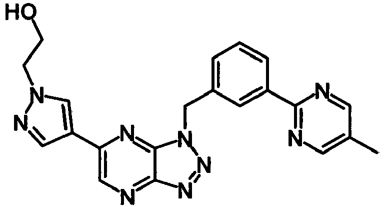 |
| "A13" | 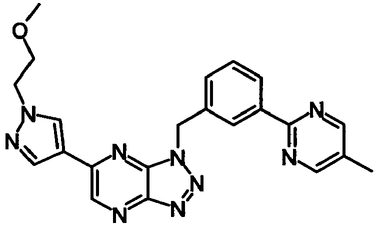 |
| "A14" | 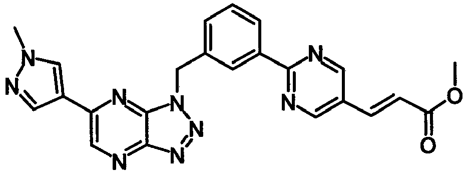 |
| "A15" | 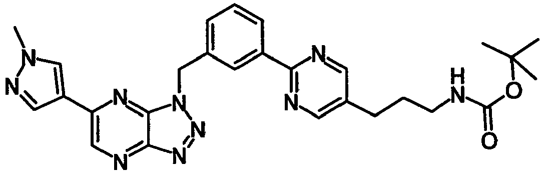 |
| "A16" | 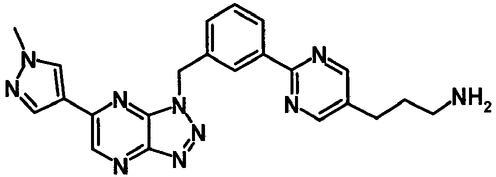 |
| "A17" | 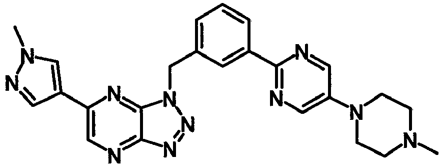 |
| "A18" | 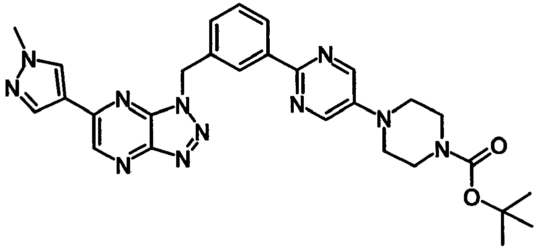 |
| "A19" | 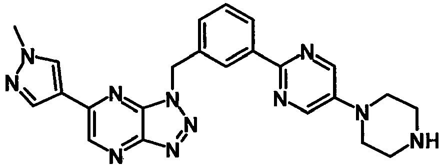 |
| "A20" | 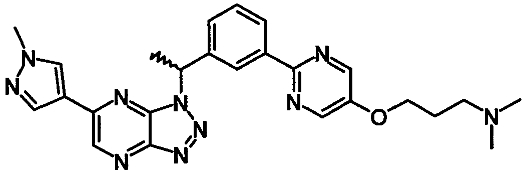 |
| "A21" | 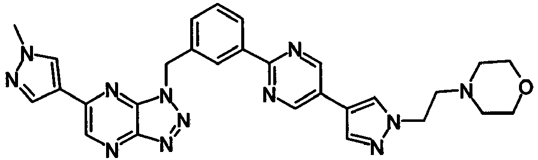 |
| "A22" | 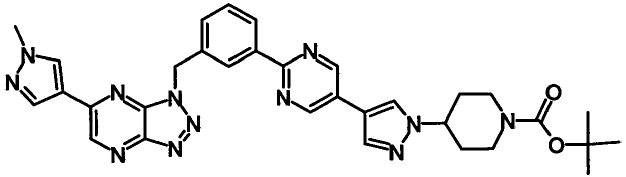 |
| "A23" | 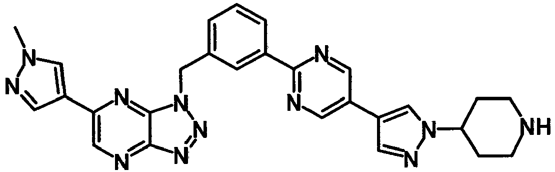 |
| "A24" | 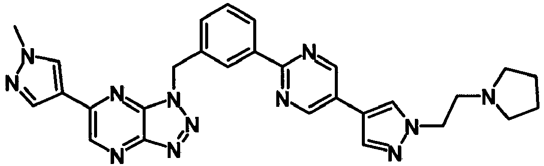 |
| "A25" | 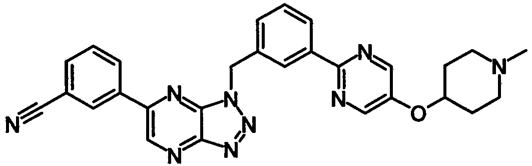 |
| "A26" | 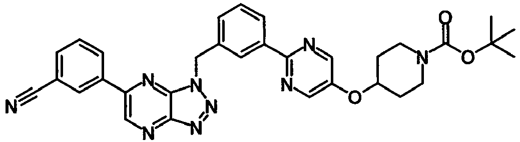 |
| "A27" | 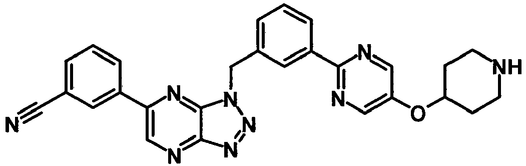 |
| "A28" | 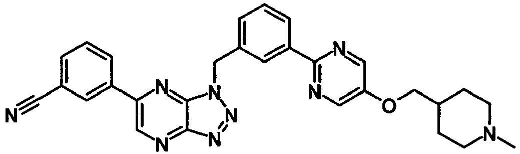 |
| "A29" | 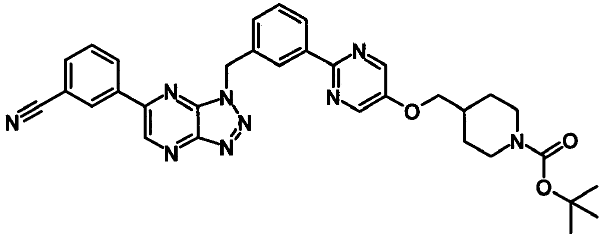 |
| "A30" | 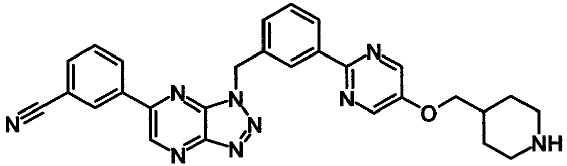 |
| "A31" | 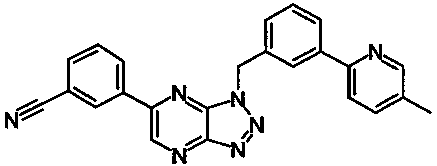 |
| "A32" | 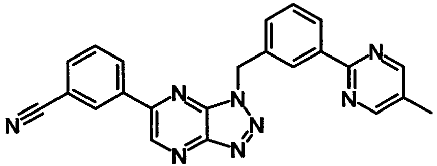 |
| "A33" | 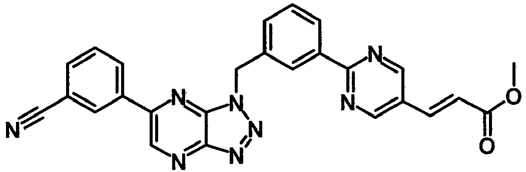 |
| "A34" | 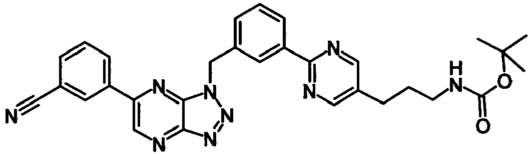 |
| "A35" | 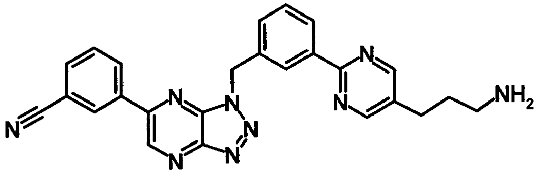 |
| "A36" | 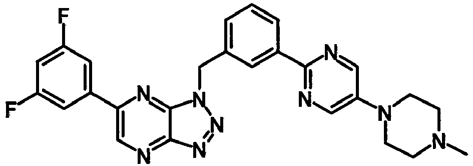 |
| "A37" | 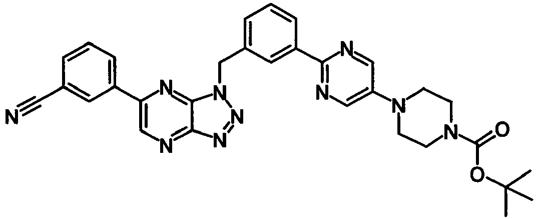 |
| "A38" | 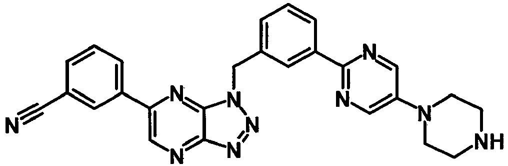 |
| "A39" | 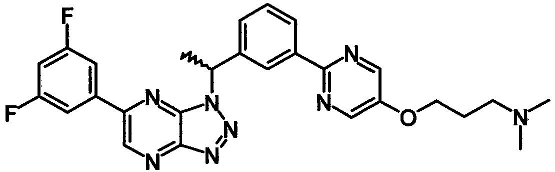 |
| "A40" | 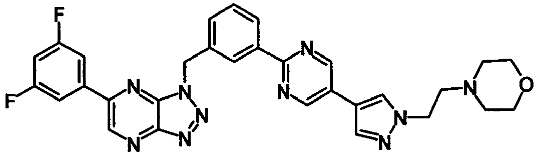 |
| "A41" | 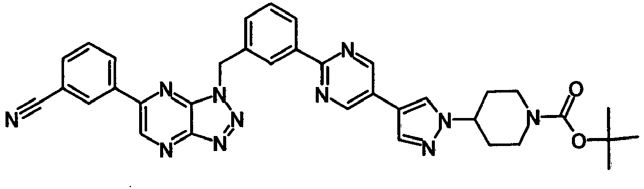 |
| "A42" | 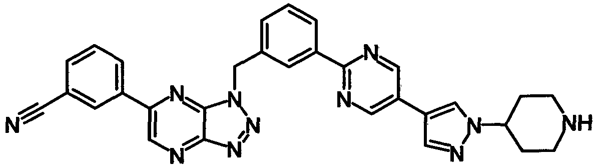 |
| "A43" | 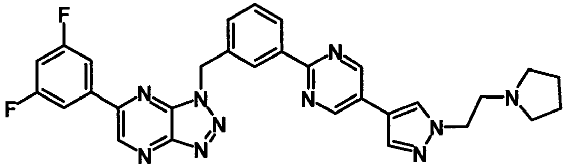 |
| "A44" | 3-{3-[5-(2-Morpholin-4-yl-ethoxy)-pyrimidin-2-yl]-benzyl}-3H-[1,2,3]triazolo[4,5-b]pyridine |
| "A45" | 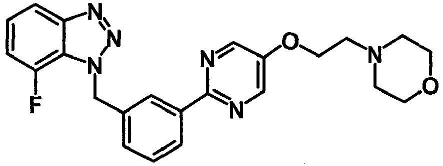 |
| "A46" | 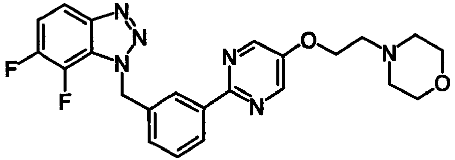 |
| "A47" | 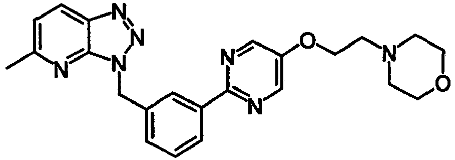 |
| "A48" | 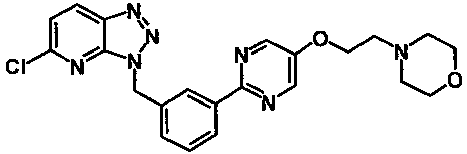 |
| "A49" | 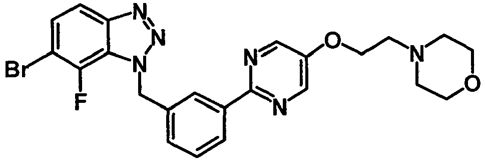 |
| "A50" | 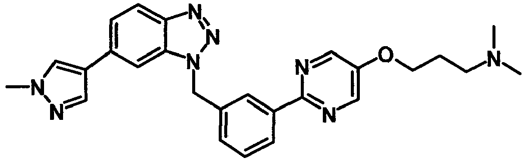 |
| "A51" | 5-(1-Methyl-1H-pyrazol-4-yl)-3-{3-[5-(2-morpholin-4-yl-ethoxy)-pyrimidin-2-yl]-benzyl}-3H-[1,2,3]triazolo[4,5-d]pyrimidin |
| "A52" | 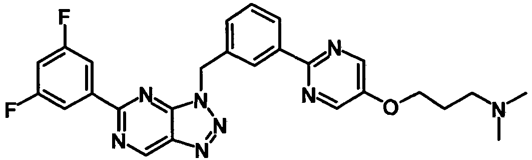 |
| "A53" | 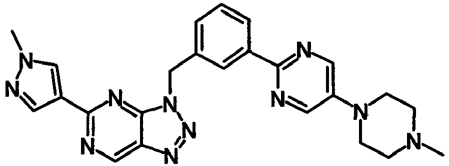 |
| "A54" | 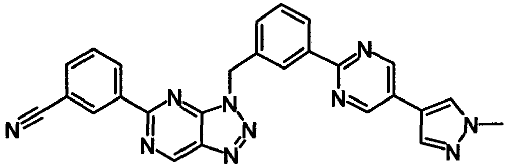 |
| "A55" | 1-{3-[5-(2-Morpholin-4-yl-ethoxy)-pyrimidin-2-yl]-benzyl}-6-(propan-1-sulfonyl)-1H-benzotriazol |
| "B1" | 6-Brom-1-[3-(5-brom-pyrimidin-2-yl)-benzyl]-1H-[1,2,3]triazolo[4,5-b]pyrazin |
| "B2" | 1-[3-(5-Brom-pyrimidin-2-yl)-benzyl]-6-(1-methyl-1H-pyrazol-4-yl)-1H-[1,2,3]triazolo[4,5-b]pyrazin |
| "B3" | 3-{3-[3-(5-Brom-pyrimidin-2-yl)-benzyl]-3H-[1,2,3]triazolo[4,5-b]pyrazin-5-yl}-benzonitril |
| "B4" | 1-[3-(5-Brom-pyrimidin-2-yl)-benzyl]-6-(3,5-difluorphenyl)-1H-[1,2,3]triazolo[4,5-b]pyrazin |
| "B5" | 1-[3-(5-Brom-pyrimidin-2-yl)-benzyl]-6-(1-methyl-1H-pyrazol-4-yloxy)-1H-[1,2,3]triazolo[4,5-b]pyrazin |
| "B6" | 3-{3-[3-(5-Brom-pyrimidin-2-yl)-benzyl]-3H-[1,2,3]triazolo[4,5-b]pyrazin-5-yloxyj-benzonitril |
| "B7" | 1-[3-(5-Brom-pyrimidin-2-yl)-benzyl]-6-(3,5-difluor-phenoxy)-1H-[1,2,3]triazolo[4,5-b]pyrazin |
| "B8" | 2-{3-[6-(1-Methyl-1H-pyrazol-4-yl)-[1,2,3]triazolo[4,5-b]pyrazin-1-ylmethyl]-phenyl}-pyrimidin-5-ol |
| "B9" | 2-{3-[3-(3-Hydroxyl-pyrimidin-2-yl)-benzyl]-3H-[1,2,3]triazolo[4,5-b]pyrazin-5-yl}-benzonitril |
| "B10" | 2-{3-[6-(3,5-Difluor-phenyl)-[1,2,3]triazolo[4,5-b]pyrazin-1-ylmethyl]-phenyl}-pyrimidin-5-ol |
| "B11" | 2-{3-[6-(1-Methyl-1H-pyrazol-4-yloxy)-[1,2,3]triazolo[4,5-b]pyrazin-1-ylmethyl]-phenyl}-pyrimidin-5-ol |
| "B12" | 3-{3-[3-(5-Hydroxy-pyrimidin-2-yl)-benzyl]-3H-[1,2,3]triazolo[4,5-b]pyrazin-5-yloxy}-benzonitril |
| "B13" | 2-{3-[6-(3,5-Difluor-phenoxy)-[1,2,3]triazolo[4,5-b]pyrazin-1-ylmethyl]-phenyl}-pyrimidin-5-ol |
| "B14" | Dimethyl-[2-(2-{3-[6-(1-methyl-1H-pyrazol-4-yl)-[1,2,3]triazolo[4,5-b]pyrazin-1-ylmethyl]-phenyl}-pyrimidin-5-yloxy)-ethyl]-amin |
| "B15" | 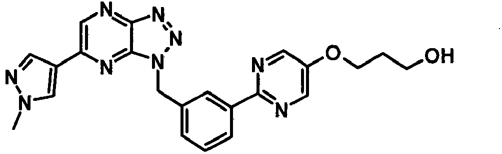 |
| "B16" | 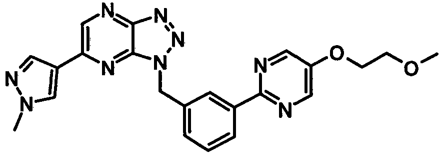 |
| "B17" | 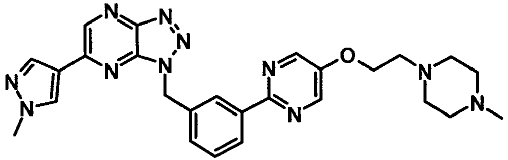 |
| "B18" | 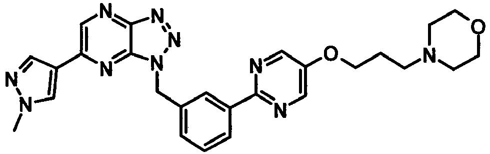 |
| "B19" | 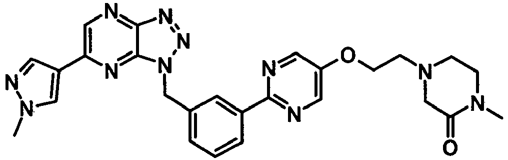 |
| "B20" | 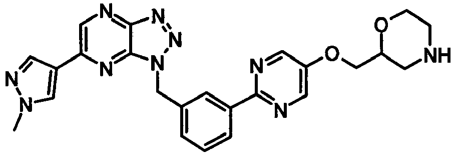 |
| "B21" | 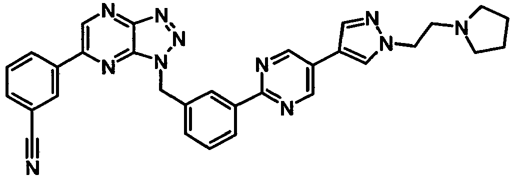 |
| "B22" | 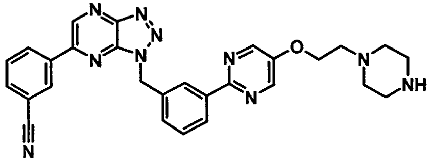 |
| "B23" | 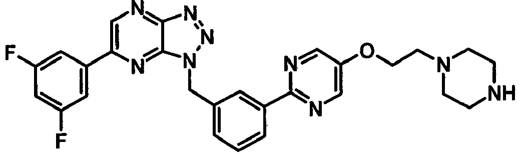 |
| "B24" | 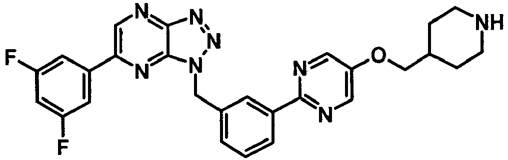 |
| "B25" | 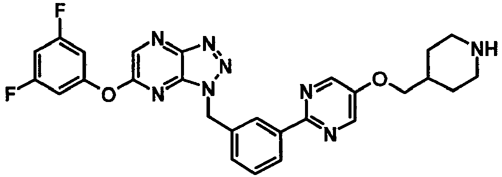 |
| "B26" | 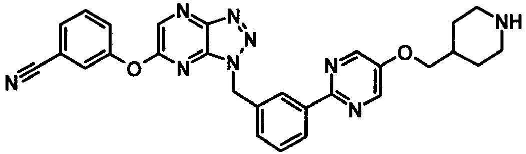 |
| "B27" | 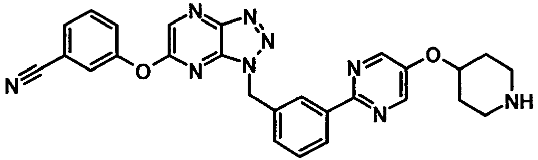 |
| "B28" | 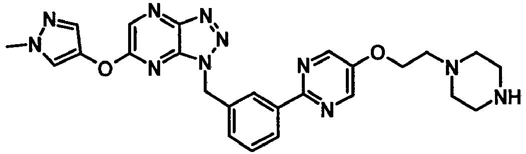 |
| "B29" | 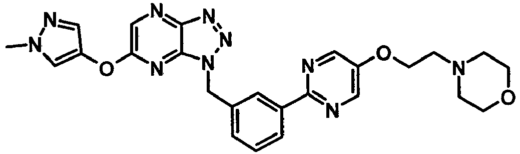 |
| "B30" | 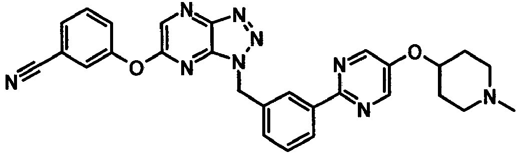 |
| "B31" | 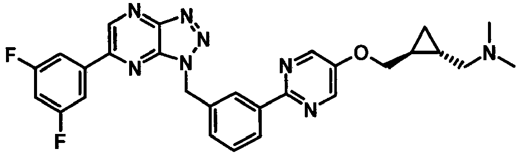 |
| "B32" | 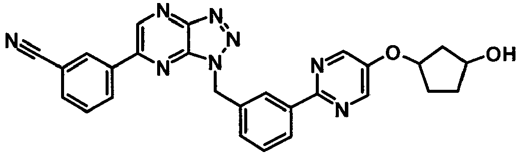 |
| "B33" | 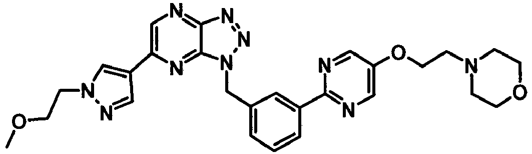 |
| "B34" | 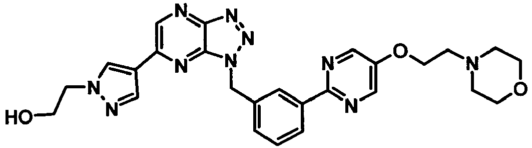 |
| "B35" | 5-Chlor-1-{3-[5-(2-morpholin-4-yl-ethoxy)-pyrimidin-2-yl]-benzyl)-1H-benzotriazol |
| "B36" | (5-Brom-2-nitro-phenyl)-{3-[5-(2-morpholin-4-yl-ethoxy)-pyrimidin-2-yl]-benzyl}-amin |
| "B37" | 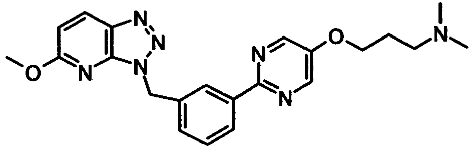 |
sowie ihre pharmazeutisch verwendbaren Salze, Tautomere und Stereoisomere, einschließlich deren Mischungen in allen Verhältnissen.

3. Verfahren zur Herstellung von Verbindungen der Formel I nach den Ansprüchen 1-2 sowie ihrer pharmazeutisch verwendbaren Salze, Tautomeren und Stereoisomeren, **dadurch gekennzeichnet, daß** man
a) eine Verbindung der Formel II 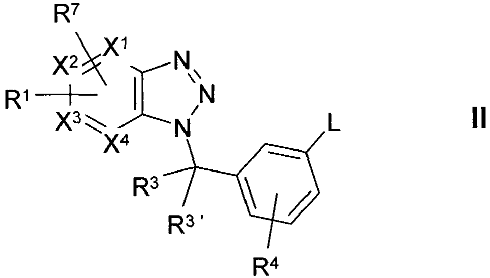 worin X¹, X², X³, X⁴, R¹, R³, R³', R⁴ und R⁷ die in Anspruch 1 angegebenen Bedeutungen haben und
L einen Boronsäure- oder Boronsäureesterrest bedeutet, mit einer Verbindung der Formel III 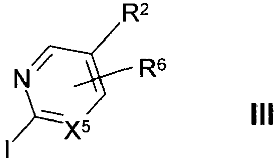 worin X⁵, R² und R⁶ die in Anspruch 1 angegebenen Bedeutungen haben,
umsetzt,
oder
b) einen Rest R¹, R² und/oder R⁷ durch einen anderen Rest R¹, R² und/oder R⁷ austauscht, indem ein Halogenatom durch einen Rest Het und/oder Ar, die die in Anspruch 1 angegebenen Bedeutungen haben, ersetzt,
oder
c) eine Verbindung der Formel IV 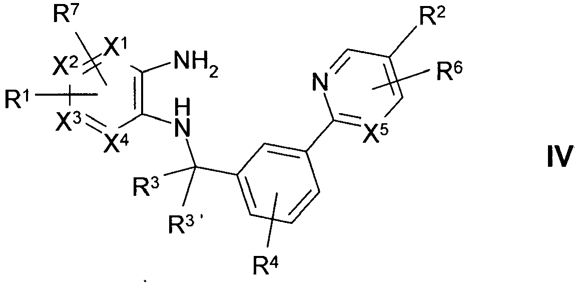 worin X¹, X² X³, X⁴, X⁵, , R¹ R², R³, R³', R⁴, R⁶ und R⁷ die in Anspruch 1 angegebenen Bedeutungen haben, mit NaNO₂ umsetzt,
und/oder
eine Base oder Säure der Formel I in eines ihrer Salze umwandelt.

4. Arzneimittel, enthaltend mindestens eine Verbindung der Formel I nach Anspruch 1-2 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

5. Verwendung von Verbindungen nach Anspruch 1-2 sowie ihrer pharmazeutisch verwendbaren Salze, Tautomeren und Stereoisomeren, einschließlich deren Mischungen in allen Verhältnissen,
zur Herstellung eines Arzneimittels zur Behandlung von Krankheiten, wobei die zu behandelnde Krankheit ein fester Tumor oder ein Tumor des Blut- und Immunsystems ist.

6. Verwendung nach Anspruch 5, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Kehlkopfs und/oder der Lunge stammt.

7. Verwendung nach Anspruch 5, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom, stammt.

8. Arzneimittel enthaltend mindestens eine Verbindung der Formel I gemäß Anspruch 1 oder 2 und/oder ihre pharmazeutisch verwendbaren Salze und Stereoisomere, einschließlich deren Mischungen in allen Verhältnissen, und mindestens einen weiteren Arzneimittelwirkstoff.

9. Set (Kit), bestehend aus getrennten Packungen von
(a) einer wirksamen Menge an einer Verbindung der Formel I gemäß Anspruch 1 oder 2 und/oder ihrer pharmazeutisch verwendbaren Salze und Stereoisomere, einschließlich deren Mischungen in allen Verhältnissen,
und
(b) einer wirksamen Menge eines weiteren Arzneimittelswirkstoffs.

## Claims

1. Compounds of the formula I 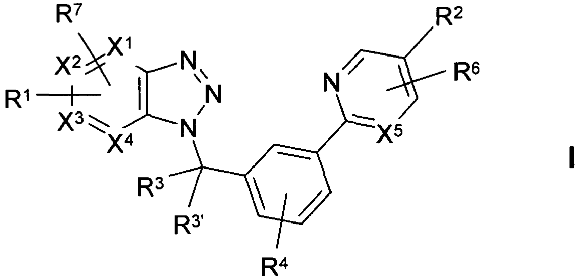 in which
X¹, X², X³, X⁴, X⁵ each, independently of one another, denote CH or N,
R¹ denotes H, Hal, A, S(O)ₘA, Ar, Het, O[C(R⁵)₂]ₙAr, O[C(R⁵)₂]ₙHet or OR⁵,
R⁷ denotes H or Hal,
R² denotes A, Hal, [C(R⁵)₂]ₙN(R⁵)₂, [C(R⁵)₂]ₙHet, O[C(R⁵)₂]ₚN(R⁵)₂, O[C(R⁵)₂]ₙHet, [C(R⁵)₂]ₙOR⁵, O[C(R⁵)₂]ₚOR⁵, O-[C(R⁵)₂]ₙ-cycloalkylene-[C(R⁵)₂]ₙ-N(R⁵)₂, [C(R⁵)₂]ₙNR⁵COOA or CH=CH-COOR⁵,
R³, R³' each, independently of one another, denote H or R⁸,
R⁴, R⁶ denote H,
R⁵ denotes H or R⁸,
R⁸ denotes unbranched or branched alkyl having 1-6 C atoms,
A denotes unbranched or branched alkyl having 1-10 C atoms,
in which 1-7 H atoms may be replaced by OH, F, Cl and/or Br, or cyclic alkyl having 3-7 C atoms, which may be mono-substituted by OH,
Ar denotes phenyl which is unsubstituted or mono-, di- or trisubstituted by Hal, A and/or CN,
Het denotes piperidinyl, pyrrolidinyl, morpholinyl, piperazinyl, oxazolidinyl, pyrazolyl, pyridinyl, pyrimidinyl, furyl, thienyl, oxazolyl, oxadiazolyl, imidazolyl, pyrrolyl, isoxa-zolyl or imidazolidinyl, where the radicals may also be mono- or disubstituted by Hal, A, COOR⁵, O[C(R⁵)₂]ₚ-OR⁵, [C(R⁵)₂]ₙHet¹, O[C(R⁵)₂]ₙHet¹ and/or =O,
Het¹ denotes piperidinyl, pyrrolidinyl, morpholinyl, piperazinyl, oxazolidinyl or imidazolidinyl, where the radicals may also be mono- or disubstituted by COOA, =O and/or A,
Hal denotes F, Cl, Br or I,
m denotes 0, 1 or 2,
n denotes 0, 1, 2, 3 or 4,
p denotes 1, 2, 3 or 4,
and pharmaceutically usable salts, tautomers and stereoisomers thereof, including mixtures thereof in all ratios.

2. Compounds according to Claim 1, selected from the group
| No. | Structure and/or name |
|---|---|
| "A1" | 6-Bromo-1-{3-[5-(2-morpholin-4-ylethoxy)pyrimidin-2-yl]-benzyl}-1H-1,2,3-triazolo[4,5-b]pyrazine |
| "A2" | 6-(1-Methyl-1H-pyrazol-4-yl)-1-{3-[5-(2-morpholin-4-y)-ethoxy)pyrimidin-2-yl]benzyl}-1H-1,2,3-triazolo[4,5-b]-pyrazine |
| "A3" | 1-{3-[5-(2-Morpholin-4-ylethoxy)pyrimidin-2-yl]benzyl}-1H-benzotriazole |
| "A4" | 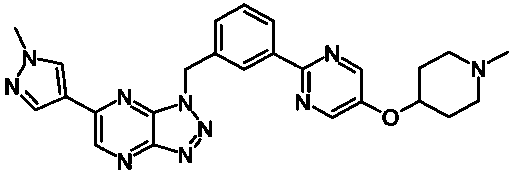 |
| "A5" | 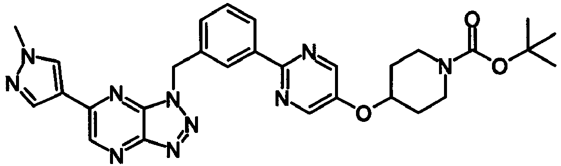 |
| "A6" | 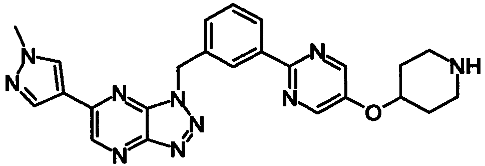 |
| "A7" | 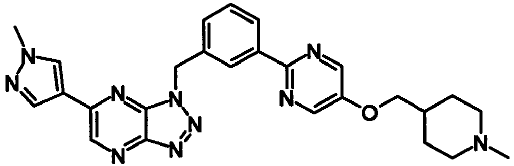 |
| "A8" | 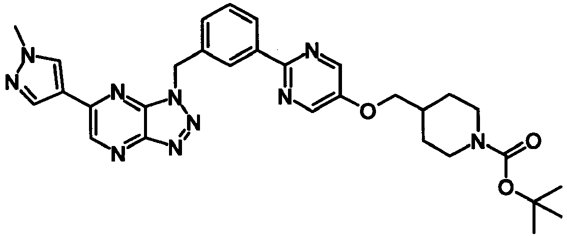 |
| "A9" | 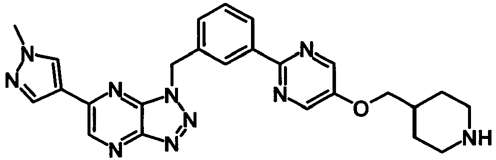 |
| "A10" | 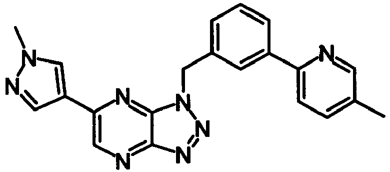 |
| "A11" | 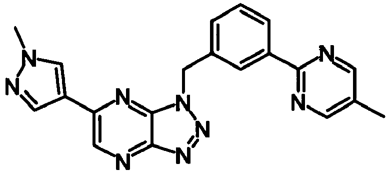 |
| "A12" | 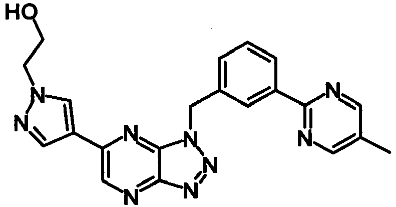 |
| "A13" | 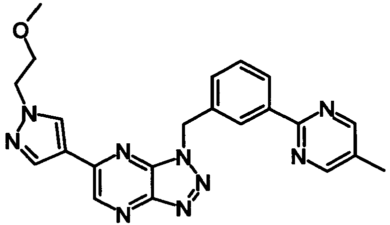 |
| "A14" | 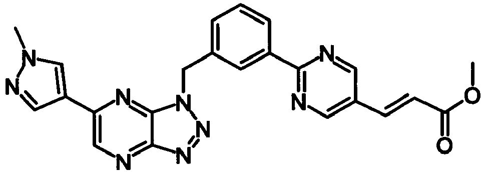 |
| "A15" | 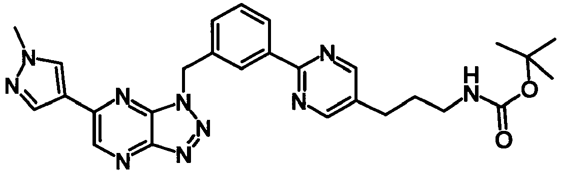 |
| "A16" | 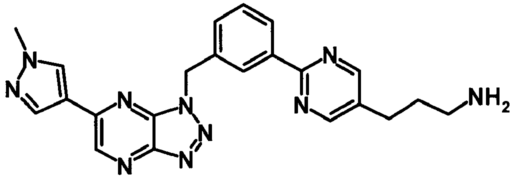 |
| "A17" | 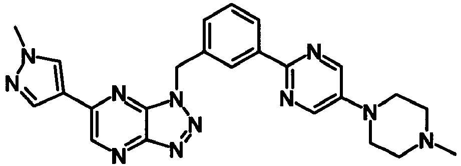 |
| "A18" | 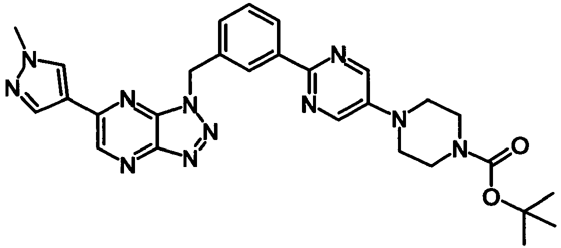 |
| "A19" | 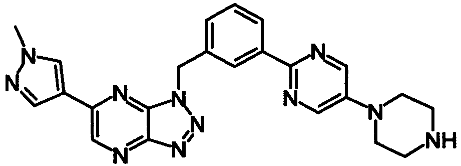 |
| "A20" | 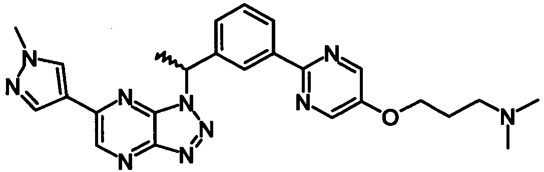 |
| "A21" | 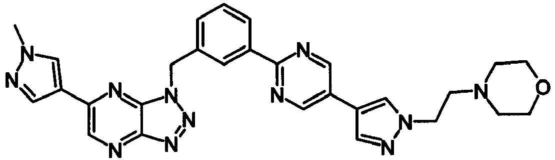 |
| "A22" | 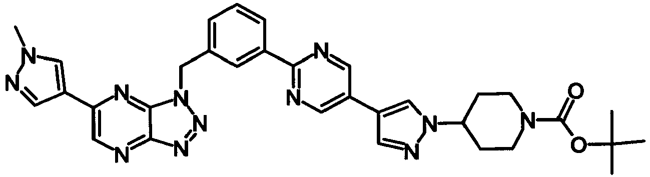 |
| "A23" | 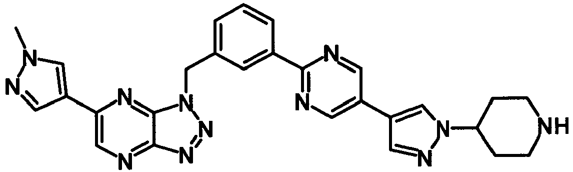 |
| "A24" | 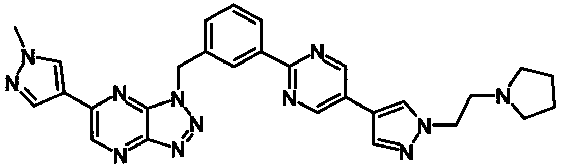 |
| "A25" | 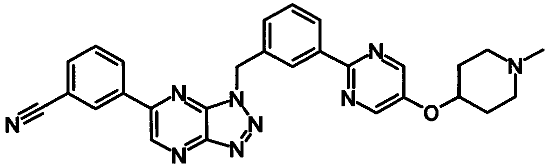 |
| "A26" | 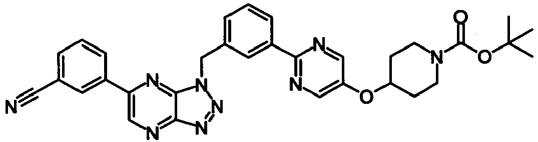 |
| "A27" | 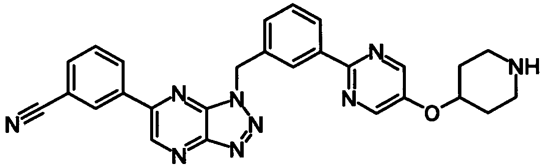 |
| "A28" | 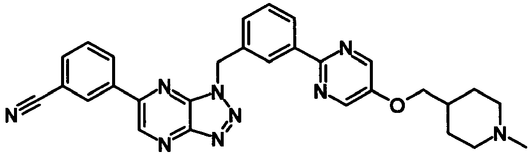 |
| "A29" | 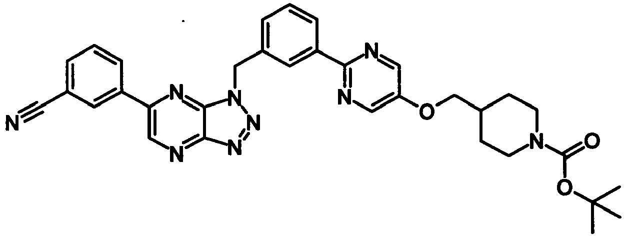 |
| "A30" | 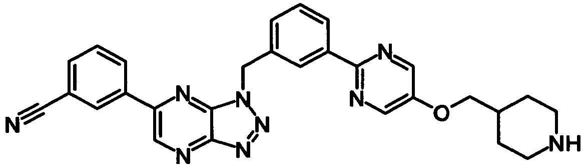 |
| "A31" | 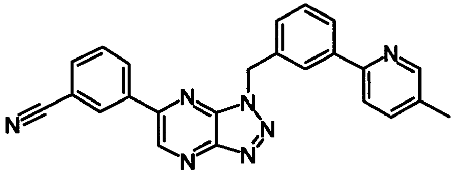 |
| "A32" | 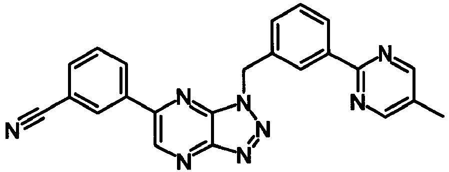 |
| "A33" | 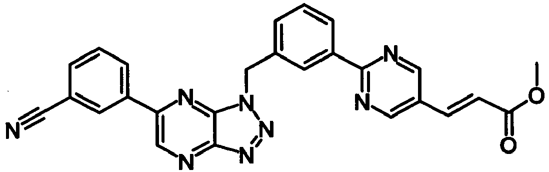 |
| "A34" | 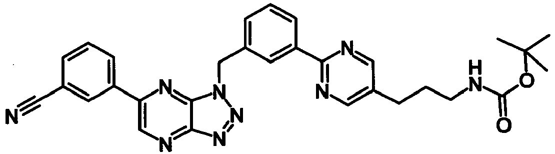 |
| "A35" | 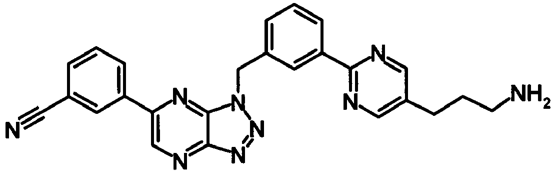 |
| "A36" | 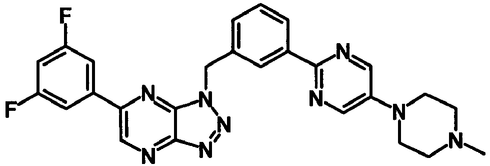 |
| "A37" | 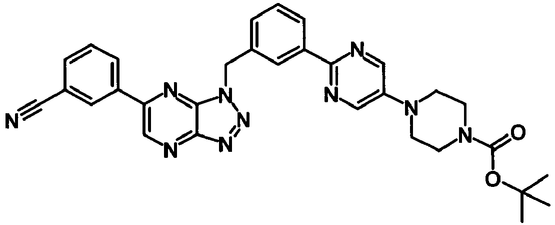 |
| "A38" | 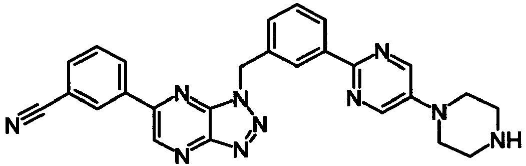 |
| "A39" | 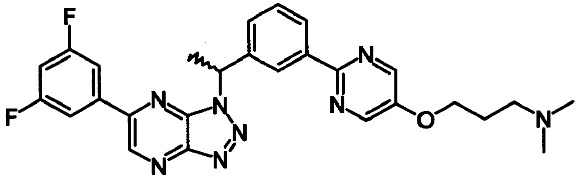 |
| "A40" | 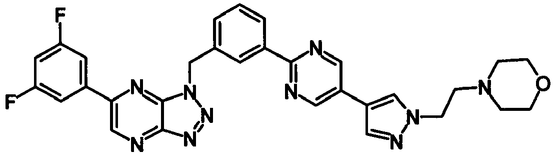 |
| "A41" | 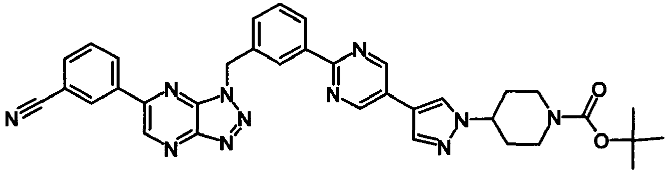 |
| "A42" | 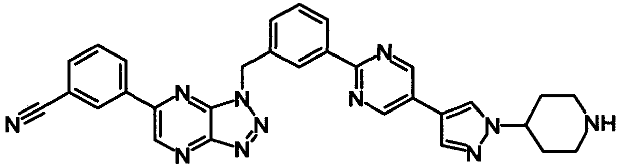 |
| "A43" | 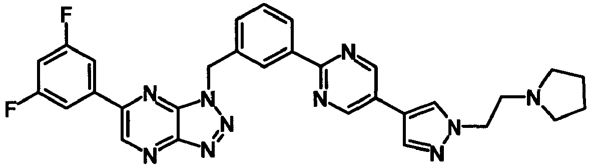 |
| "A44" | 3-{3-[5-(2-Morpholin-4-ylethoxy)pyrimidin-2-yl]benzyl}-3H-1,2,3-triazolo[4,5-b]pyridine |
| "A45" | 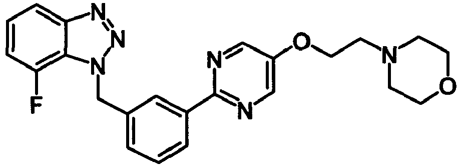 |
| "A46" | 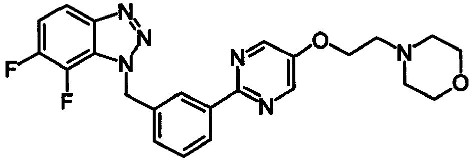 |
| "A47" | 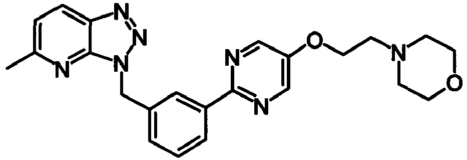 |
| "A48" | 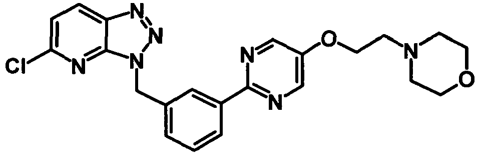 |
| "A49" | 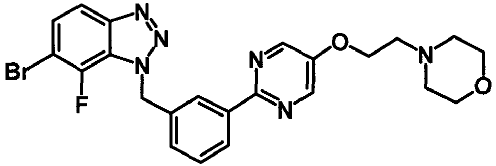 |
| "A50" | 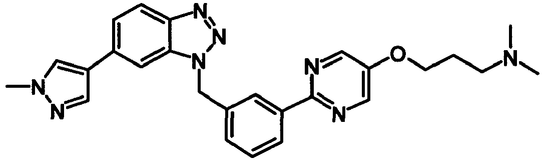 |
| "A51" | 5-(1-Methyl-1H-pyrazol-4-yl)-3-{3-[5-(2-morpholin-4-yl-ethoxy)pyrimidin-2-yl]benzyl}-3H-1,2,3-triazolo[4,5-d]-pyrimidine |
| "A52" | 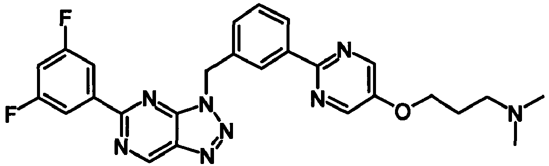 |
| "A53" | 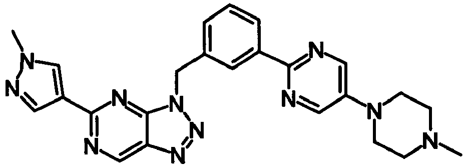 |
| "A54" | 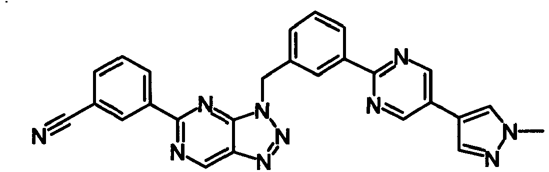 |
| "A55" | 1-{3-[5-(2-Morpholin-4-ylethoxy)pyrimidin-2-yl]benzyl}-6-(propane-1-sulfonyl)-1H-benzotriazole |
| "B1" | 6-Bromo-1-[3-(5-bromopyrimidin-2-yl)benzyl]-1H-1,2,3-triazolo[4,5-b]pyrazine |
| "B2" | 1-[3-(5-Bromopyrimidin-2-yl)benzyl]-6-(1-methyl-1H-pyrazol-4-yl)-1H-1,2,3-triazolo[4,5-b]pyrazine |
| "B3" | 3-{3-[3-(5-Bromopyrimidin-2-yl)benzyl]-3H-1,2,3-triazolo[4,5-b]pyrazin-5-yl}benzonitrile |
| "B4" | 1-[3-(5-Bromopyrimidin-2-yl)benzyl]-6-(3,5-difluorophenyl)-1H-1,2,3-triazolo[4,5-b]pyrazine |
| "B5" | 1-[3-(5-Bromopyrimidin-2-yl)benzyl]-6-(1-methyl-1H-pyrazol-4-yloxy)-1H-1,2,3-triazolo[4,5-b]pyrazine |
| "B6" | 3-{3-[3-(5-Bromopyrimidin-2-yl)benzyl]-3H-1,2,3-triazolo[4,5-b]pyrazin-5-yloxy}benzonitrile |
| "B7" | 1-[3-(5-Bromopyrimidin-2-yl)benzyl]-6-(3,5-difluorophenoxy)-1H-1,2,3-triazolo[4,5-b]pyrazine |
| "B8" | 2-{3-[6-(1-Methyl-1H-pyrazol-4-yl)-1,2,3-triazolo[4,5-b]-pyrazin-1-ylmethyl]phenyl}pyrimidin-5-ol |
| "B9" | 2-{3-[3-(3-Hydroxylpyrimidin-2-yl)benzyl]-3H-1,2,3-triazolo[4,5-b]pyrazin-5-yl}benzonitrile |
| "B10" | 2-{3-[6-(3,5-Difluorophenyl)-1,2,3-triazolo[4,5-b]pyrazin-1-ylmethyl]phenyl}pyrimidin-5-ol |
| "B11" | 2-{3-[6-(1-Methyl-1H-pyrazol-4-yloxy)-1,2,3-triazolo-[4,5-b]pyrazin-1-ylmethyl]phenyl}pyrimidin-5-ol |
| "B12" | 3-{3-[3-(5-Hydroxypyrimidin-2-yl)benzyl]-3H-1,2,3-triazolo[4,5-b]pyrazin-5-yloxy}benzonitrile |
| "B13" | 2-{3-[6-(3,5-Difluorophenoxy)-1,2,3-triazolo[4,5-b]pyrazin-1-y(methyl]phenyl}pyrimidin-5-ol |
| "B14" | Dimethyl-[2-(2-{3-[6-(1-methyl-1H-pyrazol-4-yl)-1,2,3-triazolo[4,5-b]pyrazin-1-ylmethyl]phenyl}pyrimidin-5-yl-oxy)ethyl]amine |
| "B15" | 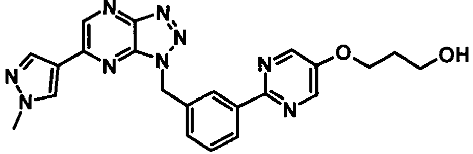 |
| "B16" | 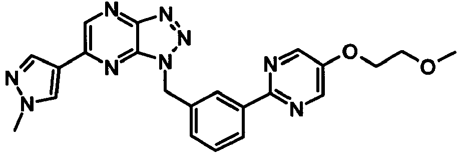 |
| "B17" | 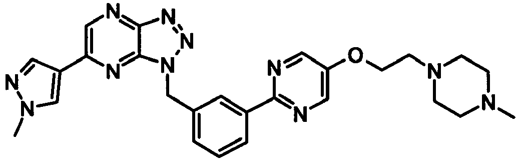 |
| "B18" | 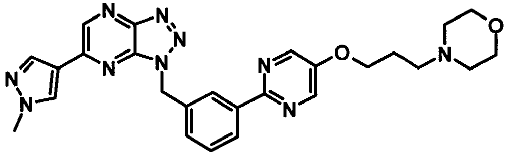 |
| "B19" | 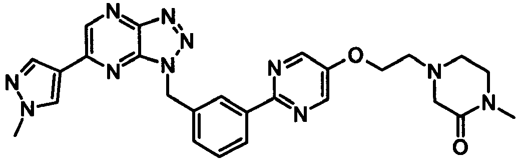 |
| "B20" | 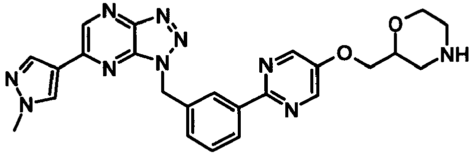 |
| "B21" | 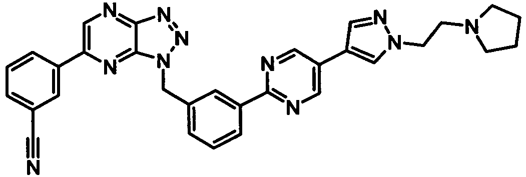 |
| "B22" | 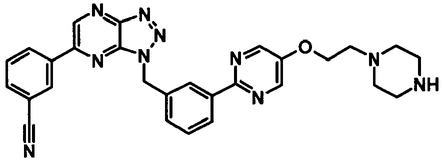 |
| "B23" | 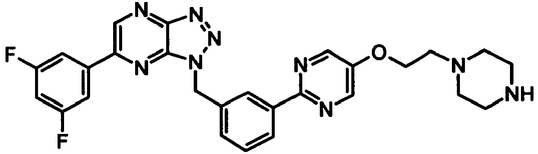 |
| "B24" | 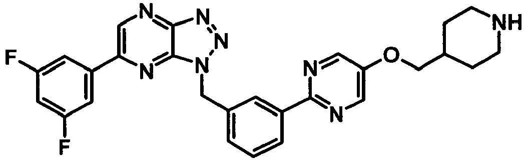 |
| "B25" | 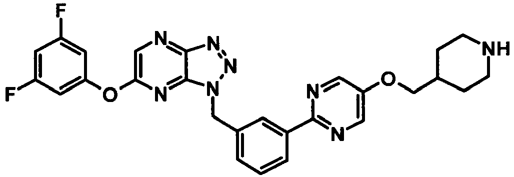 |
| "B26" | 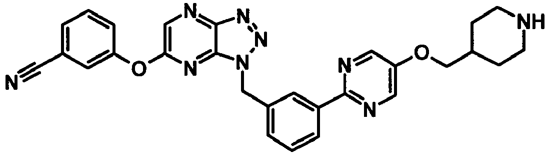 |
| "B27" | 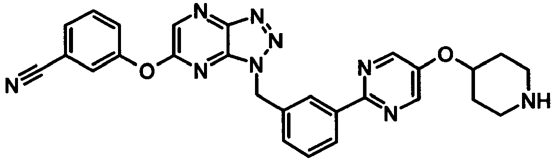 |
| "B28" | 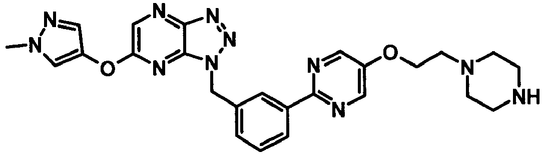 |
| "B29" | 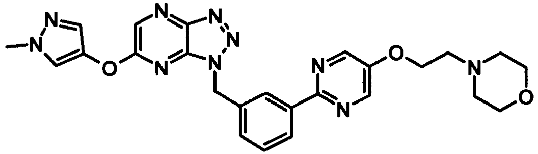 |
| "B30" | 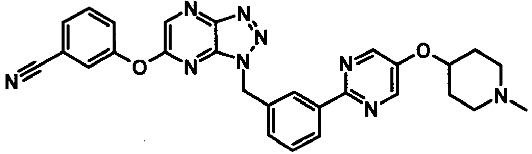 |
| "B31" | 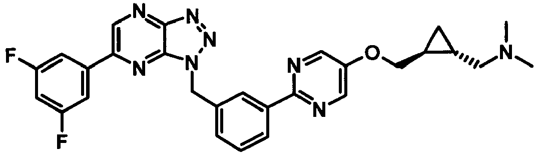 |
| "B32" | 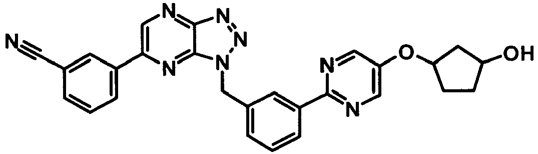 |
| "B33" | 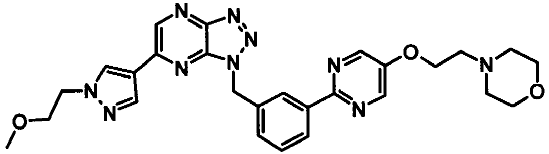 |
| "B34" | 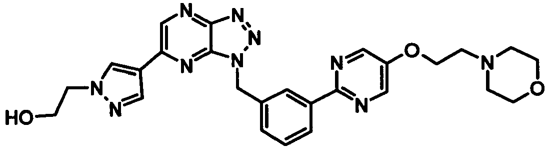 |
| "B35" | 5-Chloro-1-{3-[5-(2-morpholin-4-ylethoxy)pyrimidin-2-yl]-benzyl}-1H-benzotriazole |
| "B36" | (5-Bromo-2-nitrophenyl)-{3-[5-(2-morpholin-4-ylethoxy)-pyrimidin-2-yl]benzyl}amine |
| "B37" | 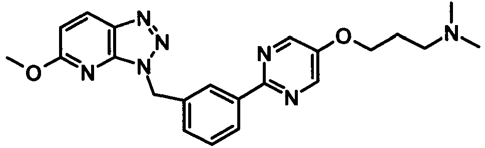 |
and pharmaceutically usable salts, tautomers and stereoisomers thereof, including mixtures thereof in all ratios.

3. Process for the preparation of compounds of the formula I according to Claims 1-2 and pharmaceutically usable salts, tautomers and stereoisomers thereof, **characterised in that**
a) a compound of the formula II 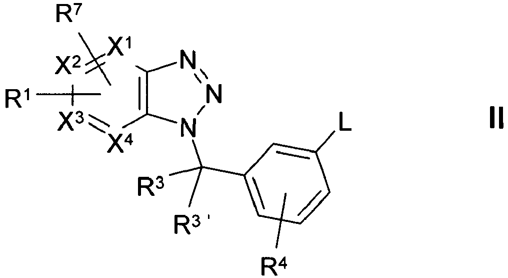
in which X¹, X², X³, X⁴, R¹, R³, R^{3'}, R⁴ and R⁷ have the meanings indicated in Claim 1 and
L denotes a boronic acid or boronic acid ester radical,
is reacted with a compound of the formula III 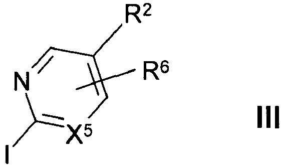
in which X⁵, R² and R⁶ have the meanings indicated in Claim 1,
or
b) a radical R¹, R² and/or R⁷ is replaced by another radical R¹, R² and/or R⁷ by replacing a halogen atom by a radical Het and/or Ar, which have the meanings indicated in Claim 1,
or
c) a compound of the formula IV 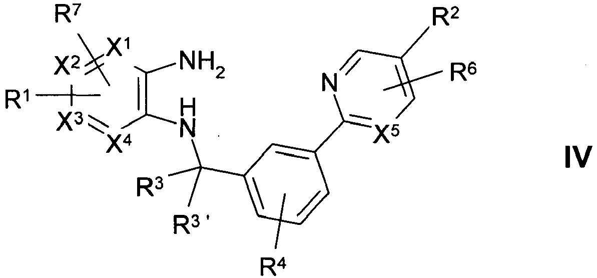
in which X¹, X², X³, X⁴, X⁵, R¹, R², R³, R^{3'}, R⁴, R⁶ and R⁷ have the meanings indicated in Claim 1,
is reacted with NaNO₂,
and/or
a base or acid of the formula I is converted into one of its salts.

4. Medicaments comprising at least one compound of the formula I according to Claims 1-2 and/or pharmaceutically usable salts, tautomers and stereoisomers thereof, including mixtures thereof in all ratios, and optionally excipients and/or adjuvants.

5. Use of compounds according to Claims 1-2
and pharmaceutically usable salts, tautomers and stereoisomers thereof, including mixtures thereof in all ratios,
for the preparation of a medicament for the treatment of diseases, where the disease to be treated is a solid tumour or a tumour of the blood and immune system.

6. Use according to Claim 5, where the solid tumour originates from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the larynx and/or of the lung.

7. Use according to Claim 5, where the solid tumour originates from the group monocytic leukaemia, lung adenocarcinoma, small-cell lung carcinomas, pancreatic cancer, glioblastomas and breast carcinoma.

8. Medicaments comprising at least one compound of the formula I according to Claim 1 or 2 and/or pharmaceutically usable salts and stereoisomers thereof, including mixtures thereof in all ratios, and at least one further medicament active compound.

9. Set (kit) consisting of separate packs of
(a) an effective amount of a compound of the formula I according to Claim 1 or 2 and/or pharmaceutically usable salts and stereoisomers thereof, including mixtures thereof in all ratios,
and
(b) an effective amount of a further medicament active compound.

## Revendications

1. Composés de formule I 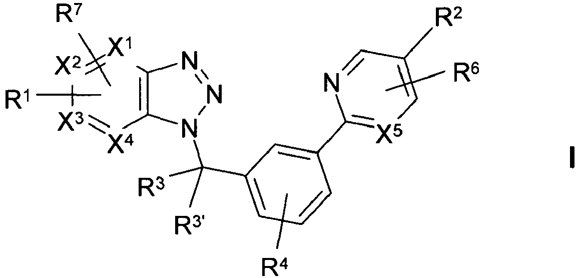 dans lesquels
X¹, X², X³, X⁴, X⁵ désignent chacun, indépendamment les uns des autres, CH ou N,
R¹ désigne H, Hal, A, S(O)ₘA, Ar, Hét, O[C(R⁵)₂]ₙAr, O[C(R⁵)₂]ₙHét ou OR⁵,
R⁷ désigne H ou Hal,
R² désigne A, Hal, [C(R⁵)₂]ₙN(R⁵)₂, [C(R⁵)₂]ₙHét, O[C(R⁵)₂]ₚN(R⁵)₂, O[C(R⁵)₂]ₙHét, [C(R⁵)₂]ₙOR⁵, O[C(R⁵)₂]ₚOR⁵, O-[C(R⁵)₂]ₙ-cycloalkylène-[C(R⁵)₂]ₙ-N(R⁵)₂, [C(R⁵)₂]ₙNR⁵COOA ou CH=CH-COOR⁵,
R³, R^{3'} désignent chacun, indépendamment les uns des autres, H ou R⁸,
R⁴, R⁶ désignent H,
R⁵ désigne H ou R⁸,
R⁸ désigne alkyle non ramifié ou ramifié ayant 1-6 atomes de C,
A désigne alkyle non ramifié ou ramifié ayant 1-10 atomes de C,
où 1-7 atomes de H peuvent être remplacés par OH, F, Cl et/ou Br,
ou
alkyle cyclique ayant 3-7 atomes de C, pouvant être monosubstitué par OH,
Ar désigne phényle qui est non substitué ou mono-, di- ou trisubstitué par Hal, A et/ou CN,
Hét désigne pipéridinyle, pyrrolidinyle, morpholinyle, pipéra-zinyle, oxazolidinyle, pyrazolyle, pyridinyle, pyrimidinyle, furyle, thiényle, oxazolyle, oxadiazolyle, imidazolyle, pyrrolyle, isoxazolyle ou imidazolidinyle, où les radicaux peuvent également être mono- ou disubstitués par Hal, A, COOR⁵, O[C(R⁵)₂]ₚOR⁵, [C(R⁵)₂]ₙHét¹, O[C(R⁵)₂]ₙHét¹ et/ou =O,
Hét¹ désigne pipéridinyle, pyrrolidinyle, morpholinyle, pipérazinyle, oxazolidinyle ou imidazolidinyle, où les radicaux peuvent également être mono- ou disubstitués par COOA, =O et/ou A,
Hal désigne F, Cl, Br ou I,
m désigne 0, 1 ou 2,
n désigne 0, 1, 2, 3 ou 4,
p désigne 1, 2, 3 ou 4,
ainsi que les sels, tautomères et stéréoisomères pharmaceutiquement utilisables de ceux-ci, y compris des mélanges de ceux-ci en toutes proportions.

2. Composés selon la revendication 1, choisis dans le groupe constitué par
| n° | Structure et/ou nom |
|---|---|
| « A1 » | 6-Bromo-1-{3-[5-(2-morpholin-4-yléthoxy)pyrimidin-2-yl]-benzyl}-1H-1,2,3-triazolo[4,5-b]pyrazine |
| « A2 » | 6-(1-Méthyl-1H-pyrazol-4-yl)-1-{3-[5-(2-morpholin-4-yl-éthoxy)pyrimidin-2-yl]benzyl}-1H-1,2,3-triazolo[4,5-b]pyrazine |
| « A3 » | 1-{3-[5-(2-Morpholin-4-yléthoxy)pyrimidin-2-yl]benzyl}-1H-benzotriazole |
| « A4 » | 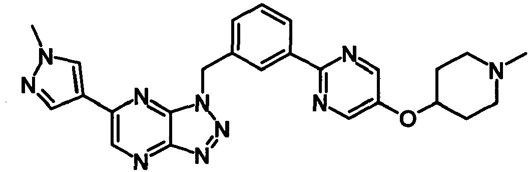 |
| « A5 » | 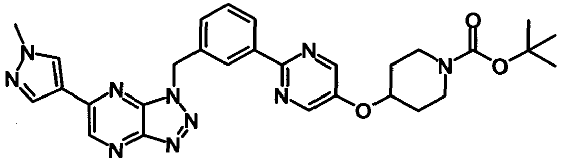 |
| « A6 » | 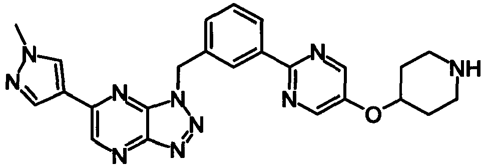 |
| « A7 » | 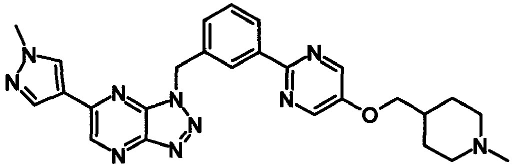 |
| « A8 » | 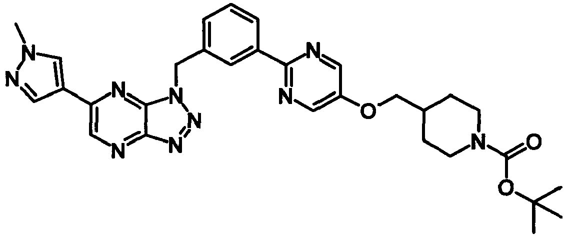 |
| « A9 » | 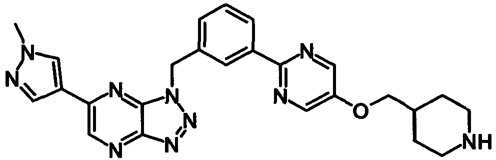 |
| « A10 » | 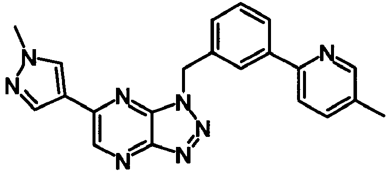 |
| « A11 » | 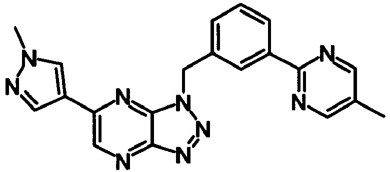 |
| « A12 » | 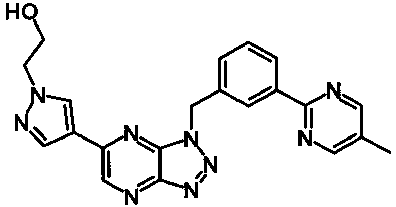 |
| « A13 » | 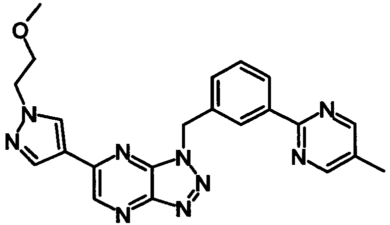 |
| « A14 » | 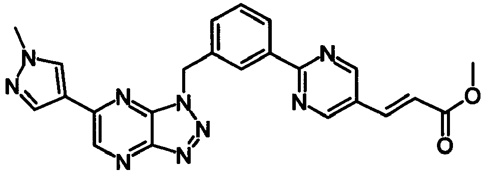 |
| « A15 » | 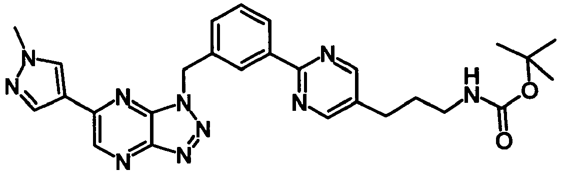 |
| « A16 » | 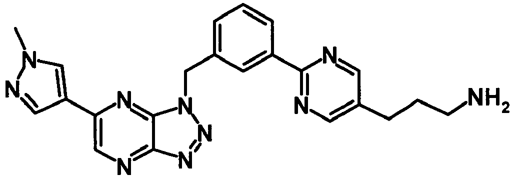 |
| « A17 » | 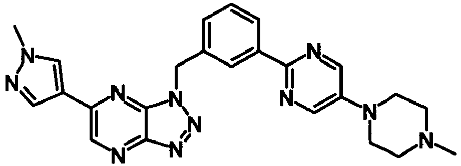 |
| «A18» | 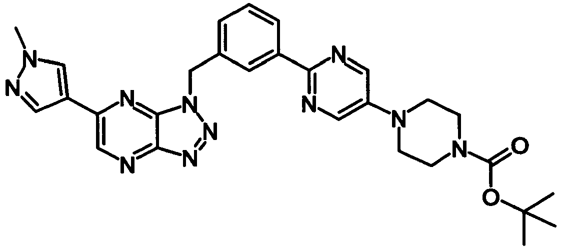 |
| « A19 » | 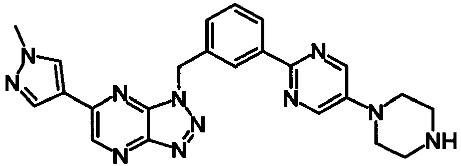 |
| « A20 » | 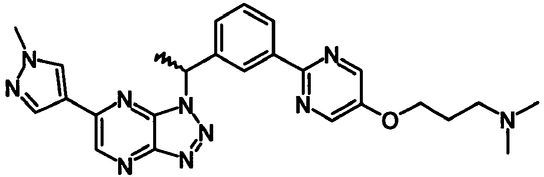 |
| « A21 » | 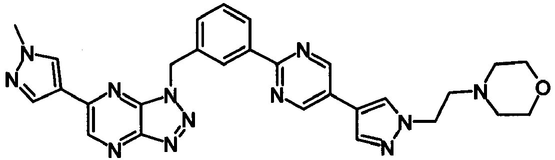 |
| « A22 » | 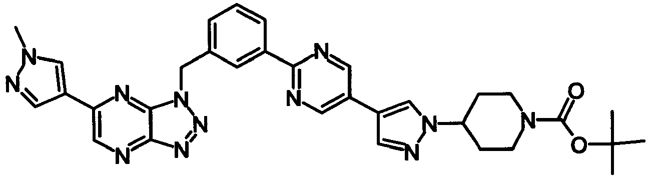 |
| « A23 » | 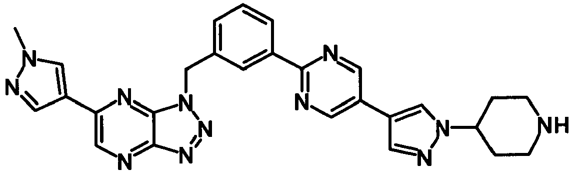 |
| « A24 » | 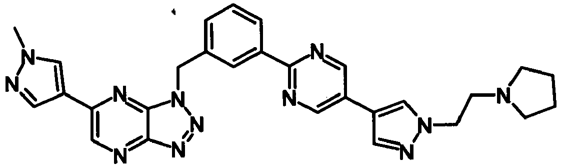 |
| « A25 » | 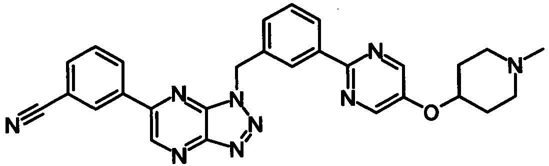 |
| « A26 » | 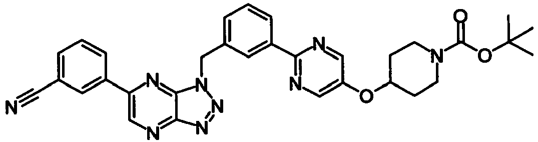 |
| « A27 » | 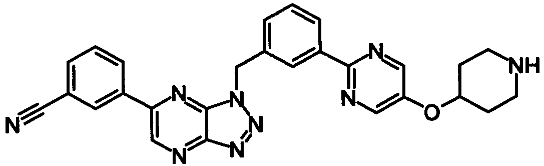 |
| « A28 » | 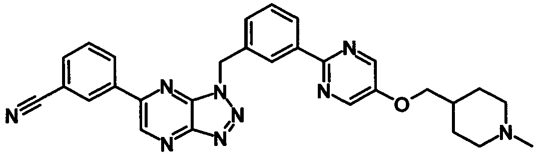 |
| « A29 » | 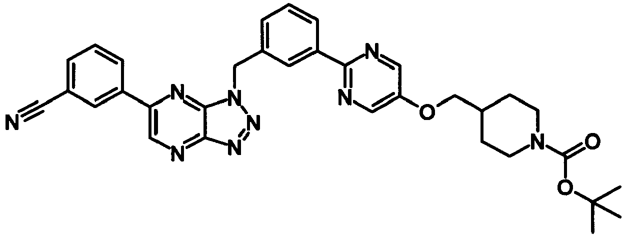 |
| « A30 » | 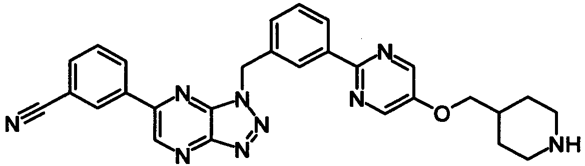 |
| « A31 » | 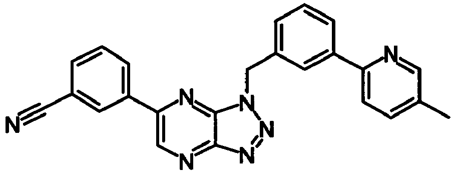 |
| « A32 » | 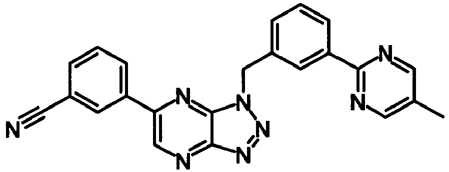 |
| « A33 » | 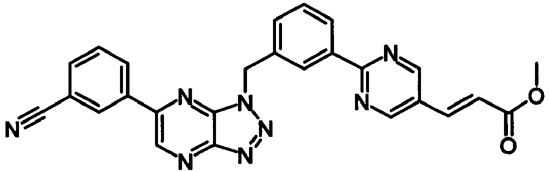 |
| « A34 » | 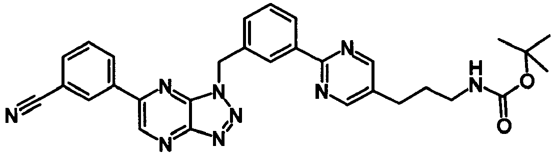 |
| « A35 » | 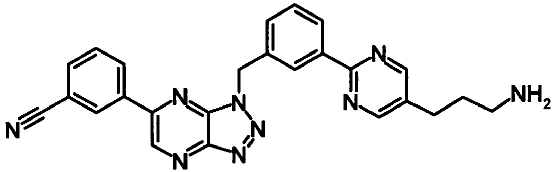 |
| « A36 » | 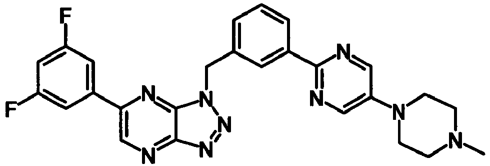 |
| « A37 » | 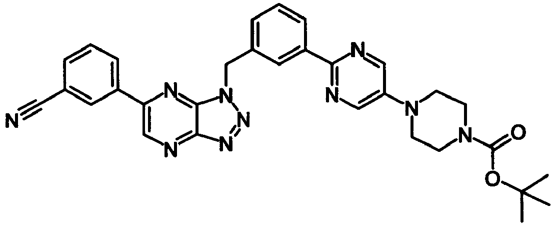 |
| « A38 » | 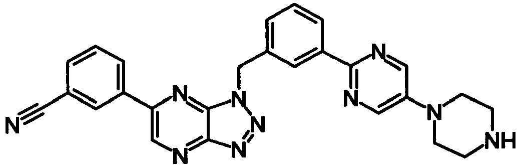 |
| « A39 » | 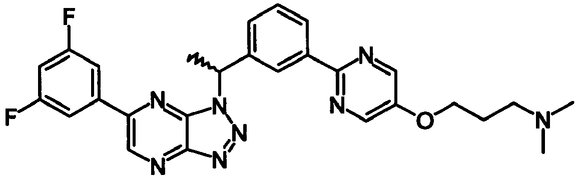 |
| « A40 » | 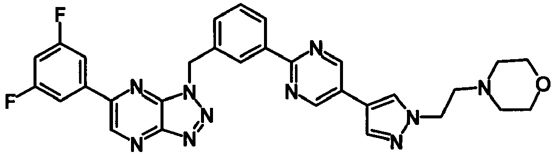 |
| « A41 » | 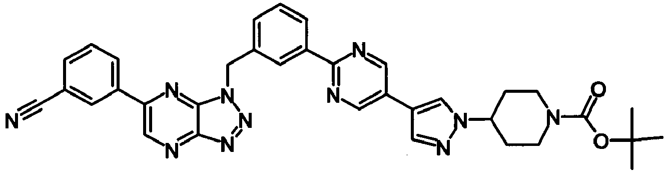 |
| « A42 » | 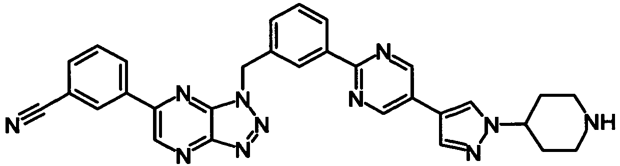 |
| « A43 » | 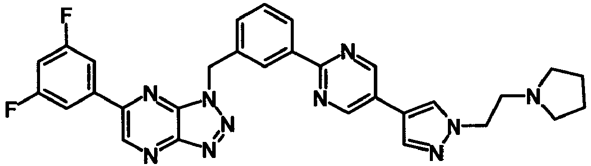 |
| « A44 » | 3-{3-[5-(2-Morpholin-4-yléthoxy)pyrimidin-2-yl]benzyl}-3H-1,2,3-triazolo[4,5-b]pyridine |
| « A45 » | 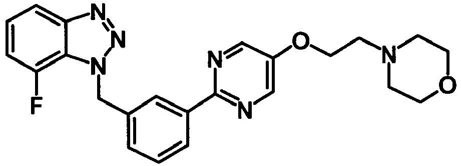 |
| « A46 » | 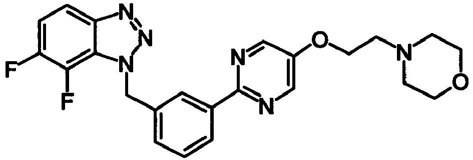 |
| « A47 » | 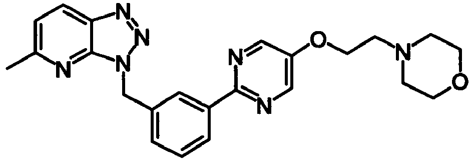 |
| « A48 » | 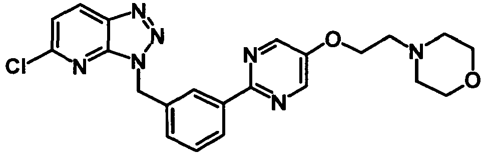 |
| « A49 » | 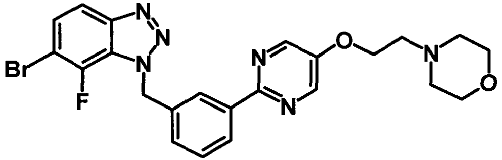 |
| « A50 » | 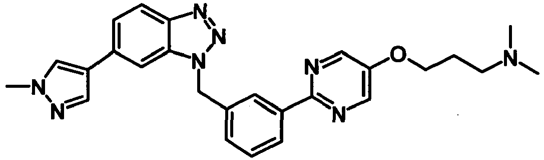 |
| « A51 » | 5-(1-Méthyl-1H-pyrazol-4-yl)-3-{3-[5-(2-morpholin-4-yl-éthoxy)pyrimidin-2-yl]benzyl)-3H-1,2,3-triazolo[4,5-d]pyrimidine |
| « A52 » | 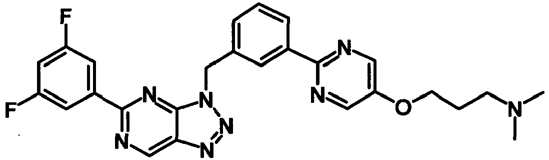 |
| « A53 » | 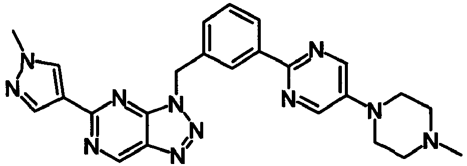 |
| « A54 » | 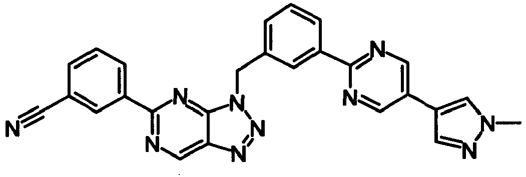 |
| « A55 » | 1-{3-[5-(2-Morpholin-4-yléthoxy)pyrimidin-2-yl]benzyl}-6-(propane-1-sulfonyl)-1H-benzotriazole |
| « B1 » | 6-Bromo-1-[3-(5-bromopyrimidin-2-yl)benzyl]-1H-1,2,3-triazolo[4,5-b]pyrazine |
| « B2 » | 1-[3-(5-Bromopyrimidin-2-yl)benzyl]-6-(1-méthyl-1H-pyrazol-4-yl)-1H-1,2,3-triazolo[4,5-b]pyrazine |
| « B3 » | 3-{3-[3-(5-Bromopyrimidin-2-yl)benzyl]-3H-1,2,3-triazolo[4,5-b]pyrazin-5-yl}benzonitrile |
| « B4 » | 1-[3-(5-Bromopyrimidin-2-yl)benzyl]-6-(3,5-difluorophényl)-1H-1,2,3-triazolo[4,5-b]pyrazine |
| « B5 » | 1-[3-(5-Bromopyrimidin-2-yl)benzyl]-6-(1-méthyl-1H-pyrazol-4-yloxy)-1H-1,2,3-triazolo[4,5-b]pyrazine |
| « B6 » | 3-{3-[3-(5-Bromopyrimidin-2-yl)benzyl]-3H-1,2,3-triazolo[4,5-b]pyrazin-5-yloxy}benzonitrile |
| « B7 » | 1-[3-(5-Bromopyrimid in-2-yl)benzyl]-6-(3,5-difluorophénoxy)-1H-1,2,3-triazolo[4,5-b]pyrazine |
| « B8 » | 2-{3-[6-(1-Méthyl-1H-pyrazol-4-yl)-1,2,3-triazolo[4,5-b]-pyrazin-1-ylméthyl]phényl}pyrimidin-5-ol |
| « B9 » | 2-{3-[3-(3-Hydroxy)pyrimidin-2-y))benzyl]-3H-1,2,3-triazolo[4,5-b]pyrazin-5-yl}benzonitrile |
| « B10 » | 2-{3-[6-(3,5-Difluorophényl)-1,2,3-triazolo[4,5-b]pyrazin-1-ylméthyl]phényl}pyrimidin-5-ol |
| « B11 » | 2-{3-[6-(1-Méthyl-1H-pyrazol-4-yloxy)-1,2,3-triazolo[4,5-b]pyrazin-1-ylméthyl]phényl}pyrimidin-5-ol |
| « B12 » | 3-{3-[3-(5-Hydroxypyrimidin-2-yl)benzyl]-3H-1,2,3-triazolo[4,5-b]pyrazin-5-yloxy}benzonitrile |
| « B13 » | 2-{3-[6-(3,5-Difluorophénoxy)-1,2,3-triazolo[4,5-b]pyrazin-1-ylméthyl]phényl}pyrimidin-5-ol |
| « B14 » | Diméthyl-[2-(2-{3-[6-(1-méthyl-1H-pyrazol-4-yl)-1,2,3-triazolo[4,5-b]pyrazin-1-ylméthyl]phényl}pyrimidin-5-yl-oxy)éthyl]amine |
| « B15 » | 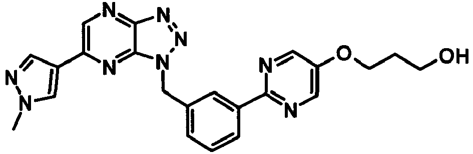 |
| « B16 » | 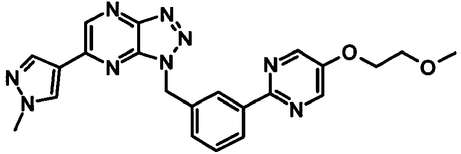 |
| « B17 » | 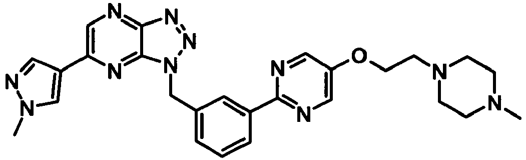 |
| « B18 » | 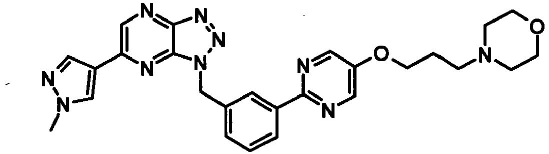 |
| « B19 » | 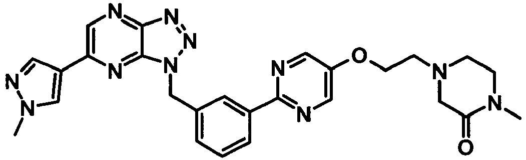 |
| « B20 » | 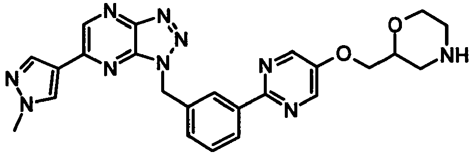 |
| « B21 » | 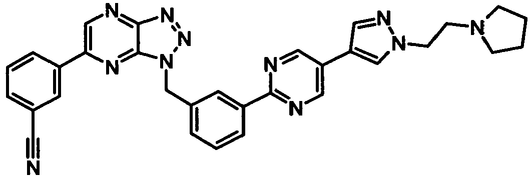 |
| « B22 » | 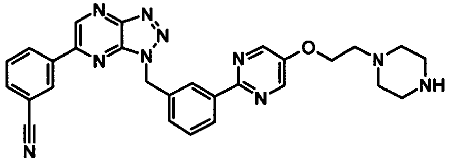 |
| « B23 » | 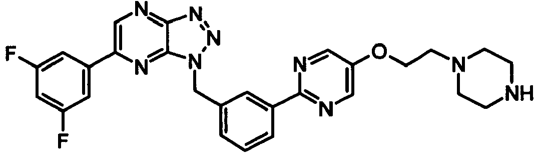 |
| « B24 » | 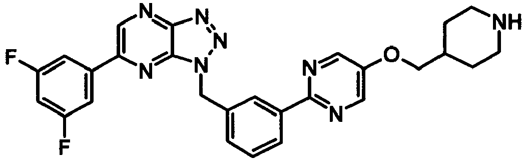 |
| « B25 » | 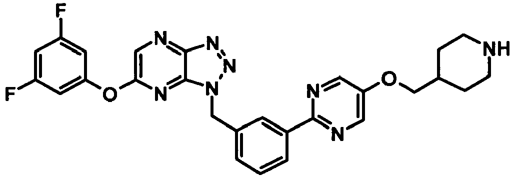 |
| « B26 » | 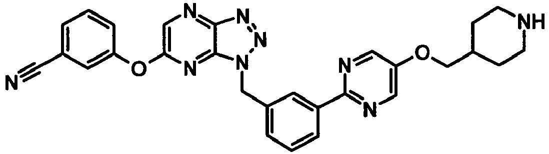 |
| « B27 » | 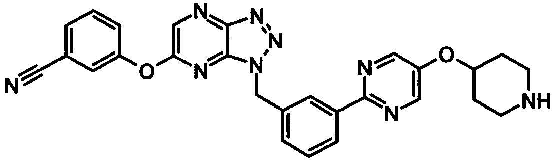 |
| « B28 » | 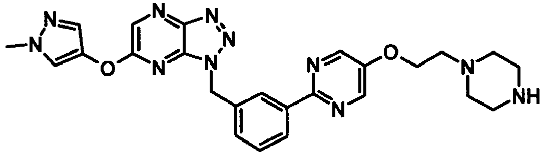 |
| « B29 » | 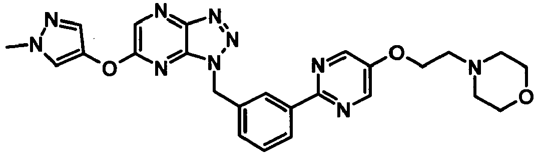 |
| « B30 » | 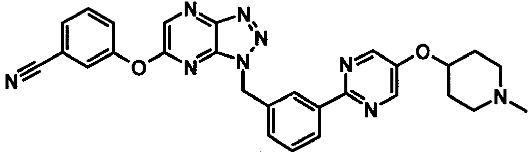 |
| « B31 » | 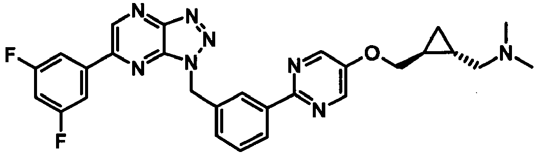 |
| « B32 » | 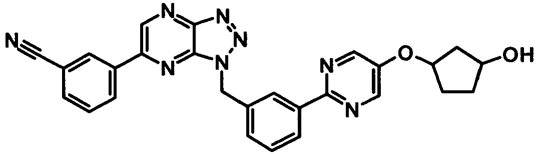 |
| « B33 » | 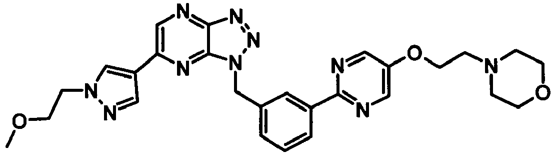 |
| « B34 » | 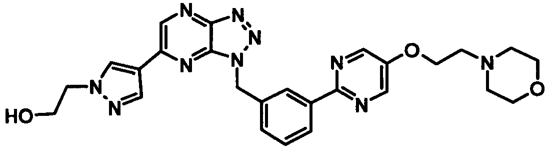 |
| « B35 » | 5-Chloro-1-{3-[5-(2-morpholin-4-yléthoxy)pyrimidin-2-yl]-benzyl}-1H-benzotriazole |
| « B36 » | (5-Bromo-2-nitrophényl)-{3-[5-(2-morpholin-4-yl-éthoxy)pyrimidin-2-yl]benzyl}amine |
| « B37 » | 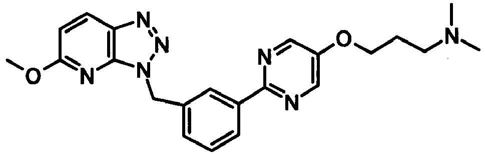 |
ainsi que les sels, tautomères et stéréoisomères pharmaceutiquement utilisables de ceux-ci, y compris des mélanges de ceux-ci en toutes proportions.

3. Procédé de préparation de composés de formule I selon les revendications 1-2 et de sels, tautomères et stéréoisomères pharmaceutiquement utilisables de ceux-ci, **caractérisé en ce que**
a) un composé de formule II 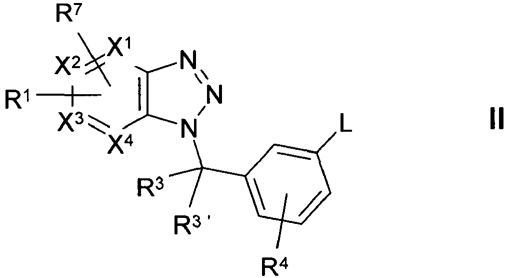
où X¹, X², X³, X⁴, R¹, R³, R^{3'}, R⁴ et R⁷ revêtent les significations indiquées selon la revendication 1 et
L désigne un acide boronique ou un radical d'ester d'acide boronique,
est réagi avec un composé de formule III 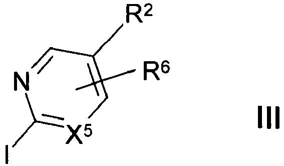
où X⁵, R² et R⁶ revêtent les significations indiquées selon la revendication 1,
ou
b) un radical R¹, R² et/ou R⁷ est remplacé par un autre radical R¹, R² et/ou R⁷ par le remplacement d'un atome d'halogène par un radical Hét et/ou Ar, qui revêtent les significations indiquées selon la revendication 1,
ou
c) un composé de formule IV 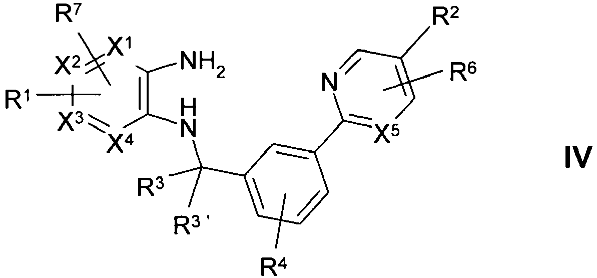
où X¹ X², X³, X⁴, X⁵, R¹, R², R³, R^{3'}, R⁴, R⁶ et R⁷ revêtent les significations indiquées selon la revendication 1,
est réagi avec NaNO₂,
et/ou
une base ou un acide de formule I est converti(e) en l'un de ses sels.

4. Médicaments comprenant au moins un composé de formule I selon les revendications 1-2 et/ou des sels, tautomères et stéréoisomères pharmaceutiquement utilisables de celui-ci, y compris des mélanges de ceux-ci en toutes proportions, et éventuellement des excipients et/ou des adjuvants.

5. Utilisation de composés selon les revendications 1-2
ainsi que de sels, tautomères et stéréoisomères pharmaceutiquement utilisables de ceux-ci, y compris des mélanges de ceux-ci en toutes proportions,
pour la préparation d'un médicament destiné au traitement de maladies, où la maladie à traiter est une tumeur solide ou une tumeur du sang et du système immunitaire.

6. Utilisation selon la revendication 5, dans laquelle la tumeur solide provient du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, du larynx et/ou du poumon.

7. Utilisation selon la revendication 5, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

8. Médicaments comprenant au moins un composé de formule I selon la revendication 1 ou 2 et/ou des sels et stéréoisomères pharmaceutiquement utilisables de celui-ci, y compris des mélanges de ceux-ci en toutes proportions, et au moins un autre composé actif médicamenteux.

9. Ensemble (kit) constitué de conditionnements séparés
(a) d'une quantité efficace d'un composé de formule I selon la revendication 1 ou 2 et/ou de sels et stéréoisomères pharmaceutiquement utilisables de celui-ci, y compris des mélanges de ceux-ci en toutes proportions,
et
(b) d'une quantité efficace d'un autre composé actif médicamenteux.
